# EUROPEAN PATENT SPECIFICATION

(11) **EP 3 107 952 B1**
(45) Date of publication and mention of the grant of the patent: **26.09.2018**
(21) Application number: 15702802.8
(22) Date of filing: 06.02.2015
(51) Int. Cl.: C08G 69/00, C07K 16/18, C40B 40/10, C40B 50/08

(54) **METHOD FOR THE GENERATION OF CHEMICAL LIBRARIES**
VERFAHREN ZUR ERZEUGUNG VON CHEMISCHEN BIBLIOTHEKEN
PROCÉDÉ POUR LA GÉNÉRATION DE BIBLIOTHÈQUES CHIMIQUES

(30) Priority: 19.02.2014 EP 14000586
(43) Date of publication of application: 28.12.2016
(73) Proprietor: ETH Zurich, 8092 Zurich (CH)
(72) Inventor: BODE, Jeffrey, CH-8008 Zürich (CH); HUANG, Yi-Lin, CH-8046 Zürich (CH); NODA, Hidetoshi, 330-0064 Saitama (JP)
(74) Representative: Bremi, Tobias Hans
(86) International application number: PCT/EP2015/052504
(87) International publication number: WO 2015/124443

(56) References cited:
- Ying-Ling Chiang ET AL: "SEQUENCE-CONTROLLED SYNTHESIS OF [beta] 3 -PEPTIDES: SOLID-PHASE SYNTHESIS AND DNA-TEMPLATED SYNTHESIS", , 2013, pages 1-212, XP055123832, Retrieved from the Internet: URL:http://media.proquest.com/media/pq/cla ssic/doc/3029152191/fmt/ai/rep/NPDF?hl=&ci t:auth=Chiang, Ying-Ling&cit:title=Sequence-controlled synthesis of beta3-peptides: Solid-phase synthesis and DNA-templated synthesis&cit:pub=ProQuest Dissertations and Theses&cit:vol=&cit:iss=&cit:pg=n/a&cit:da te=2013&i [retrieved on 2014-06-17]
- ISHIDA H ET AL: "Synthesis of an enantiopure isoxazolidine monomer for beta<3>-aspartic acid in chemoselective beta-oligopeptide synthesis", TETRAHEDRON LETTERS, PERGAMON, GB, vol. 50, no. 26, 1 July 2009 (2009-07-01), pages 3258-3260, XP026120474, ISSN: 0040-4039, DOI: 10.1016/J.TETLET.2009.02.045 [retrieved on 2009-02-11]
- YING-LING CHIANG ET AL: "Synthesis of Enantiomerically Pure Isoxazolidine Monomers for the Preparation of [beta] 3 -Oligopeptides by Iterative [alpha] -Keto AcidHydroxylamine (KAHA) Ligations", HELVETICA CHIMICA ACTA, vol. 95, no. 12, 19 December 2012 (2012-12-19), pages 2481-2501, XP055123822, ISSN: 0018-019X, DOI: 10.1002/hlca.201200484
- NANCY CARILLO ET AL: "Iterative, Aqueous Synthesis of beta3-Oligopeptides without Coupling Reagents", JOURNAL OF THE AMERICAN CHEMICAL SOCIETY, ACS PUBLICATIONS, US, vol. 128, no. 5, 8 February 2006 (2006-02-08), pages 1452-1453, XP002590576, ISSN: 0002-7863, DOI: 10.1021/JA057706J [retrieved on 2006-01-17]

## Description

### TECHNICAL FIELD

The present invention relates to a method for the generation of an oligomer or a mixture of oligomers, in particular for providing chemical libraries for activity screening. It furthermore relates to a method for identifying active compounds from such a library using a tailored screening process.

### PRIOR ART

The current practice of modern medicinal chemistry operates on a well-established strategy of hit identification, hit-to-lead, and lead optimization. These processes, in turn, rely on the preparation and high-throughput screening of vast libraries of organic compounds. For hit identification, it is not uncommon to screen one million or more compounds in a high-throughput biological assay. The screening of such large numbers of compounds requires a complex infrastructure of robotics, compound purification, and compound storage.

Typically, each compound is prepared, purified, stored, and screened as a single molecular entity. This simplifies the identification of active hits and reduces false positives, but effectively prevents the expansion of chemical libraries beyond a few million compounds.

It is now widely recognized that it is not possible for a single facility to store, handle and screen more than a few million organic compounds.

To circumvent this physical limitation, mixtures of compounds can be screened. Early attempts at the synthesis of screening of unpurified combinatorial mixtures of compounds were stymied by problems with reproducibility and biologically active impurities. Methods for screening large mixtures of tagged compounds, such as phage display, ribosome display, and DNA-encoded libraries provide a powerful and highly successful alternative.

The major limitation of these methods is the types of compounds that can be produced through biological methods are largely limited to oligopeptides comprised of naturally occurring L-amino acids. A method to rapidly synthesize, screen, and deconvolute molecules comprised of unnatural units is in high demand.

The dissertation of Ying-Ling Chiang entitled SEQUENCE-CONTROLLED SYNTHESIS OF β 3-PEPTIDES: SOLID-PHASE SYNTHESIS AND DNA-TEMPLATED SYNTHESIS (University of Pennsylvania, 2013) inter alia mentions a living homopolymerization of α-ketoacid and a single isoxazolidine monomer, but does this hypothetically and not in a specific context of using the reaction results, and further points out the intrinsic solubility and reactivity issues as well as serious decomposition and fragmentation problems. Based on these considerations in the document a different synthetic approach is taken. Termination is not an issue.

Hiroshi Ishida et al (Tetrahedron Letters 50 (2009) 3258-3260) propose a new synthesis of an enantiopure isoxazolidine monomer for β³-aspartic acid in chemoselective β-oligopeptide synthesis via chemoselective a-ketoacid-hydroxylamine amide formation.

The proposed route involves nitrone cycloaddition of 3-thiophenylpropanal and circumvents limitations of other potential starting materials.

Ying-Ling Chiang et al (Helvetica Chimica Acta - Vol. 95 (2012), 2481) propose a new method for the synthesis of enantiomerically pure isoxazolidine monomers for the synthesis of β³-oligopeptides via α-keto acid hydroxylamine (KAHA) ligation. The one-pot synthetic method utilizes in situ generated nitrones bearing gulose-derived chiral auxiliaries for the asymmetric 1,3-dipolar cycloaddition with methyl 2-methoxyacrylate.

The resulting enantiomerically pure isoxazolidine monomers bearing diverse side chains (proteinogenic and non-proteinogenic) can be synthesized in either configuration (like- and unlike-configured). The scalable and enantioselective synthesis of the isoxazolidine monomers enables the use of the synthesis of b3-oligopeptides via iterative α-keto acidhydroxylamine (KAHA) ligation.

Nancy Carrillo et al (JACS 2006, 128, 1452-1453) propose an iterative, aqueous synthesis of α³-oligopeptides without coupling reagents with isoxazolidine acetals.

### SUMMARY OF THE INVENTION

The preparation of thousands or millions of organic compounds in conjunction with high-throughput screening against a biological target is the prevailing method for developing new drugs. Methods that quickly generate chemical libraries embedded with complex functionalities through simple operations, minimal waste and easy purification are thus highly desirable. To meet these criteria, a new approach for the "on-demand" synthesis of chemical libraries has been developed by combining monomeric building blocks in aqueous buffer. This newly proposed method, which, inter alia, utilizes the chemoselective α-ketoacid-hydroxylamine (KAHA) amide-forming ligation as a core technology, allows chemical libraries to be prepared, screened, and deconvoluted using nothing more than standard pipetting or liquid handling techniques. The modular nature of the compounds enables easy tailoring for structure-activity-relationship (SAR) studies. As a proof-of-concept study, hepatitis C virus (HCV) protease inhibitor libraries were synthesized by mixing building blocks directly in multi-well plates. The reactions require no reagents and produce only CO₂ and cyclohexanone as byproducts. Each reaction well contained β-peptide α-ketoamide products and was diluted and subjected to HCV protease assay without workup or purification. Wells containing active HCV protease inhibitors were deconvoluted by preparing focused libraries and the active compounds were synthesized and confirmed with simple operations all under aqueous conditions. The final proposed inhibitor was resynthesized using the same approach, isolated, fully characterized and the activity against HCV protease confirmed to have an IC₅₀ of 1.0 µM.

The synthesis of chemical libraries for drug development is often associated with toxic reagents, organic solvents, tedious purifications, and complex handling of large numbers of isolated, individual compounds. Here a method to prepare large chemical libraries "on-demand", simply by combining building blocks in aqueous solution is proposed. The resulting libraries are subjected to high-throughput screenings in an enzymatic assay without purification or further handling. From the active mixtures, the active compounds can be identified by deconvolution, re-synthesis and isolation. This method offers simple operations, minimal waste, reproducible on-demand library preparation, and avoid the need to store thousands of discrete compounds. No reagents or protecting groups are needed and minimal amounts of innocuous byproducts are formed. This chemistry allows combinatorial mixtures of β-peptide α-ketoamides to be quickly prepared, screened and deconvoluted. Once the building blocks are obtained, the only technique needed for the library preparation is liquid handling with a microliter pipette. The resulting libraries can be screened directly on multi-well plates using an enzymatic assay. The libraries themselves do not need to be stored and can be discarded and quickly reconstituted when needed. We demonstrate the successful preparation of a library of over 6,000 compounds prepared from only 23 building blocks, the screening of this library directly from the reaction mixtures, and its deconvolution to identify a 1.0 µM inhibitor of hepatitis C virus (HCV) protease.

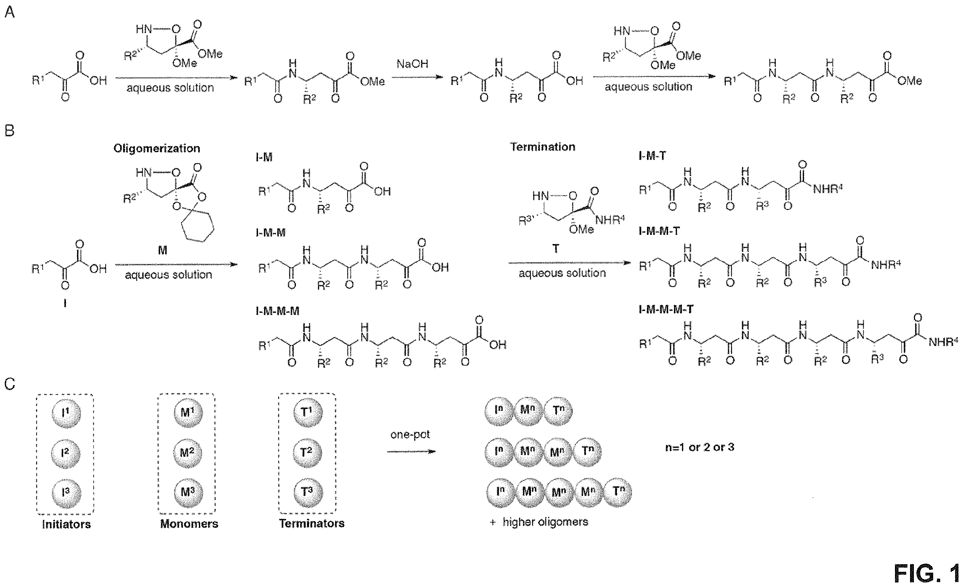

It is possible to synthesize β-peptides in solution using isoxazolidine monomers (Fig. 1A).

These monomers react chemoselectively with α-ketoacids to form amide bonds. Through consecutive repetition of monomer couplings and methyl ketoester hydrolysis, β-peptides can be obtained. This method provides a stepwise manner to form functionalized oligomers of controlled sequence; however, for syntheses of compound libraries, methods that can generate a large amount of various compounds in a one-pot fashion are needed. Therefore another type of isoxazolidine monomer (M) was designed that forms a new α-ketoacid upon ligation (Fig. 1B). The α-ketoacid can also couple with M and form oligomers of varying length and sequence; the addition of a terminator (T) ceases this oligomerization.

To prepare libraries of protease inhibitors, a terminator was designed that forms a C-terminal α-ketoamide, as it is known to be an excellent pharmacophore for several classes of proteases such as serine proteases and cysteine proteases.

The unpurified mixtures, which contain only peptide oligomers and small amounts of the terminator (T), which is used in excess, can be subjected to biological assays directly without further purification. Importantly, the number of products expands exponentially in the reaction mixture with increasing types of α-ketoacid initiators (I), monomers (M), and terminators (T) (Fig. 1C). Simply by increasing the number of each component used in the library can dramatically expand the number of potential molecules formed. For reference, the use of just 50 different initiators (I), monomers (M), and terminators (T) can form well over 300 million molecules, considering only I-T, I-M-T, and I-M-M-T! If I-M-M-M-T and higher oligomers are considered, the numbers quickly reach into the hundreds of millions. For demonstrating this concept, in the following a much smaller number of components and possible products is analyzed (6 initiators, 8 monomers, 9 terminators; around 30,000 compounds). Larger numbers of compounds are of course readily available by biological methods, but there is currently no synthetic method to prepare such large libraries from relatively few starting materials by simply mixing components in aqueous solution followed by direct biological assay. The proof of concept of this approach is given in the detailed description.

Generally speaking, the present invention thus relates to and proposes a method as claimed in claim 1, namely a method for the generation of oligomers or a mixture of oligomers to form a chemical library by amide-forming oligomerization. The proposed method comprises the following the steps, in the given order:
1) reacting a mixture of at least one initiator (I) with at least one monomer (M) to form a dimer (I-M) of the initiator (I) and the monomer (M) or to form a pre-oligomer (I-M-M, I-M-M-M; ...) with an initiator (I) attached to a chain of more than one monomer (M) or a mixture thereof by amide-bond formation;
2) adding at least one terminator (T) for the formation of a linear oligomer (I-M-T, I-M-M-T, I-M-M-M-T, ...) or a mixture of linear oligomers by amide-bond formation; or, for the formation of a cyclic oligomer or a mixture of cyclic oligomers by amide-bond formation, changing the reaction conditions relative to step 1) so as to form a linking covalent bond between the at least one initiator (I) and a monomer (M), preferably the respective terminal monomer, of the dimer or pre-oligomer formed in step 1).

In step 1) either one single type of dimer can be generated, if one single initiator is used, or if one single initiator is used combined with several different monomers, well-controlled set of dimers of the single initiator with in each case one of the different monomers. In case of corresponding reaction conditions, reactant concentrations, it's also possible to obtain, when using one single initiator, an oligomer with the initiator combined with a chain of the monomers. In case of using several initiators, it is possible to obtain corresponding mixtures of the different initiators attached to corresponding chains of monomers.

Correspondingly therefore the proposed reaction scheme is essentially free from side reactions, does not necessitate the sequential build-up of the chain, and can be controlled as concerns the distribution of the reaction products. It further allows for a well controllable, non-toxic reaction scheme to obtain either individual systems, or mixtures of systems in the sense of chemical libraries. By tailoring the distribution of the systems in a mixture of the corresponding chemical libraries can very efficiently be used in an incomplete factorial design approach to identify chemically and/or biologically active systems with a reduced separation/identification effort.

Preferably essentially enantiopure building blocks (for the initiator and/or the monomer and/or the terminator) are used.

It should further be noted that it is also possible to produce, concomitantly, linear as well as cycling systems, in that e.g. initiators for the generation of linear systems are used in a mixture with initiators for the generation of cycling systems, and in that the step 2) is carried out once for the determination of the linear systems and once for the ring closure of the cyclic systems.

The importance of producing a large array of compounds in a fast and cost-effective way is well recognized. The proposed method provides compound libraries efficiently and can be practiced routinely in industry and academia. The normally associated concerns like complicated experimental procedures, expensive stoichiometric reagents, the need for protection of common functional groups, the occurrence of false positives, costly waste handling and time-consuming purifications, the major obstacles for library synthesis, which necessitate the preparation and storage of pure, isolated compounds, can be avoided.

Herewith a self-assembly process for a β-peptide library synthesis is proposed that does not require reagents or protecting groups and operates under aqueous conditions. The resulting product mixtures can be screened directly in biological assays and the libraries can be rapidly modulated for optimization or deconvolution simply by changing the composition of the monomers or the addition order. The libraries can be quickly and reproducibly resynthesized as needed, rendering the need to isolate and purify each member obsolete.

The requisite building blocks are readily synthesized in enantiomerically pure form and are stable to prolonged storage. By simply mixing these building blocks and heating the reaction solutions in microplates, chemical libraries are synthesized "on-demand".

Libraries of oligomers with specific chemical and physical properties can be easily obtained by employing designed building blocks owing to the modular nature of the self-assembly process. In analogy to the powerful phage display method for peptide and protein library syntheses using natural 20 amino acids, the building blocks used here can be incorporated with various functional groups by straightforward syntheses and are tolerated in the mild self-assembly process conditions.

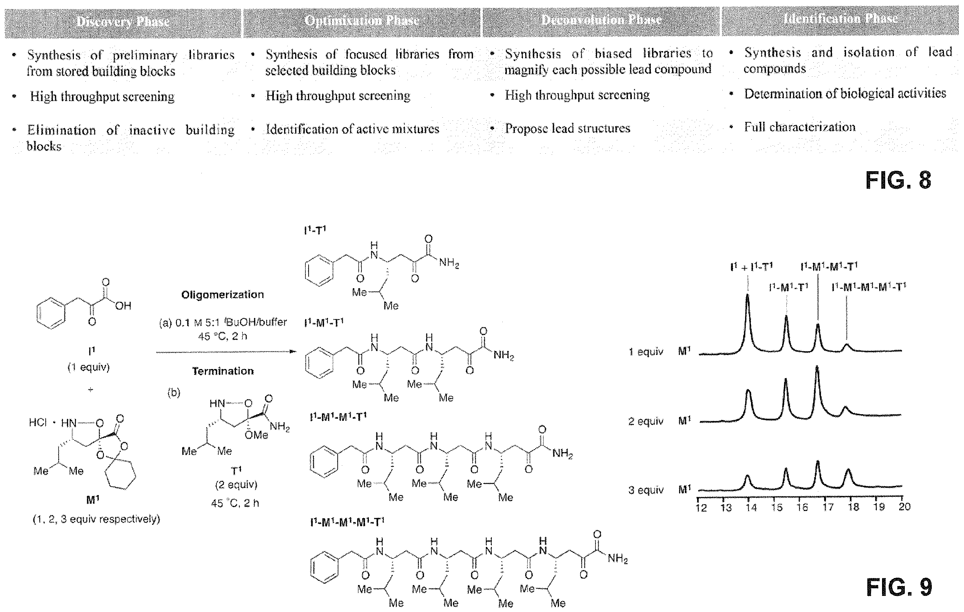

As a proof of concept given in the detailed description, HCV protease inhibitor libraries were synthesized. Our processes to find target molecules are divided into four phases (Fig. 8). (1) Discovery phase: various building blocks are engaged in library syntheses.

Components leading to inactive oligopeptides are eliminated. (2) Optimization phase: focused libraries are constructed from the selected moieties. Active mixtures are identified. (3) Deconvolution phase: the active wells in the focused libraries are deconvoluted and possible lead structures are proposed. (4) Identification phase: potential lead structures are further confined by HPLC separations. Syntheses, purification, and bioactivity determination of these compounds are carried out. Based on the preliminary lead structures, libraries with further optimized building blocks can be synthesized. With this method, a new HCV protease inhibitor with IC50 of 1.0 µM was isolated and fully characterized. Although this is only a modest inhibitor and the resulting molecule does not have ideal oral administration properties, the fact that it was discovered simply by combining a small number of monomers in aqueous conditions speaks to the promising method of lead identification inherit to this approach.

The proposed method can be applied in industry with the merit of low-cost operations and avoidance of hazardous organic chemicals. The aqueous-mediated library syntheses without involvement of purification steps minimize the amount of organic solvent waste.

The self-assembly reactions happen readily at moderate temperature and produce only innocuous by products without costly reagents and metal catalysts. Any laboratory familiar with biological sample handling can design and compose libraries from the constituent building blocks. The method can be enhanced by employing automated microfluidic devices. With stored building blocks, library syntheses can be programmed by automated systems. This reduces the amount of starting materials, ensures the accuracy of liquid handling and allows a high-throughput synthesis process. The freshly prepared libraries are ready for biological assessments directly without further treatment and also avoid the unforeseen decomposition problem with old stored compound libraries. In conclusion, a new method to rapidly synthesize chemical libraries is proposed. This method provides an alternative conceptual operation in compound library generations for various purposes. A large number of β-peptides with various side chains can be generated in a one-pot fashion by the chemoselective reaction between α-ketoacids and isoxazolidine building blocks. The resulting libraries were subjected to HCV protease assays directly without further purification. Strategies for deconvolution and optimization were presented. A new HCV protease inhibitor was identified by this method.

Specifically, preferably the **initiator (I)** is selected from the group consisting of: 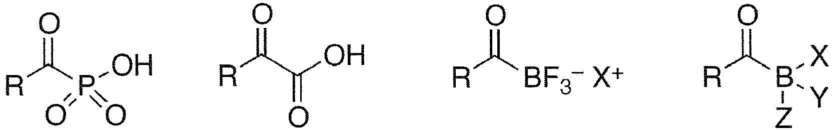 with, for the formation of a linear oligomer or a mixture of linear oligomers by amide-bond formation, R being selected from the group consisting of: substituted or unsubstituted alkyl, heteroalkyl, cycloalkyl, heterocycloalkyl, aryl, heteroaryl, alkylaryl, heteroalkylaryl, alkenyl, heteroalkenyl, alkinyl, heteroalkinyl, -C(NH₂)R^{I}, -C(NH₂){R^{I}-CO-C(NH₂)}ₙR^{I} with n=1, 2 and R^{I} being H or an amino-acid side chain, fluorescent dye, nucleic acid or derivative thereof, peptide nucleic acid, FLAG octapeptide (DYKDDDDK), biotin or affinity tag. Preferably the residue R comprises in the range of 1-10 carbon atoms, more preferably it is selected from the group consisting of benzyl, NO₂ substituted benzyl, ethyl, carboxyethyl, hexyl, tert. Butyl.

For the formation of a cyclic oligomer or a mixture of cyclic oligomers by amide-bond formation, preferably R is selected from the group consisting of: -{(CH₂)}ₙR^{c},-{(CHCH₃)}ₙR^{c}, -{(CH(1,1-dimethylethyl))}ₙR^{c}, -{(CH(benzyl))}ₙR^{c}, in each case with n=1,2 and R^{c} being a linker structure allowing to form a linking covalent amide bond to the respective terminal monomer of the dimer or oligomer formed in step 1).

In both cases (linear and cyclic oligomer formation) the following definitions preferably apply:
X+ is a counterion, selected from the group consisting of: K⁺, Cs⁺, Li⁺, Na⁺, R₄N⁺, R₄P⁺ or R₃S⁺ with R being an organic substituent or H, preferably selected from the group consisting of: substituted or unsubstituted alkyl, heteroalkyl, cycloalkyl, heterocycloalkyl, aryl, heteroaryl, alkylaryl, heteroalkylaryl, alkenyl, heteroalkenyl, alkinyl, heteroalkinyl, preferably the residue R comprises in the range of 1-10 carbon atoms;
X, Y, Z, are, independently from each other, selected from the group consisting of: F, OR, N⁺R₃, N⁺R₂OR, N⁺R₂SR, and N⁺R₂NR₂, and are optionally forming a cyclic or a bicyclic structure; wherein R is an organic substituent or H, preferably selected from the group consisting of: substituted or unsubstituted alkyl, heteroalkyl, cycloalkyl, heterocycloalkyl, aryl, heteroaryl, alkylaryl, heteroalkylaryl, alkenyl, heteroalkenyl, alkinyl, heteroalkinyl, preferably the residue R comprises in the range of 1-10 carbon atoms.

According to a preferred embodiment, the initiator (I) for the formation of a linear oligomer or a mixture of linear oligomers by amide-bond formation is selected from the group consisting of: 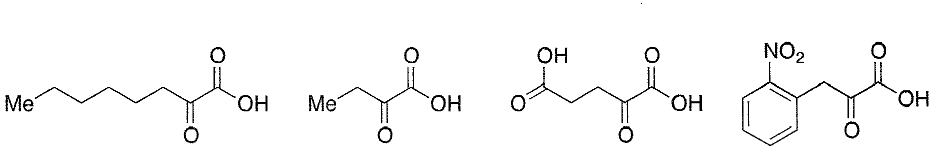 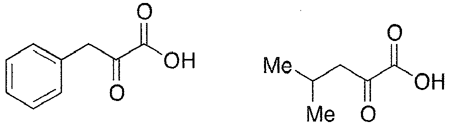 wherein Me= -CH₃.

According to yet another preferred embodiment, the initiator (I) for the formation of a cyclic oligomer or a mixture of cyclic oligomers by amide-bond formation is selected in that the linker structure is selected from the group consisting of: a chain of one or two elements selected from the group of: amino acid, -CO((CH₂)₂NH-, and this chain preferably terminated by a group selected from:

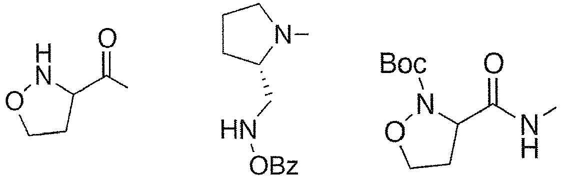

One of these groups can also be directly, so without an element as outlined in the paragraph before, the linker element, so can be the residue R of the initiator as defined above.

Further preferably, the initiator (I), also but not necessarily, for the formation of a cyclic oligomer or a mixture of cyclic oligomers by amide-bond formation can be given by covalent dimers or trimmers of the above structures, in which case the residue R is a common linker element. Possible are thus structures comprising at least two initiator moieties selected from the group consisting of: 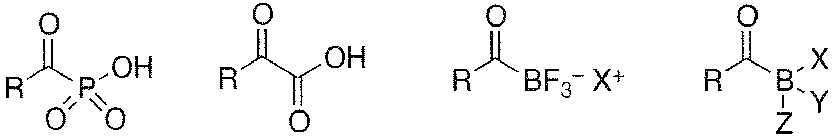 with
- R: being a linker element
- X⁺: being a counterion, selected from the group consisting of: K⁺, Cs⁺, Li⁺, Na⁺, R₄N⁺, R₄P⁺ or R₃S⁺ with R being an organic substituent or H;
- X, Y, Z,: being, independently from each other, selected from the group consisting of: F, OR, N⁺R₃, N⁺R₂OR, N⁺R₂SR, and N⁺R₂NR₂, and are optionally forming a cyclic or a bicyclic structure; wherein R is an organic substituent or H;
linked by said linker element.

Such dimers or trimers for the initiator (I) can be of the following general structure 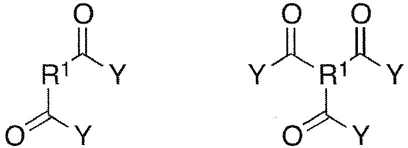 wherein
- R¹: is a linker element;
- Y: being selected from the group consisting of: -PO₃H, -COOH, -BF₃⁻X⁺, -BXYZ, wherein X⁺, X, Y, Z are defined as given above in the context of the initiator (I).

The initiator (I) for the formation of a cyclic oligomer or a mixture of cyclic oligomers by amide-bond formation can be given by a structure comprising at least one such terminator moiety as defined further below and at least one such initiator moiety as given just above.

Generally speaking, preferably the initiator (I) for the formation of a cyclic oligomer or a mixture of cyclic oligomers can be given by a structure consisting of a hydroxylamine at one end a ketoacid or acylboronate at the other end.

Preferably the initiator (I) for the formation of a cyclic oligomer or a mixture of cyclic oligomers by amide-bond formation is selected from the group consisting of: 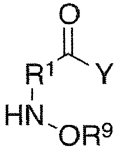 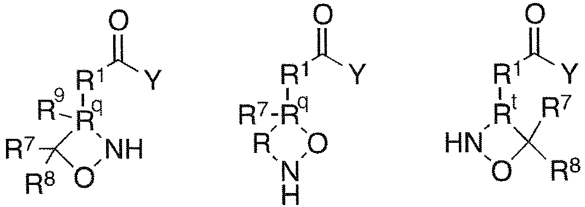 wherein
- R¹: is a linker element;
- R^{q}: is a structural element complementing to a 4, 5, 6, or 7 membered ring, preferably selected from the group consisting of: -C-, -CH₂-C-, -CHR^{T}-C-, -(CH₂)₂-C-,-(CHR^{T})₂-C-, -(CH₂)-C-(CH₂)-, -(CHR^{T})-C-(CH₂)-, -(CHR^{T})-C-(CHR^{T})-, -(CH₂)₃-C, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
- R^{t}: is a structural element complementing to a 4, 5, 6, or 7 membered ring, preferably selected from the group consisting of: -CR^{U}-, -CH₂-CR^{U}-, -CHR^{T}-CR^{U}-, -(CH₂)₂-CR^{U}-, -(CHR^{T})₂-CR^{U}-, -(CH₂)-CR^{U}-(CH₂)-, -(CHR^{T})-CR^{U}-(CH₂)-, -(CHR^{T})-CR^{U}-(CHR^{T})-, -(CH₂)₃-CR^{U}, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, and wherein R^{U} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
- R⁷-R⁹: being, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, ester, carbamate, sulfonate, sulfinate, phosphate, silyl, as well as cyclic forms linking these among each other and carbonyl, imidate, thiomidate,
- R: being selected from the group consisting of: O, S, NR₁, SiR₁R₂, CR₁R₂; wherein R₁ and R₂ are, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl
- Y: being selected from the group consisting of: -PO₃H, -COOH, -BF₃⁻X⁺, -BXYZ, wherein X⁺, X, Y, Z are defined as given above in the context of the initiator (I).

According to yet another preferred embodiment, the initiator (I) for the formation of a cyclic oligomer or a mixture of cyclic oligomers by amide-bond formation is selected from the group consisting of: 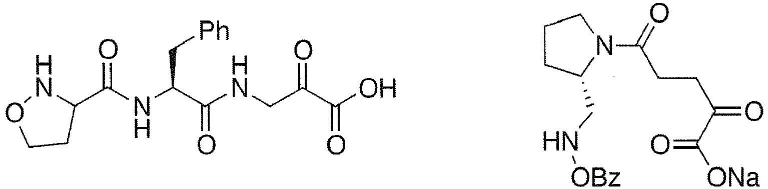 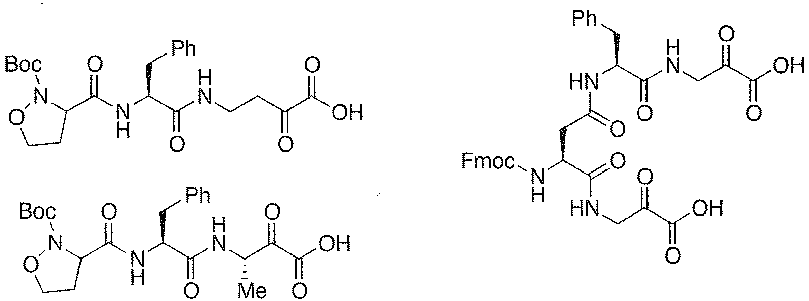 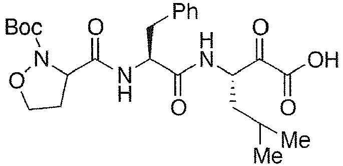 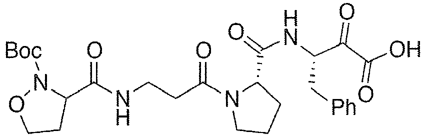 wherein boc=tert-butyloxycarbonyl, Ph=phenyl, Bz=benzyl, Fmoc= fluorenylmethyleneoxycarbonyl, Me=-CH₃.

The **monomer (M)** is preferably selected from the group consisting of: 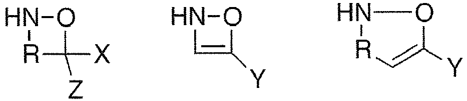 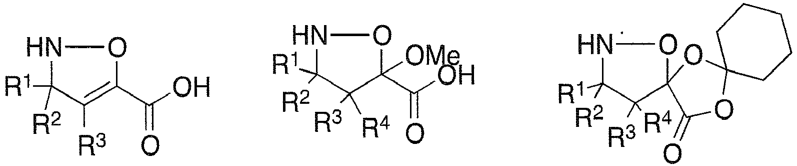 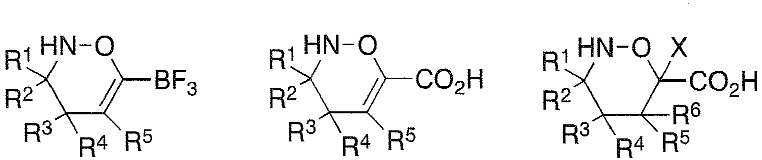 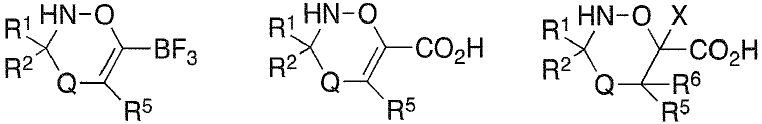 with preferably the following definitions:
- X: is selected from the group consisting of: halogen, -OH, -COOH, -NH₂,-O-Alkyl (e.g. -OEt, -OiPr, -OBn), -O-Aryl (e.g. -OPh, -OBz), -O-CO-Alkyl, -O-CO-Aryl,-SH, S-Alkyl (e.g. -S-Me), -S-Aryl (e.g. -S-Ph), N-Acyl, -NH-Alkyl, -NH-Aryl,-N(Alkyl)₂, -N(Aryl)₂, -N(Alkyl)(Aryl), -CO-NH-Alkyl, -CO-NH-Aryl, -CO-N(Alkyl)₂, -CO-N(Aryl)₂, -CO-N(Alkyl)(Aryl),, -CN, -NO₂, -N₃, -S(O)Aryl,-S(O)₂Aryl;
- Y: is selected from the group consisting of: -PO₃H, -COOH, -BF₃⁻X⁺, -BXYZ, wherein X⁺, X, Y, Z are defined as given above in the context of the initiator (I),
- Z: is selected from the group consisting of: -PO₃H, -COOH, -BF₃⁻X⁺, -BXYZ, wherein X+, X, Y, Z are defined as given above in the context of the initiator (I), as well as derivatives thereof which upon collapse of X and Z upon cleavage of the NO bond lead to Y;
- R: is selected from the group consisting of: O, S, NR¹, Si, CHR¹R² (R¹ and R² being defined as in the definition of R¹-R⁸ below);
- R¹-R⁸: are, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, as well as cyclic forms linking these among each other, wherein preferably the residue comprises in the range of 1-20 carbon atoms;
- Q: is selected from the group consisting of: O, S, Si, NR¹¹, where R¹¹ is an organic substituent or H, preferably defined as in the definition of R¹-R⁸ above.

Preferably, the monomer is selected from the following group, with the definitions as given just above:

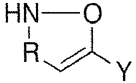

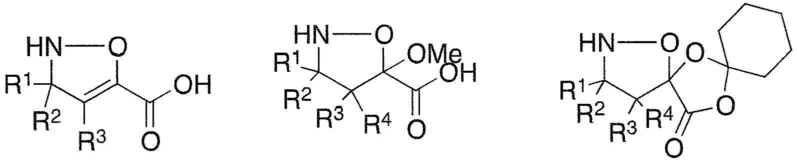

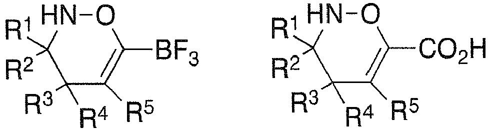

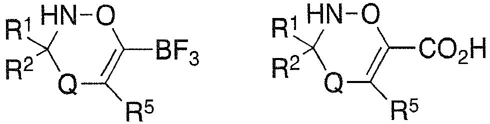

As for the monomer of the type:

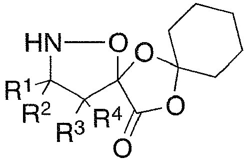

The methods of how to synthesize them are outlined in detail in the experimental section.

As for the other monomers of the above group, represented with numbering as follows: 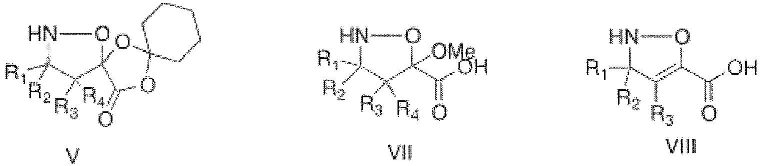 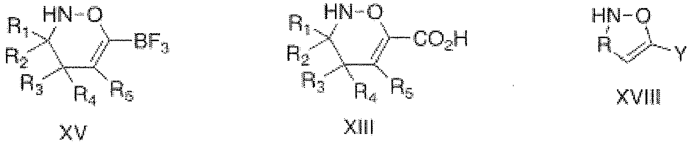 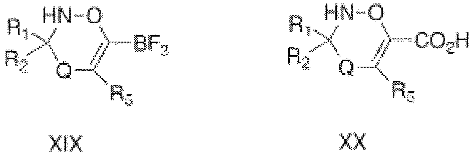 the following synthetic routes are readily available to the skilled person:
A monomer of formula (V) can be prepared in an analogous manner to that described in Org. Process. Res. Dev., 2012, 16, 687-696, as shown in the following Scheme:

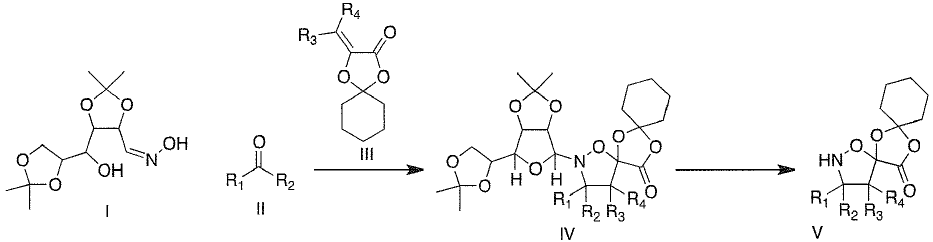

A monomer of formula (VII) can be prepared in an analogous manner to that described in Helv. Chim. Acta, 2012, 95, 2481, as shown in the following Scheme:

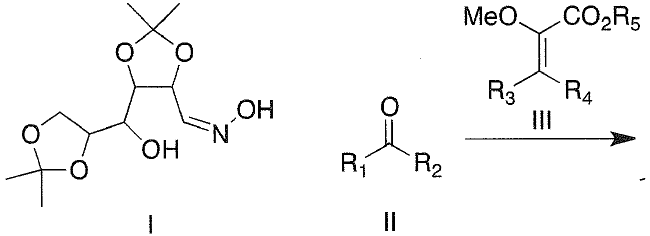

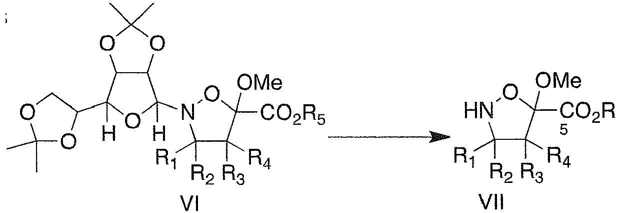

A monomer of formula (VIII) can be prepared, as shown in the following scheme, by treating a compound of formula (VII) with a suitable base, such as NaOMe, in a suitable solvent, such as methanol:

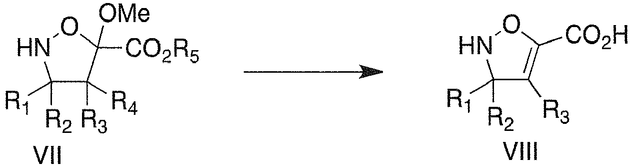

A monomer of formulae (XIII) can be prepared as shown in the following Scheme. By treating a compound of formula (IX) with a reducing agent, such as SmI₂, in a suitable solvent such as methanol/tetrahydrofuran, to give a compound of formula (X). Treatment of a compound of formula (X) with a carboxylic acid activating agent, such as Ac₂O/AcONa or DCC, in a suitable solvent such as dichloromethane, will give a compound of formula (XI). Conversion of a compound of formula (XI) to a compound of formula (XII) can be carried out either using a triflating agent, such as Tf₂O or PhNTf₂, and a suitable base, such as Et₃N or KHMDS, in a suitable solvent, such as tetrahydrofuran and DMPU. Treatment of a compound of formula (XII) with CO in the presence of a suitable catalyst, such as Pd(PPh₃)₄, and a suitable base, such as Et₃N, in a suitable solvent such as DMF, could give a compound of formula (XV).

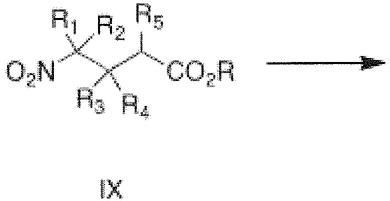

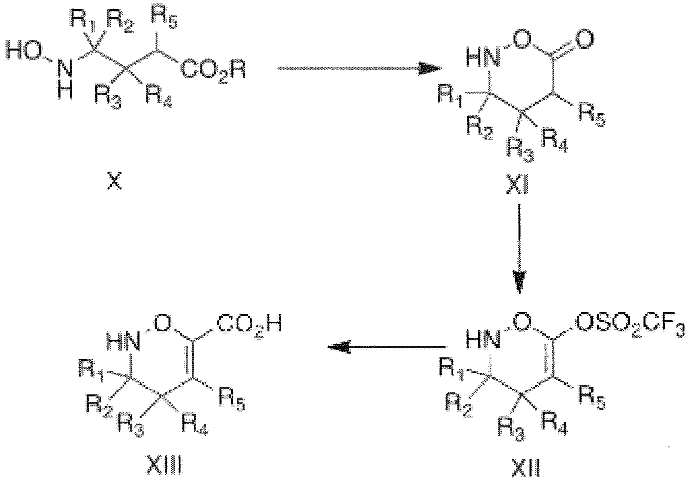

A monomer of formula (XIV) can be prepared by treating a compound of formula (XII) with a suitable catalyst, such as PdCl₂dppf, and a suitable boron reagent, such as B₂(pin)₂, in the presence of a suitable base, such as AcOK, and in a suitable solvent, such as 1,4-dioxan, at elevated temperature (see Scheme below). Further treatment of XIV with a suitable fluorinating agent, such as KHF₂, in a suitable solvent, such acetone/water, will give rise to a monomer of formula (XV):

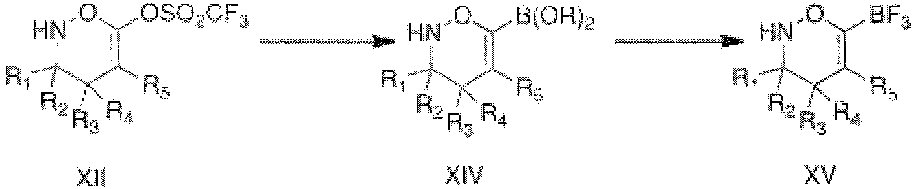

Monomers of formulae (XIX) and (XX) can be prepared in an analogous manner to monomers (XV) and (XIII) respectively, as described above.

A monomer of formula (XVIII), where R is e.g. CR₁R₂, can be prepared by a cycloaddition reaction between a compound of formula (XVI) and a compound of formula (XVII), in a suitable solvent, such as xylene (see Scheme below). Interconversion of the Y functionality can be carried out using a method known to those skilled in the art.

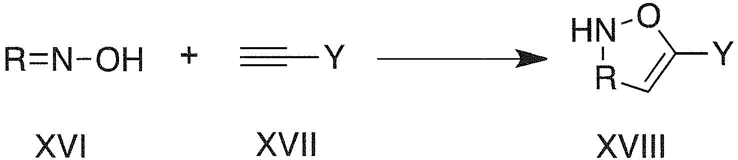

According to a preferred embodiment, the monomer (I) is selected from the group consisting of:

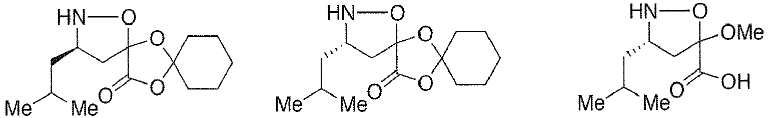

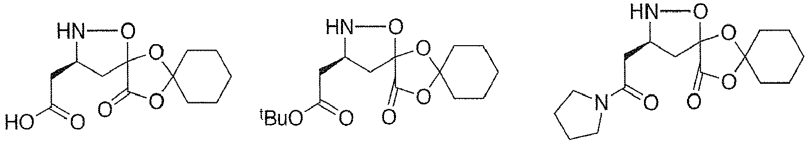

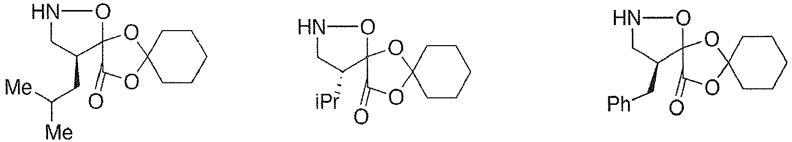

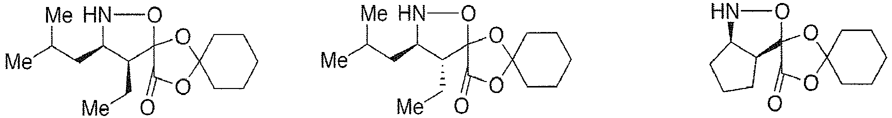

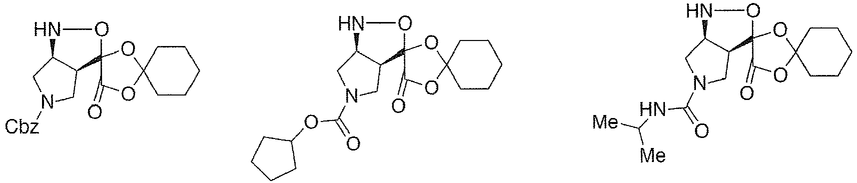

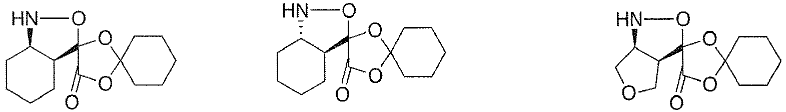

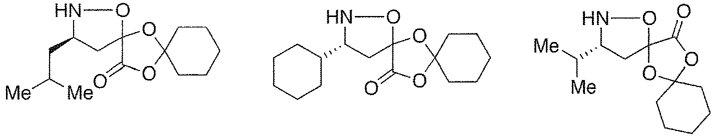

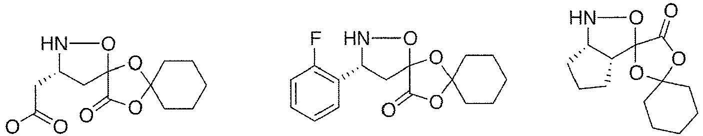

Wherein Me = -CH₃, ^{t}Bu= 1,1-dimethylethyl, Cbz=benzyloxycarbonyl, iPr=isopropyl, Ph=phenyl.

The **terminator (T),** if used, so in case of linear products, is selected from the group consisting of: 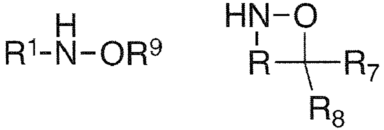 with preferably the following definitions:
- R: is selected from the group consisting of: CH₂, (CH₂)₂, CHRT, CH₂CHR^{T}, (CH₂)₃, (CH₂)₂CHR^{T}, CH₂CHR^{T}CH₂, (CH₂)₄, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, wherein preferably the residue comprises in the range of 1-20 carbon atoms, more preferably in the range of 1 - 10 carbon atoms;
- R¹: is selected from the group consisting of: substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, ester, carbamate, sulfonate, sulfinate, phosphate, silyl, wherein preferably the residue comprises in the range of 1-20 carbon atoms, more preferably in the range of 1 - 10 carbon atoms;
- R⁷-R⁹: are, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, ester, carbamate, sulfonate, sulfinate, phosphate, silyl, as well as cyclic forms linking these among each other and carbonyl, imidate, thiomidate.

According to a preferred embodiment, the terminator (T) is used in excess, and furthermore preferably it is selected from the group consisting of: 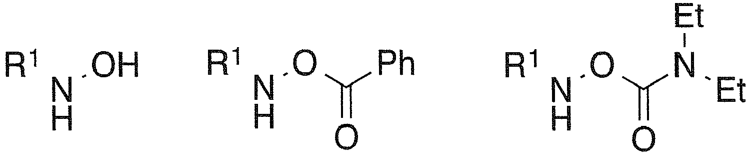 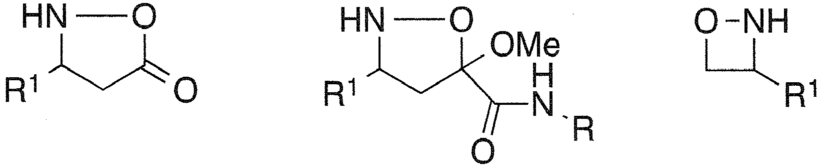 Possible are in particular also structures for the terminator (T) of the type 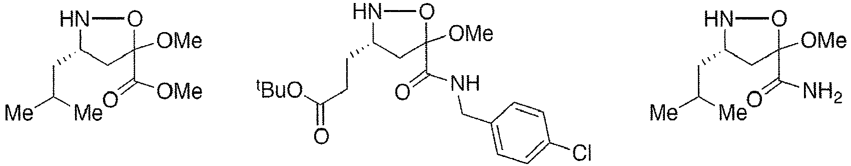 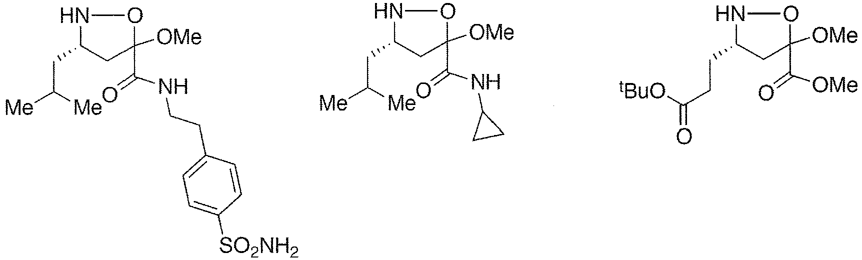 with Me= -CH₃, ^{t}Bu= 1,1-dimethylethyl.

Alternatively, the terminator (T), also but not necessarily for the formation of a cyclic oligomer or a mixture of cyclic oligomers by amide-bond formation, can be given by covalent dimers or trimers of the above structures, in which case then R¹ and R⁷ or R⁸ are common linker elements. So the terminators can also be given by a structure comprising at least two terminator moieties selected from the group consisting of: 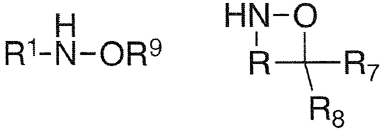 with
- R: being selected from the group consisting of: CH₂, (CH₂)₂, CHR^{T}, CH₂CHR^{T}, (CH₂)₃, (CH₂)₂CHR^{T}, CH₂CHR^{T}CH₂, (CH₂)₄, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
- R¹: being a common linker element;
- R⁷-R⁹: being, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, ester, carbamate, sulfonate, sulfinate, phosphate, silyl, as well as cyclic forms linking these among each other and carbonyl, imidate, thiomidate, with the proviso that at least one of R⁷ or R⁸ is a common linker element.

So dimeric or trimeric structures of the following type are possible for the terminator (T): 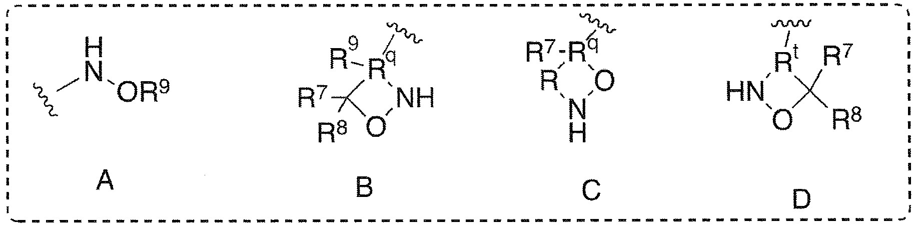 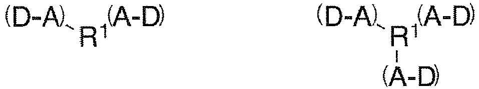 wherein
- R¹: is a linker element;
- R^{q}: is a structural element complementing to a 4, 5, 6, or 7 membered ring, preferably selected from the group consisting of: -C-, -CH₂-C-, -CHRT-C-, -(CH₂)₂-C-,-(CHR^{T})₂-C-, -(CH₂)-C-(CH₂)-, -(CHR^{T})-C-(CH₂)-, -(CHR^{T})-C-(CHR^{T})-, -(CH₂)₃-C, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
- R^{t}: is a structural element complementing to a 4, 5, 6, or 7 membered ring, preferably selected from the group consisting of: -CR^{U}-, -CH₂-CR^{U}-, -CHR^{T}-CR^{U}-, -(CH₂)₂-CR^{U}-, -(CHR^{T})₂-CR^{U}-, -(CH₂)-CR^{U}-(CH₂)-, -(CHR^{T})-CR^{U}-(CH₂)-, -(CHR^{T})-CR^{U}-(CHR^{T})-, -(CH₂)₃-CR^{U}, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, and wherein R^{U} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
- R⁷-R⁹: being, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, ester, carbamate, sulfonate, sulfinate, phosphate, silyl, as well as cyclic forms linking these among each other and carbonyl, imidate, thiomidate,
- R: being selected from the group consisting of: O, S, NR₁, SiR₁R₂, CR₁R₂; wherein R₁ and R₂ are, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
- (A-D): or (D-A), respectively, indicating one of the possible structures illustrated in the dashed box,

According to a preferred embodiment, one single initiator (I) can be used in step 1) and, in case of the formation of a linear oligomer or a mixture of linear oligomers by amide-bond formation, one single terminator (T) can be used in step 2).

Step 1) and/or 2) can be carried out, apart from solvent(s), without any added further chemical reagents or catalysts, which simplifies the procedure and reduces toxicity issues. In step 1) and/or 2) organic solvents, water, aqueous buffer or combinations thereof can be used.

According to yet another preferred embodiment, in step 1) more than 1, preferably 2-6, more preferably 2-4 different monomers can be used.

In step 1) the reaction can be carried over to lead to oligomers with at least 2 interlinked monomers, preferably in the range of 2-10, more preferably in the range of 2-6 interlinked monomers.

In step 1) the reaction conditions, preferably temperature and/or pressure, and/or reactant concentrations and/or reactant addition order and/or reactant addition time can be selected so as to lead, between different batches, to targeted different distributions of different oligomers in the mixture.

In step 1) one single initiator (I), one single monomer (M) and, in case of the generation of a linear oligomers, in step 2) one single terminator (T) can be used, and the reaction conditions in step 1) and/or step 2) can be adapted such as to form a specific trimer structure.

Furthermore the present invention relates to a method of identification of biologically and/or chemically active systems from a chemical library preferably based on at least one mixture of oligomers made using a method as outlined above, wherein preferably the mixtures of oligomers are screened for activity prior to purification or separation of the compounds from the mixture.

According to a preferred embodiment of this method, a number of specifically differing mixtures made using a method as outlined above can be used, checking these mixtures for biological and/or chemical activity, inferring from activity patterns initiators and/or monomers and/or terminators inducing activity, optionally preparing further mixtures using a method as outlined above based on the identified active initiators and/or monomers and/or terminators only, thereby successively reducing the number of possible active oligomers. Like this the method can be very efficiently used in an incomplete factorial design screening process.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows the chemoselective couplings of α-ketoacid initiators (I) and isoxazolidine monomers (M); (A) iterative synthesis of β-peptides; (B) one-pot chemical library synthesis; (C) possible products expand dramatically with increasing numbers of building blocks;
- Fig. 2: shows HPLC traces of product distributions with building blocks, I¹ (1.0 equiv), M¹ (0.50 equiv), M² (0.50 equiv) and T¹ (2.0 equiv), while M¹ and M² were added at different order; (A) M¹ was added first; (B) M² was added first;

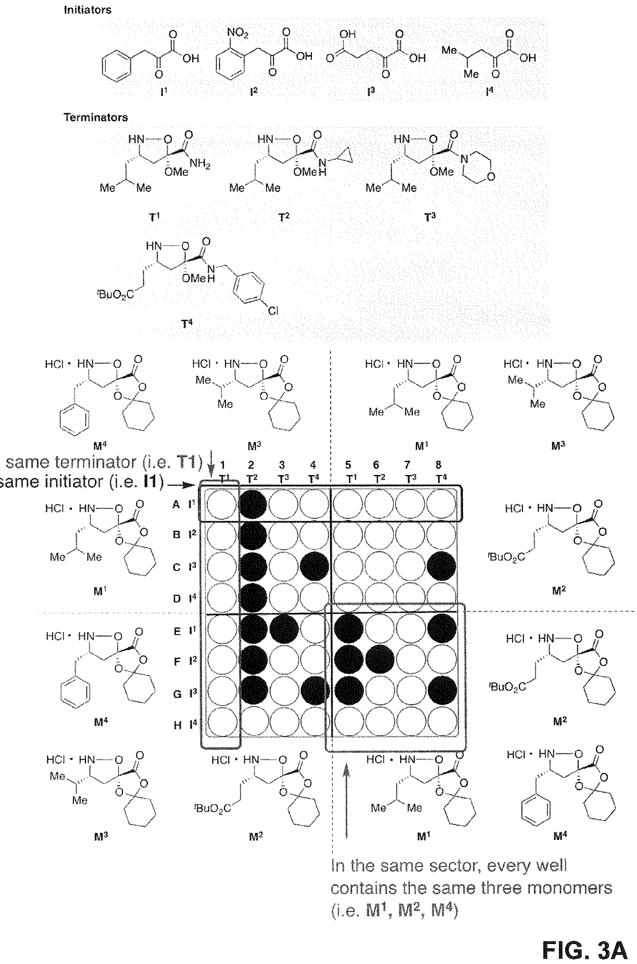

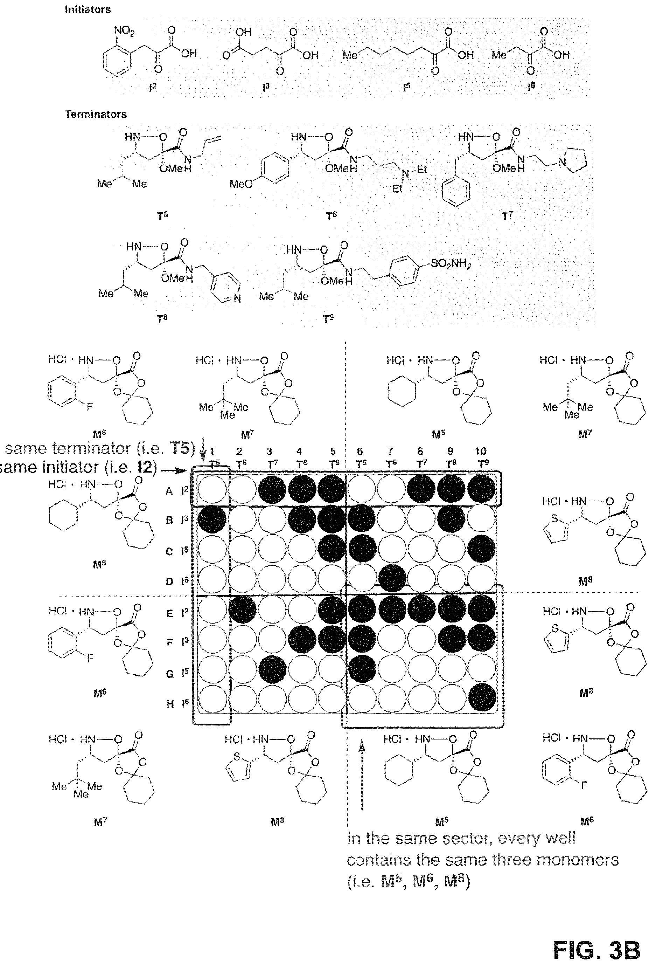

- Fig. 3: shows preliminary libraries, library 1 (A) and library 2 (B); each well contained one initiator (1.0 equiv), three monomers (total 2.0 equiv) and one terminator (2.0 equiv); row and column had its assigned specific initiator/terminator; for example in library 1 (A), A1-A8 wells contained initiator I¹ and A3-H3 wells had terminator T₃; each preliminary library was divided into four sectors and each sector differed only in monomer mixtures: three out of four types of monomers; for example in library 1 (A), well A1 had initiator I¹, monomer M¹ M³ M⁴ and terminator T¹ while well E5 had initiator I¹, monomer M¹ M² M⁴ and terminator T¹; active wells are presented dark (for selection standard, see description of the preferred embodiments);
- Fig. 4: shows a focused library, library 3; in sector 1, each well contained the assigned initiator (1.0 equiv), monomer M² M⁴ M⁵ (total 2.0 equiv) and the assigned terminator (2.0 equiv); after the oligomerization and termination were complete, sector 1 was 10-fold diluted to give sector 2; active wells are presented dark (for selection standard, see description of the preferred embodiments);
- Fig. 5: shows deconvolution library 1 and 2, library 4 and 5; active wells are presented dark (Among the active wells in rows B-E, wells with the best results are highlighted (for selection standard, see description of the preferred embodiments);
- Fig. 6: shows HPLC traces and the fluorescence read outs from the HCV assay of well A5, C5 and E4 in library 5; the fraction at 27.5 min was active in the HCV assay;
- Fig. 7: shows the synthesis of the lead compound I³-M⁵-M⁵-M⁵-T⁴ in a) and in b) HPLC trace of this compound and its dose-response curve to HCV protease; the compound inhibits HCV protease with an IC50 of 1.0 µM;
- Fig. 8: shows a possible standard operating procedure for "on-demand" chemical library synthesis;
- Fig. 9: shows HPLC traces of product distributions with building blocks, I¹ (1.0 equiv), M1 (1.0, 2.0, 3.0 equiv respectively) and T¹ (2.0 equiv); and
- Fig. 10: illustrates the experimental procedures of library synthesis.

### DESCRIPTION OF PREFERRED EMBODIMENTS

As a proof of concept, the "on-demand" library synthesis was used to prepare and screen inhibitors of HCV protease. The library syntheses were performed directly in 96-well plates. In contrast to the conventional "one well-one-compound" technique, each well, containing a mixture of products, was diluted and screened directly.

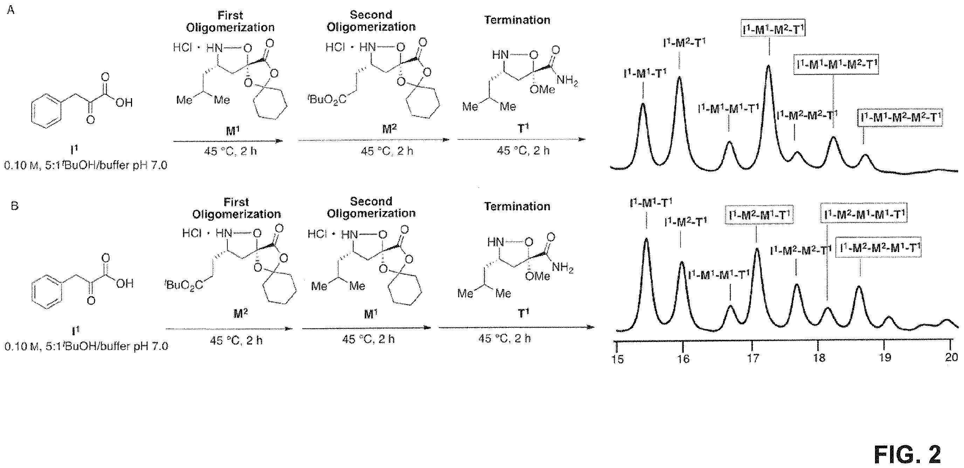

Active compounds were identified by simple deconvolution strategies. This method provides a rapid and simple way to generate protease inhibitor libraries and to identify the potent leads. Two initial experiments to study the reactivity of monomers (M) and terminators (T) were performed. First, we determined if the product distributions reflected the quantities of monomers used in the self-assembly process. One equivalent of monomer M¹ produced I¹-M¹-T¹ as the major product. The product distributions shifted to higher oligomers when more M¹ was used (see further below). By simply varying the quantities of monomers, the distribution of products could be altered. The reproducibility of the results was confirmed by repetitions, as well as reactions involving different monomers. Second, we demonstrated that the product sequences could be biased by addition order. An experiment with M¹ and M² added in different order was performed. In Fig. 2A, M¹ was added to react with initiator I¹ for 2 h, followed by addition of M² and finally termination with T¹. Among I¹-M-M-T¹ products, I¹-M¹-M²-T¹ was found to be the main product while I¹-M²-M¹-T¹ was not detected. Higher order products I¹-M¹-M²-M²-T¹ and I¹-M¹-M¹-M²-T¹ were also formed. Products were isolated and characterized by MS/MS. These results showed that when a product was constituted of both M¹ and M², M¹ was incorporated before M² as it was added first. The same trend was observed in the experiment when M² was added first (Fig. 2B) with product distributions considerably changed when M¹ and M² were added in a different order.

Our finding that product distributions could be systematically controlled by changing monomer quantities and the addition order allowed us to predict the major products in the reaction mixture when more types of monomers were involved. This self-assembly method was applied to protease inhibitor library syntheses and a commercially available HCV protease assay was chosen for screening. HCV protease lacks a well-defined pocket in its active site. The major interaction between the reported inhibitors and the shallow enzyme pocket is a reversible covalent bond formation from nucleophilic attack of the protease serine of the catalytic triad to the α-ketoamide on the inhibitors and hydrophobic interactions with the rest of the molecules. Two preliminary libraries (library 1 and 2) of a total size ∼6,000 compounds (∼40 compounds/well) were synthesized and screened. Based on the reported inhibition mechanism, both monomers and terminators used had hydrophobic side chains and the crucial α-ketoamide functional group was produced in the termination step.

The arrangement of initiators, monomers and terminators in preliminary libraries is shown in Fig. 3: (1) Row/column had its assigned specific initiator/terminator. Wells containing active initiators and terminators were recognized based on the highest occurrence from the active wells. For example, in library 1, initiator I¹ I³ and terminator T² T⁴ were identified. (2) Each library was divided into four sectors and wells in the same sector had the assigned three out of four monomers. The same approach can be used with a larger number of monomers, initiators or terminators, thereby increasing the number of possible products that can be formed in each well. This setting was designed to facilitate the monomer selection processes. The three monomers in the sector with the least numbers of active wells were considered inactive and eliminated. For example, in library 1 sector containing M¹ M² M³ but not M⁴ had the least active wells. This benzyl monomer M⁴ was selected for further studies. In library 2, initiator I² I³ I⁵, monomer M⁵ and terminator T⁵ T⁸ T⁹ were selected according to the same principle. By this evaluation standard, we were able to quickly eliminate the poorly performing moieties and select building blocks for the following focused library.

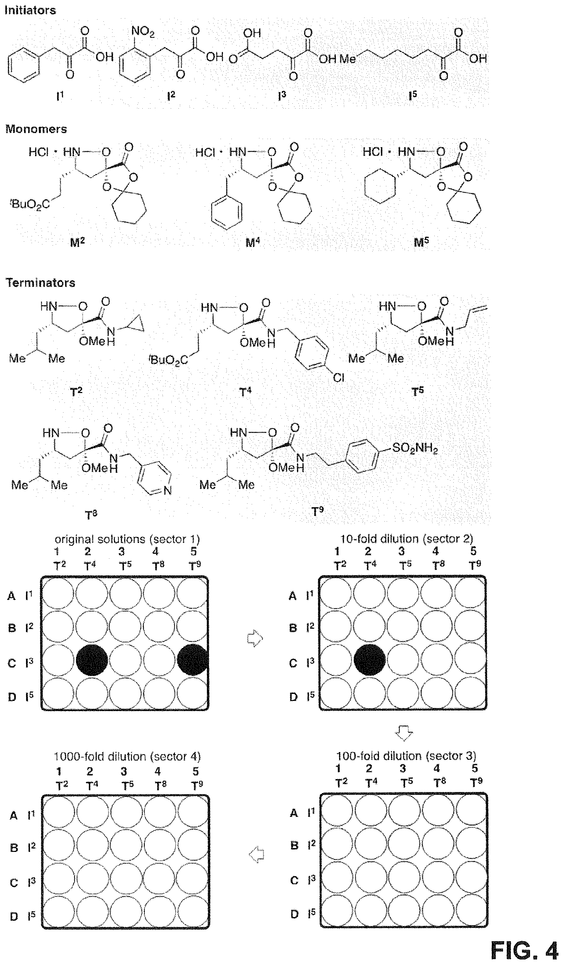

Selected initiator I¹ I² I³ I⁵, monomer M² M⁴ M⁵ and terminator T² T⁴ T⁵ T⁸ T⁹ were included in a 5 × 4 well focused library (library 3, sector 1). (Both M¹ and M² were the second best monomers in library 1, but M² had an ester functionality, which could provide some varieties to the monomer selection, and was therefore preferred over M¹). The selection was made on the basis of a biochemical assay, in this case inhibitor of HCV protease as determined by a commercially available kit using a fluorescent substrate. The size of this focused library was limited to ∼800 compounds. The library arrangement is shown in Fig. 4. After the library was synthesized, it was diluted to make sector 2 (10-fold dilution). Well C2 showed positive results both in the original and in 10-folddiluted concentration while C5 showed reactivity only at the original concentration; well C² was therefore chosen for deconvolution. To simplify the deconvolution of well C2 in library 3, which contained initiator I³, monomer M² M⁴ M⁵, and terminator T⁴, we assumed that if the active compound was constituted with the inclusion of three molecules of the monomer, reaction wells containing only one or two equivalents of monomers were less active.

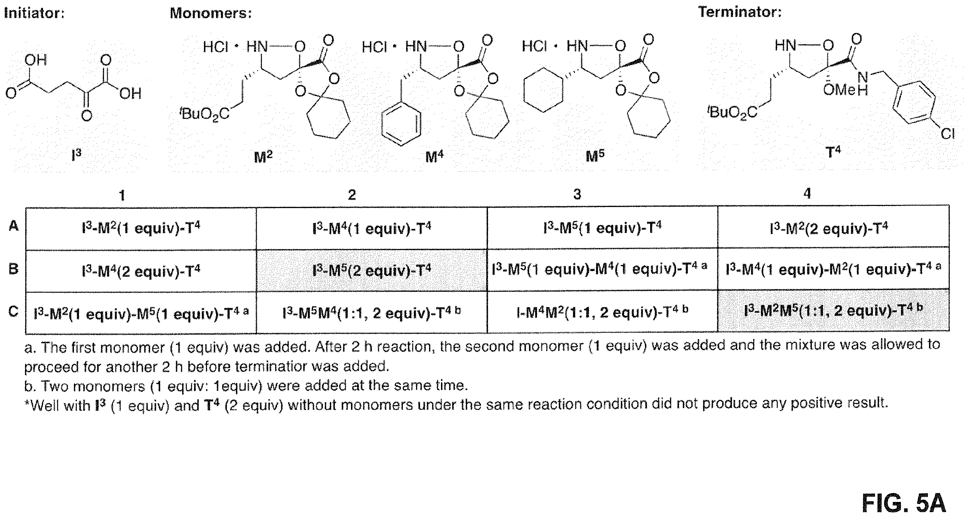

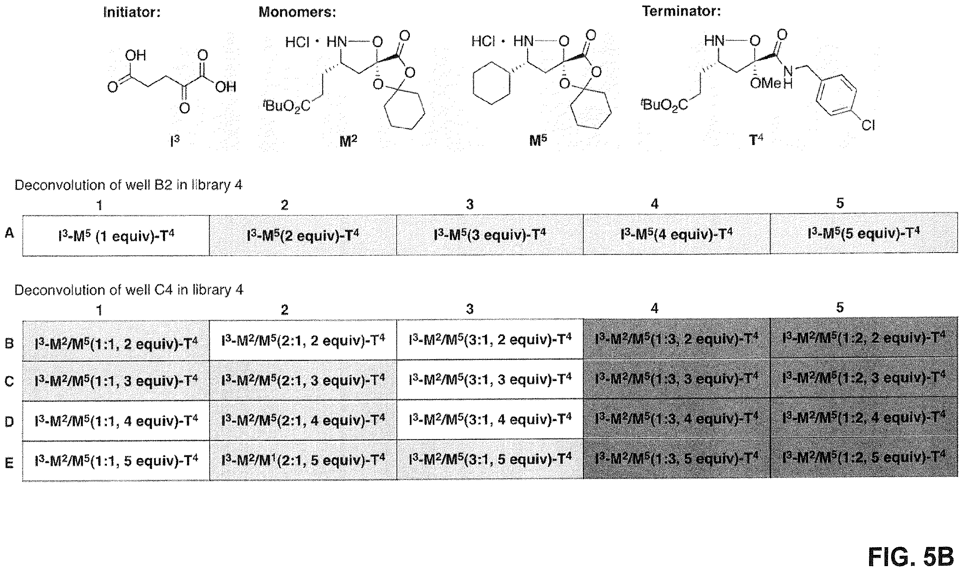

Based on this assertion, library 4 was synthesized. The library setting is shown in Fig. 5: (1) Well A1, A2, A3, A4, B1 and B²: each well contained only one type of monomers. Well A1 and A4 both had monomer M² but in different quantity to cover the different range of product distribution. Well A2/B1 and A3/B2 shared the same principle. (2) Well B3, B4, C1: wells consisted of two types of indicated monomers added at different order. (3) Well C2, C3 and C4: as in (2), but monomers were added at the same time. Comparing the overlapping/non-overlapping products of wells in (2) and (3), possible lead structures could be further limited. From the HCV assay result, well B2 and C4 showed activity. The active compound in B2 could be I³-M⁵-M⁵-T⁴ or I³-M⁵-M⁵-M⁵-T⁴. Because there was no inhibition shown in C1, the active molecule in C4 is most likely to be I³-M⁵-M⁵-T⁴, I³-M⁵-M²-T⁴, I³-M⁵-M⁵-M⁵-T⁴, I³-M⁵-M²-M²-T⁴ or I³-M⁵-M⁵-M²-T⁴. Further deconvolution of these confined lead candidates was performed. Different quantities of monomer M⁵ were used to deconvolute the active compounds in well B2 in library 4. In library 5 row A (Fig. 5), positive results were observed with wells containing more than one equivalent of M⁵.

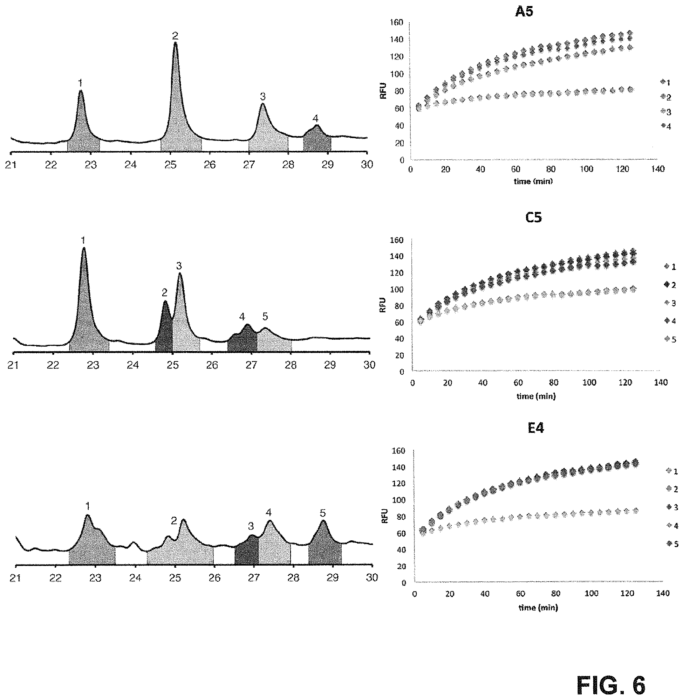

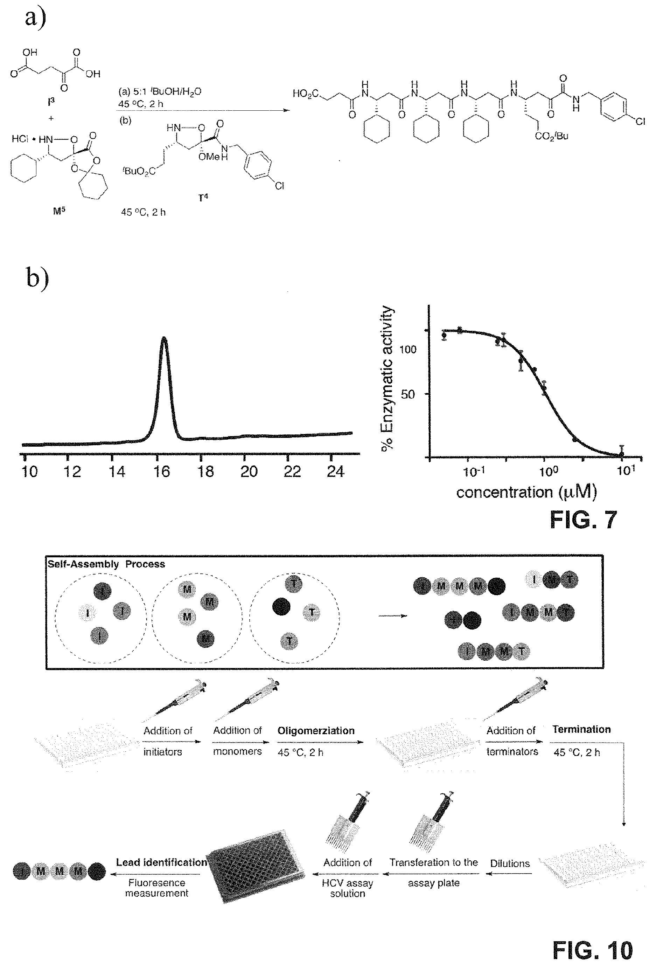

Between I³-M⁵-M⁵-T⁴ and I³-M⁵-M⁵-M⁵-T⁴, I³-M⁵-M⁵-M⁵-T⁴ was more likely to be the inhibitor. Rows B-E were designed to deconvolute C4 well in library 4. M⁵ and M² were added in different ratios and quantities as indicated at the same time. A positive trend was seen when the ratio of M⁵/M² was greater than 1. This result implied that the lead compounds incorporated more M⁵ than M². Possible active structures were thus limited to I³-M⁵-M⁵-M⁵-T⁴ and I³-M⁵-M⁵-M²-T⁴. At this stage, possible lead structures were already confined, but before the organic syntheses and purifications of each proposed compound was performed, HPLC analyses were carried out. HPLC traces of well A5, C5 and E4 in library 5 are shown in Fig. 6. The fractions were collected, lyophilized, redissolved in DMSO, and subjected to the HCV protease assay. The result showed that the fraction at 27.5 min was responsible for the HCV protease inhibition in all these three wells and corresponded to I³-M⁵-M⁵-M⁵-T⁴. Compound I³-M⁵-M⁵-M⁵-T⁴ was synthesized under aqueous conditions by combining 1.0 equiv I3 and 3.6 equiv M⁵ for 2 h, followed by the addition of 2.0 equiv T⁴. The product was isolated by HPLC and fully characterized. The pure material was subjected to the HCV protease assay and an IC50 of 1.0 µM was measured (Fig. 7). This confirmed its identity as the most active compound in the focused library, library 3.

### Experimental details:

### Synthesis of Libraries, Initial Studies:

### (a) Product Distribution with Various Amount of M¹

To a 5:1 *^{t}*BuOH/50 mM Tris-HCl buffer, pH 7.0 (abbreviated as buffer in the following context) (v/v) (80 µL) solution, initiator **I¹** (1.3 mg, 8.1 µmol, 1.0 equiv) and monomer **M¹** (2.5 mg, 8.1 µmol, 1.0 equiv) were added. The mixture was allowed to stir at 45 °C for 2 h. The solution was cooled to RT and terminator **T¹** (3.3 mg, 16 µmol, 2.0 equiv) in 16 µL 5:1 *^{t}*BuOH/buffer was added and the mixture was heated at 45 °C for 2 h. Two other experiments with monomer **M¹** (5.0 mg, 2.0 equiv and 7.5 mg, 3.0 equiv) were performed separately according to the same procedure. The reaction mixtures were analyzed by HPLC (gradient 10 to 90% CH₃CN with 0.1% TFA in 20 min). For the results see Figure 9.

### (b) Product Distribution with Different Addition Order of M¹ and M²

To a 5:1 *^{t}*BuOH/buffer (60 µL) solution, initiator **I¹** (1.0 mg, 6.1 µmol, 1.0 equiv) and monomer **M¹** (0.93 mg, 3.1 µmol, 0.50 equiv) were added. The mixture was allowed to stir at 45 °C for 2 h. The solution was cooled to RT and monomer **M²** (1.2 mg, 3.1 µmol, 0.50 equiv) was added. The mixture was allowed to stir at 45 °C for 2 h. The solution was cooled to RT and terminator **T¹** was added and the mixture was heated at 45 °C for 2 h.

The experiment with the reversed order of **M¹** and **M²** addition was performed according to the same procedure. The reaction mixtures were analyzed by HPLC (gradient 10 to 90% CH₃CN with 0.1% TFA in 20 min). For the results see Figure 2.

### General Procedures for "On-Demand" Synthesis of Libraries:

### (a) Liquid Handling

The syntheses of libraries were carried out in 96-well plates (Thermofast AB-1100). Required amounts of initiators and monomers were dissolved in in 5:1 *^{t}*BuOH/buffer solution and added to their corresponding wells by a micropipettor (Eppendorf Research). (Details are described in the synthesis of each library.)

### (b) Oligomerization and Termination Conditions

After complete addition of initiators and monomers, the 96-well plate was capped, centrifuged for 2 min at 2000 rpm, and heated in the PCR machine (equipped with a heated lid at 110 °C to prevent solvent condensation on the cap) at 45 °C for 2 h. The plate was cooled to RT and corresponding terminators from stock solutions were added to each well by a micropipettor. The plate was capped, centrifuged and heated at 45 °C for 2 h.

### (c) Dilutions

The resulting crude mixtures were serially diluted with 5:1 *^{t}*BuOH/buffer to reach the optimal concentration for biological assays. (Details are described in the synthesis of each library.)

### (d) HCV Protease FRET Assay

The HCV protease assay kits were purchased from ProteinOne and experiments were performed according to the manual provided. Without inhibitors, HCV protease cleaves the FRET substrate and results in a fluorescence signal at 530 nm (excitation wavelength at 490 nm).

With the optimal concentration, 1.0 µL diluted crude mixture from each well was transferred to an assay plate (Sigma NUNC Maxisorp). Assay solution (100 µL) was added to each well by a multichannel pipette (Eppendorf Research) and the fluorescence signals were recorded immediately. Signals were recorded every 5 min for 2 h and active inhibitors were identified by reduced fluorescence. In the control experiments, all initiators, monomers and terminators were proved inactive at 10 µM. Likewise, solvents, 5:1 *^{t}*BuOH/buffer solution and DMSO, did not interfere the results.

### Libraries

### (a) Library 1 and 2: Preliminary Library 1 and 2

For library 1 and 2, the arrangement of the initiators, monomers, and terminators is indicated in Figure 3A Figure 3B. The wells in the same row had the same assigned initiator and the wells in the same column had the same assigned terminator. In each preliminary library, a total of four types of monomers were used. The plate was divided into four sectors and each of them contained three out of four types of monomers. During the oligomerization process, each well contained one initiator (0.5 µmol, 1.0 equiv) and three assigned monomers (total 1.0 µmol, 2.0 equiv) in a total of 5 µL 5:1 *^{t}*BuOH/buffer solution. The three monomers were in a 1:1:1 ratio (i.e. 0.67 µmol of each monomer). For example, well A1 in library 1 contained initiator **I¹** (0.50 µmol, 1.0 equiv), monomer **M¹** (0.33 µmol, 0.67 equiv), monomer **M³** (0.33 µmol, 0.67 equiv) and monomer **M⁴** (0.33 µmol, 0.67 equiv). When the oligomerization process was finished, terminator **T¹** (1.0 µmol, 1.0 µL, 2.0 equiv) was added.

After the reactions were complete, each well was diluted to 35 µL with 5:1 *^{t}*BuOH/buffer and 1.0 µL of this crude solution was used for the HCV assay. The relative fluorescence units (RFU) values from assay results were normalized, with the highest value as 100, and active wells were identified with normalized RFU value < 65 (Table 1).

**Table 1. RFU raw values (above) and normalized values (below) at 90 min in (a) library 1 (b) library 2.**

| **A** | | | | | | | | | | |
|---|---|---|---|---|---|---|---|---|---|---|
| | 1 | 2 | 3 | 4 | 5 | 6 | 7 | 8 | | |
| A | 784 | 339 | 768 | 668 | 759 | 680 | 714 | 597 | | |
| B | 721 | 407 | 568 | 634 | 670 | 787 | 774 | 739 | | |
| C | 724 | 375 | 647 | 316 | 708 | 667 | 693 | 439 | | |
| D | 720 | 450 | 695 | 658 | 654 | 715 | 714 | 625 | | |
| E | 690 | 210 | 335 | 603 | 396 | 534 | 513 | 479 | | |
| F | 656 | 188 | 541 | 514 | 452 | 471 | 540 | 540 | | |
| G | 617 | 482 | 516 | 293 | 464 | 555 | 564 | 328 | | |
| H | 689 | 686 | 665 | 707 | 670 | 573 | 571 | 544 | | |

| | 1 | 2 | 3 | 4 | 5 | 6 | 7 | 8 | | |
|---|---|---|---|---|---|---|---|---|---|---|
| A | 100 | 43 | 98 | 85 | 96 | 86 | 91 | 76 | | |
| B | 92 | 52 | 72 | 81 | 85 | 100 | 98 | 94 | | |
| C | 92 | 48 | 82 | 40 | 90 | 85 | 88 | 56 | | |
| D | 91 | 57 | 88 | 84 | 83 | 91 | 91 | 79 | | |
| E | 88 | 27 | 43 | 77 | 50 | 68 | 65 | 61 | | |
| F | 83 | 24 | 69 | 65 | 57 | 60 | 69 | 69 | | |
| G | 78 | 61 | 66 | 37 | 59 | 71 | 72 | 42 | | |
| H | 88 | 87 | 84 | 90 | 85 | 73 | 73 | 69 | | |

| | 1 | 2 | 3 | 4 | 5 | 6 | 7 | 8 | 9 | 10 |
|---|---|---|---|---|---|---|---|---|---|---|
| **B** | | | | | | | | | | |
| A | 361 | 357 | 253 | 254 | 215 | 308 | 292 | 277 | 270 | 255 |
| B | 249 | 372 | 431 | 245 | 226 | 241 | 349 | 382 | 248 | 292 |
| C | 419 | 412 | 358 | 353 | 274 | 274 | 404 | 371 | 331 | 286 |
| D | 415 | 379 | 345 | 370 | 313 | 357 | 269 | 378 | 360 | 280 |
| E | 360 | 277 | 391 | 330 | 277 | 208 | 248 | 230 | 240 | 247 |
| F | 297 | 353 | 361 | 239 | 240 | 203 | 396 | 337 | 235 | 259 |
| G | 402 | 280 | 255 | 345 | 314 | 275 | 375 | 376 | 337 | 355 |
| H | 427 | 309 | 429 | 375 | 386 | 317 | 289 | 317 | 294 | 263 |

| | 1 | 2 | 3 | 4 | 5 | 6 | 7 | 8 | 9 | 10 |
|---|---|---|---|---|---|---|---|---|---|---|
| A | 84 | 83 | 59 | 59 | 50 | 72 | 68 | 65 | 63 | 59 |
| B | 58 | 87 | 100 | 57 | 53 | 56 | 81 | 89 | 58 | 68 |
| C | 98 | 96 | 83 | 82 | 64 | 64 | 94 | 86 | 77 | 67 |
| D | 97 | 88 | 80 | 86 | 73 | 83 | 63 | 88 | 84 | 65 |
| E | 84 | 65 | 91 | 77 | 65 | 48 | 58 | 54 | 56 | 58 |
| F | 69 | 82 | 84 | 56 | 56 | 47 | 92 | 79 | 55 | 60 |
| G | 94 | 65 | 59 | 80 | 73 | 64 | 87 | 88 | 79 | 83 |
| H | 100 | 72 | 100 | 87 | 90 | 74 | 67 | 74 | 69 | 61 |

### (b) Library 3: Focused Library

In library 3, the arrangement of the initiators, monomers, and terminators is indicated in Figure 4. In sector 1, the wells in the same row had the same assigned initiator, the wells in the same column had the same assigned terminator and every well contained all three monomers.

During the oligomerization process, each well in sector 1 contained one initiator (0.5 µmol, 1.0 equiv) and all three monomers (total 1.0 µmol, 2.0 equiv) in a total of 5.0 µL 5:1 *^{t}*BuOH/buffer solution. The three monomers were in a 1:1:1 ratio (i.e. 0.67 µmol of each monomer). For example, well A1 contained initiator **I¹** (0.50 µmol, 1.0 equiv), monomer **M²** (0.33 µmol, 0.67 equiv), monomer **M⁴** (0.33 µmol, 0.67 equiv), and monomer **M⁵** (0.33 µmol, 0.67 equiv). When the oligomerization process was finished, terminator **T²** (1.0 µmol, 1.0 µL, 2.0 equiv) was added. After the reactions were complete, each well was diluted to 35 µL with 5:1 *^{t}*BuOH/buffer. Sector 1 was serially diluted with 5:1 *^{t}*BuOH/buffer solution to give sector 2, 3, and 4 and 1.0 µL of each well was used for the HCV assay. Based on the assay results (Table 2), well C2 was selected.

**Table 2. RFU raw values at 90 min in library 3. (Active wells are shaded.)**

| | 1 | 2 | 3 | 4 | 5 | 6 | 7 | 8 | 9 | 10 |
|---|---|---|---|---|---|---|---|---|---|---|
| A | 492 | 486 | 532 | 455 | 489 | 536 | 503 | 510 | 522 | 501 |
| B | 448 | 404 | 407 | 453 | 373 | 429 | 416 | 435 | 460 | 425 |
| C | 334 | 231 | 367 | 368 | 239 | 462 | 293 | 445 | 309 | 372 |
| D | 575 | 504 | 509 | 510 | 508 | 545 | 507 | 474 | 516 | 530 |
| E | 487 | 441 | 484 | 427 | 506 | 528 | 431 | 451 | 433 | 466 |
| F | 450 | 417 | 449 | 434 | 431 | 426 | 352 | 457 | 426 | 420 |
| G | 417 | 401 | 433 | 398 | 392 | 477 | 436 | 446 | 377 | 463 |
| H | 451 | 389 | 452 | 393 | 470 | 475 | 462 | 472 | 452 | 313 |

In library 4, the arrangement of the initiators, monomers, and terminators is indicated in Figure 5A. During the oligomerization process, each well contained initiator **I³** (0.5 µmol, 1.0 equiv) and monomers (as indicated) in a total of 5.0 µL 5:1 *^{t}*BuOH/buffer solution. For example, well A1 contained initiator **I³** (0.50 µmol, 1.0 equiv), and monomer **M²** (0.5 µmol, 1.0 equiv). Well C3 contained initiator **I³** (0.50 µmol, 1.0 equiv), monomer **M⁵** (0.50 µmol, 1.0 equiv) and monomer **M⁴** (0.50 µmol, 1.0 equiv).

For wells where two monomers were added in sequence (well B3, B4, and C1), the first monomer (0.50 µmol, 1.0 equiv) was allowed to react with the initiator (0.5 µmol, 1.0 equiv) in a total 5.0 µL 5:1 *^{t}*BuOH/buffer solution for 2 h. The second monomer (0.50 µmol, 1.0 equiv) in 2.0 µL 5:1 *^{t}*BuOH/buffer solution was added to the solution and the reaction mixture was allowed to react for another 2 h. For example, well C1 contained initiator **I³** (0.50 µmol, 1.0 equiv) and monomer **M²** (0.50 µmol, 1.0 equiv) in a total 5.0 µL 5:1 *^{t}*BuOH/buffer solution. After 2 h reaction time, monomer **M⁵** (0.50 µmol, 1.0 equiv) in 2.0 µL 5:1 *^{t}*BuOH/buffer solution was added.

When the oligomerization process was finished, terminator **T⁴** (1.0 µmol, 1.0 µL, 2.0 equiv) was added to each well. After the reactions were complete, each well was diluted to 50 µL with 5:1 *^{t}*BuOH/buffer (first dilution). From this diluted solution, 10 µL were taken and further diluted with 5:1 *^{t}*BuOH/buffer to 100 µL (second dilution). One microliter of the second dilution solution was used for the HCV assay. Based on the assay results (Table 3), wells B2 and C4, with the lowest RFU, were considered active.

**Table 3. RFU raw values at 90 min in in library 4. (The control well with I³ (1 equiv) and T⁴ (2 equiv) without monomers under the same reaction condition had RFU 448.) (Selected wells are shaded)**

| | 1 | 2 | 3 | 4 |
|---|---|---|---|---|
| A | 429 | 386 | 389 | 302 |
| B | 330 | 225 | 272 | 316 |
| C | 374 | 354 | 414 | 239 |

In library 5, the arrangement of the initiators, monomers, and terminators is indicated in Figure 5B. Row A was designed for deconvolution of B2 in library 4, row B to E for C4 in library 4.

During the oligomerization process, each well contained initiator **I³** (0.5 µmol, 1.0 equiv) and monomers (as indicated) in a total of 5.0 µL 5:1 *^{t}*BuOH/buffer solution. For example, well A4 contained initiator **I³** (0.50 µmol, 1.0 equiv) and monomer **M⁵** (2.0 µmol, 4.0 equiv) in a total 5.0 µL 5:1 *^{t}*BuOH/buffer solution. Well C2 contained initiator **I³** (0.50 µmol, 1.0 equiv), monomer **M²** (1.0 µmol, 2.0 equiv) and monomer **M⁵** (0.50 µmol, 1.0 equiv) in a total 5.0 µL 5:1 *^{t}*BuOH/buffer solution.

When the oligomerization process was finished, terminator **T⁴** (1.0 µmol, 1.0 µL, 2.0 equiv.) was added to each well. After the reactions were complete, each well was diluted to 50 µL with 5:1 *^{t}*BuOH/buffer (first dilution). From this diluted solution, 10 µL were taken and further diluted with 5:1 *^{t}*BuOH/buffer to 100 µL (second dilution). One microliter of the second dilution solution was used for the HCV assay. Active wells were identified with normalized RFU value < 65. In the library of deconvolution of well C4 in library 4 (rows B-E), highlighted wells were with normalized RFU ≤ 40 (Table 4).

**Table 4. RFU raw values (above) and normalized values (below) at 90 min in library 5. (The selected wells are shaded in the table of RFU raw values.)**

| | 1 | 2 | 3 | 4 | 5 |
|---|---|---|---|---|---|
| A | 621 | 242 | 243 | 236 | 234 |
| | | | | | |
| B | 389 | 474 | 429 | 227 | 240 |
| C | 262 | 325 | 465 | 247 | 207 |
| D | 429 | 357 | 439 | 226 | 221 |
| E | 446 | 371 | 387 | 205 | 246 |

| | 1 | 2 | 3 | 4 | 5 |
|---|---|---|---|---|---|
| A | 100 | 39 | 39 | 38 | 38 |
| B | 63 | 76 | 69 | 37 | 39 |
| C | 42 | 52 | 75 | 40 | 33 |
| D | 69 | 57 | 71 | 36 | 36 |
| E | 72 | 60 | 62 | 33 | 40 |

### (e) HPLC Separations: Deconvolution of Well A5, C5, and E4 in Library 5

From the selected well A5, C5 and E4, in library 5, 10 µL first dilution solution was taken and subjected to analytical HPLC separation (gradient of 10 to 90% CH₃CN with 0.1% TFA, 30 min). Major fractions were collected and the solvent was removed by lyophilization. Each fraction was redissolved in 50 µL DMSO. One microliter was used for the HCV protease assay.

In the case of A5, four fractions (1, 2, 3, 4) were collected and only fraction 3 (retention time = 27.5 min) showed inhibition of HCV protease (Figure 6, top).

In the case of C5, five fractions (1, 2, 3, 4, 5) were collected and only fraction 5 (retention time = 27.5 min) showed inhibition of HCV protease (Figure 6, middle).

In the case of E4, five fractions (1, 2, 3, 4, 5) were collected and only fraction 4 (retention time = 27.5 min) showed inhibition of HCV protease (Figure 6, bottom).

### Lead Inhibitor Synthesis, Isolation, and Characterizations:

To a 5:1 *^{t}*BuOH/buffer solution (0.30 mL, 0.1 M) of *α*-ketoglutaric acid **I³** (4.4 mg, 0.030 mmol, 1.0 equiv), monomer **M⁵** (40 mg, 0.12 mmol, 3.6 equiv) was added. The mixture was allowed to stir at 45 °C for 2 h. The solution was cooled to RT and terminator **T⁴** (24 mg, 0.060 mmol, 2.0 equiv) was added and the mixture was allowed to react at 45 °C for another 2 h. The crude reaction mixture was purified by preparative HPLC (gradient of 55 to 75% CH₃CN with 0.1% TFA, 30 min) at 28 min and the collected product fraction was lyophilized to give as a white solid (6.6 mg, 0.0071 mmol, 24 %). [α]_{D}²⁵ (c = 0.055, HFIP) = - 5.2; mp > 200 °C; ¹H NMR (600 MHz, *d*₆-DMSO) δ 12.04 (br s, 1H), 9.07 (t, *J* = 6.4 Hz, 1H), 7.74 (d*, J* = 8.1 Hz, 1H), 7.53 (d, *J* = 9.1 Hz, 1H), 7.48 (d, *J* = 8.7 Hz, 2H), 7.35 (d, *J* = 8.5 Hz, 2H), 7.30 (d, *J* = 8.5 Hz, 2H), 4.29 (d, *J* = 6.4 Hz, 2H), 4.14-4.07 (m, 1H), 4.03-3.95 (m, 3H), 3.00 (dd, *J* = 5.8, 16.7 Hz, 1H), 2.84 (dd, *J* = 7.5, 16.7 Hz, 1H), 2.45-2.00 (m, 12H), 1.73-1.60 (m, 10H), 1.60-1.47 (m, 7H), 1.38 (s, 9H), 1.36-1.27 (m, 3H), 1.16-1.00 (m, 9H), 0.97-0.86 (m, 6H); ¹³C NMR (150 MHz, *d*₆-DMSO) δ 196.4, 173.9, 171.9, 170.2, 169.8, 169.5, 169.5, 160.9, 137.7, 131.4, 129.2, 128.2, 79.5, 50.3, 50.2, 50.0, 44.2, 42.2, 41.4, 40.9, 40.8, 40.5, 38.6, 38.4, 38.2, 31.3, 30.2, 29.5, 29.4, 29.2, 27.7, 27.1, 27.1, 27.1, 27.0, 26.0, 26.0, 25.9, 25.8, 25.7; IR (thin film) v 3303, 2925, 2852, 1644, 1539 cm⁻¹; HRMS (ESI) calcd for C₄₉H₇₅ClN₅O₁₀[M + H]⁺ 928.5197, found, 928.5204.

IC₅₀: Ten different concentrations (10, 5.0, 2.5, 1.0, 0.75, 0.50, 0.30, 0.25, 0.080, 0.050 µM in DMSO) of **I³-M⁵-M⁵-M⁵-T⁴** were measured with HCV protease assay. Fluorescence signals were recorded at 90 min. The experiment was repeated in triplicate. IC₅₀ was calculated via nonlinear regression using the software package GraphPad Prism 5.

### General Methods:

Chemicals were purchased from Acros, Sigma-Aldrich, or ABCR and used without further purification. Thin layer chromatography (TLC) was performed on glass backed plates precoated with silica gel (Merck, Silica Gel 60 F254) and were visualized by fluorescence quenching under UV light or by staining with ceric sulfate or potassium permanganate. Flash column chromatography was performed on Silicycle Silica Flash F60 (230-400 Mesh) using a forced flow of air at 0.5-1.0 bar. NMR spectra were measured on VARIAN Mercury 300 MHz, 75 MHz, Bruker Avance 400 MHz, 100 MHz or Bruker AV-II 600 MHz, 150 MHz with a cryoprobe. Chemical shifts are expressed in parts per million (ppm) and are referenced to CDCl₃ 7.26 ppm, 77.0 ppm; CD₃OD 3.31 ppm, 49.0 ppm; *d*₆-DMSO 2.50 ppm, 39.5 ppm. Coupling constants are reported as Hertz (Hz). Splitting patterns are indicated as follows: br, broad; s, singlet; d, doublet; t, triplet; dd, doublet of doublet; dt, doublet of triplet; m, multiplet. Infrared (IR) spectra were recorded on a JASCO FT/IR-4100 spectrophotometer and are reported as wavenumber (cm⁻¹). Optical rotations were measured in a Jasco P-2000 polarimeter with a 100 mm path length cell operating at the sodium D line (589 nm) and reported as [α]_{D}²⁵ (concentration g/100 mL, solvent), T = temperature (°C). High-resolution mass spectra and MS/MS spectra were measured on a Bruker Daltonics maXis ESI-QTOF by the mass spectrometry service of the Laboratorium für Organische Chemie at the ETH Zürich. Melting points were measured on an Electrothermal Mel-Temp melting point apparatus using open glass capillaries and are uncorrected. HPLC (high performance liquid chromatography) was performed on JASCO analytical and preparative instruments. Columns used for the analytical and preparative HPLC were Shiseido CAPCELL PAK C18 UG120 (4.6 mm I.D. × 250 mm) and Shiseido Capcell Pak C18 MG II (10 mm I.D. × 250 mm) column with flow rates 1.0 mL/min and 10 mL/min respectively. The mobile phase were MQ-H₂O with 0.1% TFA (eluent A) and HPLC grade CH₃CN with 0.1% TFA (eluent B). Signals were monitored at 220, 254 and 301 nm. Library synthesis was performed on Techne PCR machine. The fluorescence was recorded on Molecular Devices and Thermo plate readers.

### Synthesis of Monomers and Terminators

### General Procedures (A) Synthesis of Monomers. (B) Synthesis of Terminators:

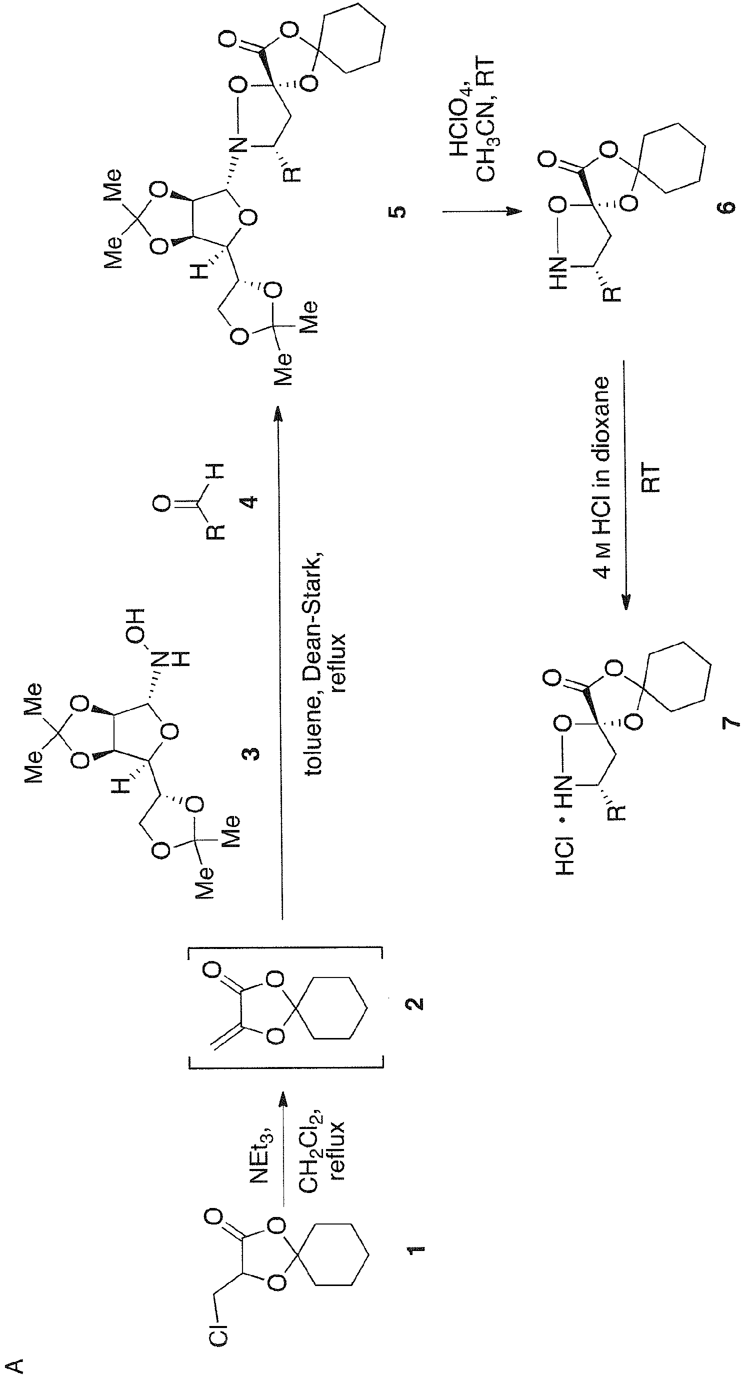

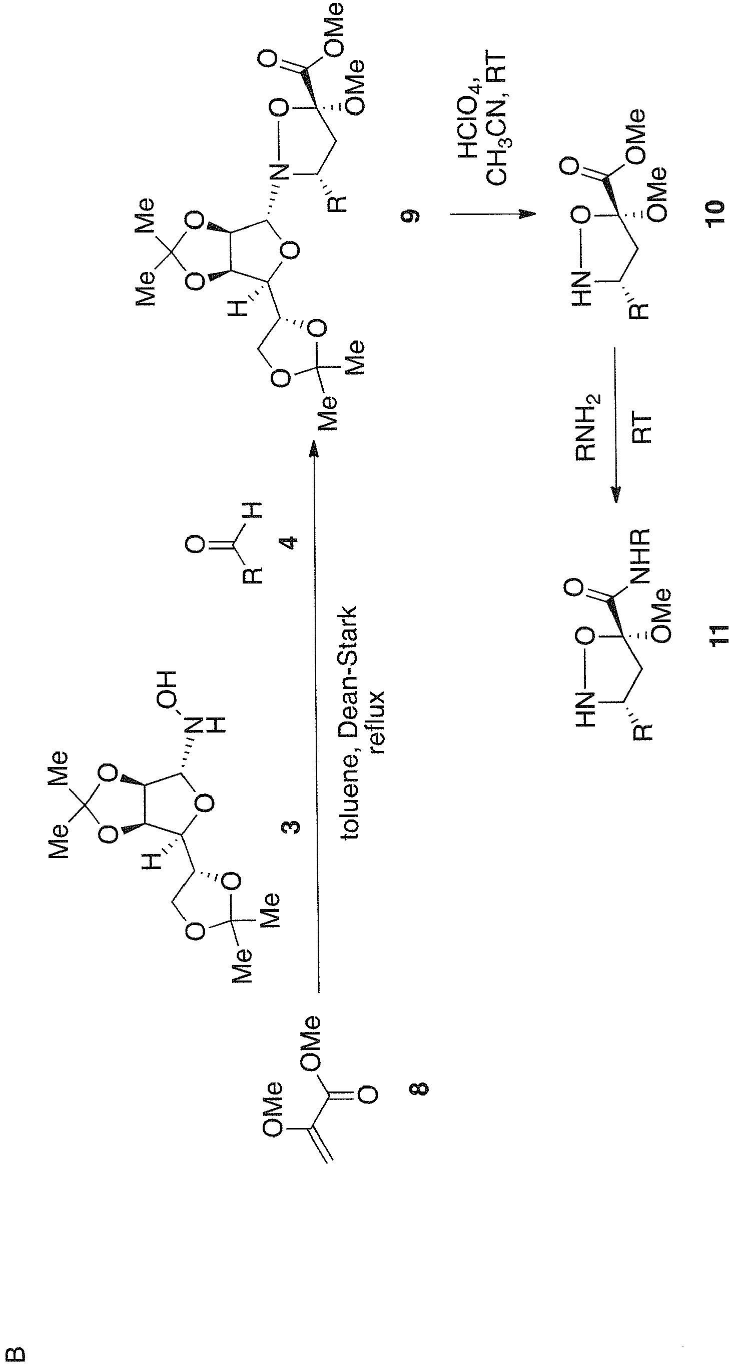

### (a) Preparation of 3-Methylene-1,4-dioxaspiro[4.5]decan-2-one (2)

To a stirred solution of 5-chloromethyl-2,2-pentamethylene-1,3-dioxolan-4-one (**1**) (1.0 equiv) in CHCl₃ (0.50 M), NEt₃ (2.0 equiv) was added and the solution heated to reflux for 18 h. The solution was cooled to RT and CHCl₃ was removed under reduced pressure to provide acrylate (**2**), which was used without further purification.

### (b) Preparation of Methyl 2-Methoxyacrylate (8)

Methyl 2-methoxy acrylate (**8**) was prepared according to the literature procedure.

### (c) Cycloaddition

A toluene solution (0.20-0.50 M) of 2,3:5,6-*O*-diisopropylidene-D-gulose oxime (**3**)¹ (1.0 equiv), aldehyde **4** (1.0 equiv) and acrylate **2** or **8** (1.0-2.0 equiv) was heated with a Dean-Stark apparatus fitted with a reflux condenser for 24 h. The solution was cooled to RT and toluene removed under reduced pressure. The crude cycloadduct D-gulose-isoxazolidine **5** or **9** was purified by flash chromatography and recrystallization.

### (d) Cleavage of Chiral Auxiliary

To a solution of cycloadduct **5** or **9** (1.0 equiv) in CH₃CN (0.10 M), HClO₄ (70% w/w, 3.0 equiv) was added and the mixture was allowed to stir at RT for 5 h. The reaction mixture was neutralized with saturated NaHCO₃ and extracted with EtOAc (3×). The combined organic layers were washed with brine (2×), dried over Na₂SO₄, and filtered. The solvent was removed under reduced pressure, and the crude reaction mixture was purified by flash chromatography to afford unprotected isoxazolidine **6** or **10.**

### (e) Preparation of Monomer HCl Salts

Unprotected isoxazolidine **6** (1.0 equiv) was dissolved in Et₂O (0.1 M) and 4 M HCl in dioxane (1.1 equiv) was added. The solution was allowed to stir at RT for 15 min and a white precipitate formed. The precipitate was collected by filtration and dried under vacuum to provide the desired isoxazolidine hydrochloride salt 7 as a white solid.

### (f) Preparation of Terminators

The corresponding amine (5.0-10 equiv) was added to isoxazolidine **10** (1.0 equiv) and the mixture was allowed to stir at RT for 12 h. The reaction mixture was diluted with EtOAc and washed with saturated NaHCO₃ (2×). The combined organic layers was washed with brine (2×), dried over Na₂SO₄, filtered and concentrated under reduced pressure to give the product. If necessary, further purification was performed by flash chromatography.

### Experimental Procedures and Characterization Data for the Synthesis of Monomers:

### D-Gulose-β³h-(thiophen 2-yl)-isoxazolidine (12):

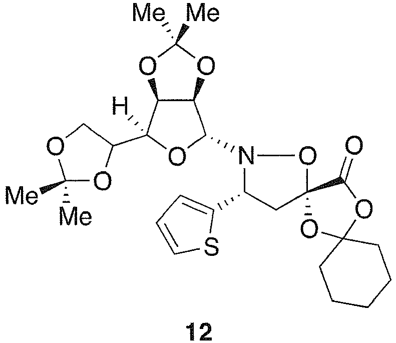

Acrylate **2** was prepared according to General Procedure (a) from 5-chloromethyl-2,2-pentamethylene-1,3-dioxolan-4-one (**1**) (0.15 g, 0.73 mmol, 1.0 equiv) and NEt₃ (0.19 mL, 1.5 mmol, 2.1 equiv) in CHCl₃ (1.5 mL). The cycloaddition was performed according to General Procedure (c) from the crude acrylate **2,** D-gulose oxime **3** (0.20 g, 0.73 mmol, 1.0 equiv) and thiophene 2-carboxaldehyde (68 µL, 0.73 mmol, 1.0 equiv) in toluene (1.5 mL). The cycloadduct was purified by flash chromatography (9:1 hexanes/EtOAc) and recrystallized from hexanes/EtOAc to afford the product as a white solid (0.19 g, 0.35 mmol, 48%). [α]_{D}²⁵ (c = 0.3, CH₂Cl₂) = + 15.0; mp = 162-164 °C; ¹H NMR (400 MHz, CDCl₃) δ 7.24 (dd, *J* = 1.2, 5.1 Hz, 1H), 7.16-7.04 (m, 1H), 6.95 (dd, *J* = 3.5, 5.1 Hz, 1H), 5.13 (dd, *J* = 3.6, 8.1 Hz, 1H), 4.93 (d*, J* = 6.0 Hz, 1H), 4.84 (s, 1H), 4.67 (d, *J* = 4.0, 6.0 Hz, 1H), 4.35 (dt, *J* = 6.8, 8.5 Hz, 1H), 4.18 (dd, *J* = 6.8, 8.5 Hz, 1H), 4.07 (dd, *J* = 4.0, 8.5 Hz, 1H), 3.68 (dd, *J* = 6.8, 8.5 Hz, 1H), 3.19 (dd, *J* = 8.1, 14.0 Hz, 1H), 2.71 (dd, *J* = 3.6, 14.0 Hz, 1H), 1.91-1.58 (m, 8H), 1.50-1.42 (m, 5H), 1.40 (s, 3H), 1.37 (s, 3H), 1.29 (s, 3H); ¹³C NMR (100 MHz, CDCl₃) δ 168.9, 142.9, 126.8, 125.5, 125.3, 112.9, 112.1, 109.8, 105.5, 97.1, 84.6, 83.7, 80.3, 75.6, 66.0, 60.2, 43.1, 37.4, 36.3, 26.7, 26.1, 25.3, 24.8, 24.3, 22.9, 22.8; IR (thin film) v 2984, 2938, 1866, 1801, 1372, 1209, 1090 cm⁻¹; HRMS (ESI) calcd for C₂₆H₃₆NO₉S [M + H]⁺ 538.2105, found, 538.2098.

### β³h-(Thiophen 2-yl)-isoxazolidine hydrochloride (M⁸):

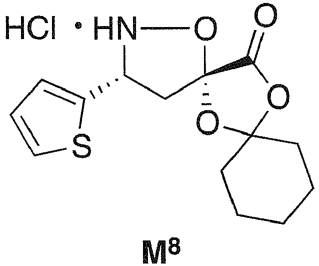

Auxiliary Cleavage was performed according to General Procedure (d) from D-gulose-*β*³h-(thiophen-2-yl)-isoxazolidine (**12**) (0.40 g, 0.74 mmol, 1.0 equiv) and HClO₄ (0.19 mL, 2.2 mmol, 3.0 equiv) in CH₃CN (7.4 mL). The crude reaction mixture was purified by flash chromatography (3:1 hexanes/EtOAc) to afford the unprotected *β*³h-(thiophen-2-yl)-isoxazolidine as a colorless liquid (0.21g, 0.71 mmol, 96%). According to General Procedure (e), the unprotected isoxazolidine was redissolved in Et₂O (7.0 mL) and treated with 4 M HCl in dioxane (0.20 mL, 0.81 mmol, 1.1 equiv) to give hydrochloride salt **M⁸** as a white solid (0.20 g, 0.60 mmol, 85 %). [α]_{D}²⁵ (c = 0.3, CH₂Cl₂) = + 30.0; mp = 115-116 °C; ¹H NMR (300 MHz, CD₃OD) δ 7.55 (dd, *J* = 1.2, 5.1 Hz, 1H), 7.43-7.27 (m, 1H), 7.11 (d, *J* = 3.6, 5.1 Hz, 1H), 5.40-5.09 (m, 1H), 3.46-3.24 (m, 1H), 2.90 (dd, *J* = 8.2, 14.2 Hz, 1H), 2.0-1.45 (m, 10H); ¹³C NMR (100 MHz, CD₃OD) δ 168.1, 136.4, 129.3, 128.5, 128.4, 113.9, 108.4, 60.9, 44.5, 38.2, 36.9, 25.2, 24.0, 24.0; IR (thin film) v 3208, 2940, 2864, 1801, 1449, 1373, 1269, 1177, 931, 703 cm⁻¹; HRMS (ESI) calcd for C₁₄H₁₈NO₄S [M + H]⁺ 296.0951, found, 296.0945.

### D-Gulose-β³h-(2-fluorophenyl)-isoxazolidine (13):

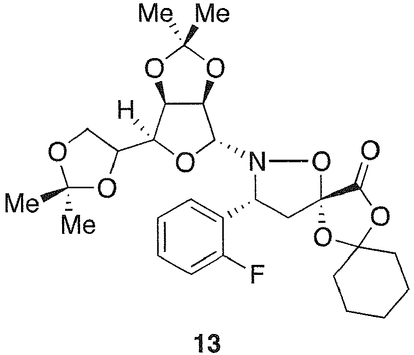

Acrylate **2** was prepared according to General Procedure (a) from 5-chloromethyl-2,2-pentamethylene-1,3-dioxolan-4-one (**1**) (1.5 g, 7.4 mmol, 1.0 equiv) and NEt₃ (2.0 mL, 15 mmol, 2.0 equiv) in CHCl₃ (15 mL). The cycloaddition was performed according to General Procedure (c) from the crude acrylate **2,** D-gulose oxime **3** (2.0 g, 7.4 mmol, 1.0 equiv) and 2-fluorobenzylaldehyde (0.78 mL, 7.4 mmol, 1.0 equiv) in toluene (15 7.9mL). The cycloadduct was purified by flash chromatography (5:1 hexanes/EtOAc) and recrystallized from hexanes to afford the product as a white solid (2.2 g, 3.9 mmol, 53%). [α]_{D}²⁵ (c = 0.5, CH₃OH) = + 24.1; mp = 105-106 °C; ¹H NMR (400 MHz, CDCl₃) δ 7.67-7.62 (m, 1H), 7.35-7.20 (m, 1H), 7.15-7.09 (m, 1H), 7.06-6.99 (m, 1H), 5.16 (dd, *J* = 8.2, 3.6 Hz, 1H), 4.97 (d, *J* = 6.0 Hz, 1H), 4.85 (s, 1H), 4.67 (dd, *J* = 6.0, 4.0 Hz, 1H), 4.32 (dt, *J* = 6.6, 8.5 Hz, 1H), 4.14 (dd, *J* = 6.6, 8.5 Hz, 1H), 3.94 (dd, *J* = 4.0, 8.5 Hz, 1H), 3.64 (dd, *J* = 6.6, 8.5 Hz, 1H), 3.22 (dd, *J* = 8.2, 13.9 Hz, 1H), 2.55 (dd, *J* = 3.6, 13.9 Hz, 1H), 1.92-1.80 (m, 2H), 1.80-1.54 (m, 6H), 1.50-1.37 (m, 5H), 1.33 (s, 3H), 1.30 (s, 3H), 1.29 (s, 3H); ¹³C NMR (100 MHz, CDCl₃) δ 168.9, 160.3 (d, *J* = 247 Hz), 129.1 (d, *J* = 8.2 Hz), 128.6 (d, *J* = 3.6 Hz), 126.7 (d, *J* = 12.7 Hz), 124.0 (d, *J* = 3.6 Hz), 115.2 (d, *J* = 21.4 Hz), 112.9, 111.9, 109.7, 105.2, 97.1, 84.4, 83.7, 80.3, 75.6, 66.0, 57.8, 42.7, 37.4, 36.2, 26.6, 26.1, 25.3, 24.9, 24.2, 22.9, 22.8; IR (thin film) v 2986, 2866, 1802, 1490, 1454, 1156, 1036, 849 cm⁻¹; HRMS (ESI) calcd for C₂₈H₃₇FNO₉ [M + H]⁺ 550.2447, found, 550.2441.

### β³h-(2-Fluorophenyl)-isoxazolidine hydrochloride (M⁶):

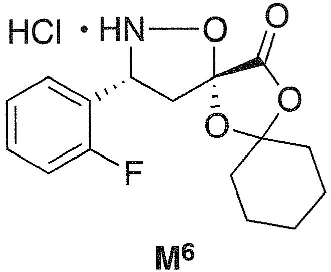

Auxiliary Cleavage was performed according to General Procedure (d) from D-gulose-*β*³h-(2-fluorophenyl)-isoxazolidine (**13**) (1.0 g, 1.8 mmol, 1.0 equiv) and HClO₄ (0.47 mL, 5.5 mmol, 3.1 equiv) in CH₃CN (18 mL). The crude reaction mixture was purified by flash chromatography (5:1 hexanes/EtOAc) to afford the unprotected *β*³h-(2-fluorophenyl)-isoxazolidine as a colorless liquid (0.51 g, 1.7 mmol, 94%). According to General Procedure (e), the unprotected isoxazolidine was redissolved in Et₂O (17 mL) and treated with 4 M HCl in dioxane (0.48 mL, 1.9 mmol, 1.1 equiv) to give hydrochloride salt **M⁶** as a white solid (0.40 g, 1.2 mmol, 71%). [α]_{D}²⁵ (c = 0.5, CH₃OH) = + 15.2; mp = 137-138 °C; ¹H NMR (400 MHz, CD₃OD) δ 7.70-7.63 (m, 1H), 7.54-7.45 (m, 1H), 7.33-7.20 (m, 2H), 5.31 (m, 1H), 3.40-3.32 (m, 1H), 2.93 (dd, *J* = 7.3, 14.2 Hz, 1H), 1.97-1.80 (m, 4H), 1.79 (m, 4H), 1.60-1.34 (m, 2H); ¹³C NMR (100 MHz, CD₃OD) δ 167.9, 162.3 (d, *J* = 246.5 Hz), 132.7 (d, *J* = 8.5 Hz), 130.1 (d, *J* = 3.1 Hz), 126.0 (d, *J* = 3.6 Hz), 122.0 (d, *J* = 13.7 Hz), 116.8 (d, *J* = 21.6 Hz), 114.0, 108.1, 59.13 (d, *J* = 3.4 Hz), 42.6, 38.2, 36.8, 25.2, 24.0, 24.0; IR (thin film) v 3398, 2940, 2864, 1798, 1682, 1454 cm⁻¹; HRMS (ESI) calcd for C₁₆H₁₉FNO₄ [M - Cl]⁺ 308.1293, found, 308.1287.

### Experimental Procedures and Characterization Data for the Synthesis of Terminators:

### (3S,5R)-3-Isobutyl-5-methoxyisoxazolidine-5-carboxamide (T¹):

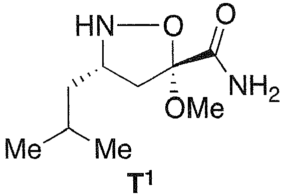

Terminator **T¹** was prepared according to General Procedure (f) from ammonium hydroxide solution (25% w/w, 0.50 mL, 3.3 mmol, 8.3 equiv) and (3*S*,5*R*)-methyl 3-isobutyl-5-methoxyisoxazolidine-5-carboxylate (86 mg, 0.40 mmol, 1.0 equiv). The product was isolated as a white solid (73 mg, 0.36 mmol, 90%). [α]_{D}²⁵ (c = 0.4, CH₃OH) = + 118.0; mp = 136-138 °C; ¹H NMR (400 MHz, CDCl₃) δ 6.68 (br d, 1H), 6.51 (br d, 1H), 5.59 (br d, 1H), 3.50-3.36 (m, 1H), 3.28 (s, 3H), 2.63 (dd, *J* = 8.2, 13.6 Hz, 1H), 1.98 (dd, *J* = 8.4, 13.6 Hz, 1H), 1.70-1.55 (m, 1H), 1.53-1.27 (m, 2H), 0.94-0.83 (m, 6H); ¹³C NMR (100 MHz, CDCl₃) δ 170.5, 109.0, 59.2, 51.6, 48.4, 40.4, 26.5, 22.6, 22.6; IR (thin film) v 3399, 3220, 3145, 2952, 2875, 1666, 1227, 1047 cm⁻¹; HRMS (ESI) calcd for C₉H₁₉N₂O₃ [M + H]⁺ 203.1390, found, 203.1381.

### (3S,5R)-N-Cyclopropyl-3-isobutyl-5-methoxyisoxazolidine-5-carboxamide (T²):

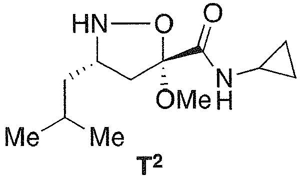

Terminator **T²** was prepared according to General Procedure (f) from cyclopropylamine (0.20 mL, 2.9 mmol, 4.8 equiv) and (3*S*,5*R*)-methyl 3-isobutyl-5-methoxyisoxazolidine-5-carboxylate (0.13 g, 0.60 mmol, 1.0 equiv). The product was isolated as a colorless liquid (92 mg, 0.38 mmol, 63%). [α]_{D}²⁵ (c = 0.8, CH₃OH) = + 67.4; ¹H NMR (400 MHz, CDCl₃) δ 6.66 (br d, 1H), 5.55 (br d, 1H), 3.47-3.30 (m, 1H), 3.24 (s, 3H), 2.84-2.72 (m, 1H), 2.58 (dd, *J* = 8.2, 13.6 Hz, 1H), 1.98 (dd*, J* = 8.3, 13.6 Hz, 1H), 1.70-1.55 (m, 1H), 1.50-1.27 (m, 2H), 0.95-0.85 (m, 6H), 0.85-0.70 (m, 2H), 0.55-0.45 (m, 2H); ¹³C NMR (100 MHz, CDCl₃) δ 168.8, 109.1, 59.3, 51.5, 48.1, 40.5, 26.5, 22.7, 22.6, 22.3, 6.6, 6.3; IR (thin film) v 3318, 2956, 1678, 1519, 1074, 1035 cm⁻¹; HRMS (ESI) calcd for C₁₂H₂₃N₂O₃ [M + H]⁺ 243.1703, found, 243.1696.

### ((3S,5R)-3-Isobutyl-5-methoxyisoxazolidin-5-yl)(morpholino)methanone (T³):

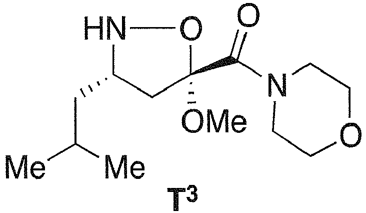

Morpholine (0.30 mL, 3.4 mmol, 6.2 equiv), 1,2,4-triazole (8.0 mg, 0.12 mmol, 0.22 equiv) and 1,8-diazabicyclo[5.4.0]undec-7-ene (16 µL, 0.11 mmol, 0.20 equiv) were added to (3*S*,5*R*)-methyl 3-isobutyl-5-methoxyisoxazolidine-5-carboxylate (0.12 g, 0.55 mmol, 1.0 equiv). The mixture was stirred at 60 °C for 12 h. The crude reaction mixture was purified by flash chromatography (1:3 hexanes/EtOAc) and the product was isolated as a clear liquid (89 mg, 0.33 mmol, 60%). [α]_{D}²⁵ (c = 0.6, CH₂Cl₂) = + 71.7; ¹H NMR (400 MHz, CDCl₃) δ 5.50 (br d, 1H), 3.93-3.42 (m, 9H), 3.28 (s, 3H), 2.88 (dd, *J* = 8.0, 12.9 Hz, 1H), 1.90 (dd, *J* = 8.0, 12.9 Hz, 1H), 1.75-1.56 (m, 1H), 1.47 (dd, *J* = 6.9, 13.8 Hz, 1H), 1.36 (dd, *J* = 7.0, 13.8 Hz, 1H), 0.96-0.58 (m, 6H); ¹³C NMR (100 MHz, CDCl₃) δ 165.3, 110.2, 67.0, 66.9, 59.3, 51.4, 46.7, 45.8, 43.1, 40.9, 26.5, 22.7, 22.7; IR (thin film) v 3203, 2956, 2867, 1651, 1434, 1253, 1116, 1068, 1029 cm⁻¹; HRMS (ESI) calcd for C₁₃H₂₄N₂NaO₄ [M + Na]⁺ 295.1628, found, 295.1613.

### tert-Butyl 3-((3S,5R)-5-((4-chlorobenzyl)carbamoyl)-5-methoxyisoxazolidin-3-yl)propanoate (T⁴):

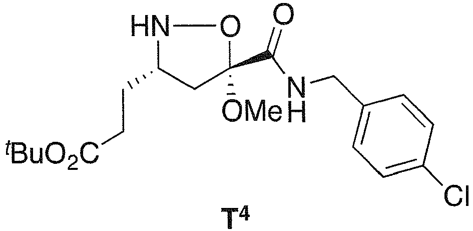

Terminator **T⁴** was prepared according to General Procedure (f) from 4-chlorobenzylamine (1.5 mL, 12 mmol, 8.6 equiv) and tert-butyl 3-((3*S*,5*R*)-5-((4-chlorobenzyl)carbamoyl)-5-methoxyisoxazolidin-3-yl)propanoate (0.40 g, 1.4 mmol, 1.0 equiv). The crude reaction mixture was purified by flash chromatography (1:1 hexanes/EtOAc) and the product was isolated as a white solid (0.48 g, 1.2 mmol, 86%). [α]_{D}²⁵ (c = 0.5, CH₂Cl₂) = + 51.0; mp = 65-67 °C; ¹H NMR (400 MHz, CDCl₃) δ 7.29 (d, *J* = 8.5 Hz, 2H), 7.18 (d, *J* = 8.5 Hz, 2H), 6.95 (br t, 1H), 5.68 (br d, 1H), 4.50-4.35 (m, 2H), 3.42-3.30 (m, 1H), 3.26 (s, 3H), 2.60 (dd, *J* = 8.2, 13.6 Hz, 1H), 2.30 (t, *J* = 7.4 Hz, 2H), 2.05 (dd, *J* = 8.1, 13.6 Hz, 1H), 1.88-1.75 (m, 2H), 1.42 (s, 9H); ¹³C NMR (100 MHz, CDCl₃) δ 171.9, 167.4, 136.1, 133.5, 129.1, 128.9, 109.1, 80.7, 60.5, 51.5, 47.6, 42.6, 32.9, 28.0, 26.4; IR (thin film) v 3353, 2978, 2936, 1726, 1681, 1523, 1154, 1089 cm⁻¹; HRMS (ESI) calcd for C₁₉H₂₇ClN₂NaO₅ [M + Na]⁺ 421.1501, found, 421.1490.

### (3S,5R)-N-Allyl-3-isobutyl-5-methoxyisoxazolidine-5-carboxamide (T⁵):

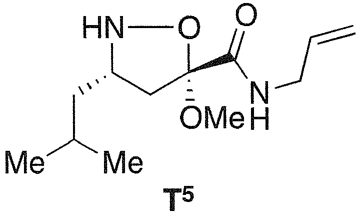

Terminator **T⁵** was prepared according to General Procedure (f) from allylamine (0.35 mL, 4.6 mmol, 5.9 equiv) and (3*S*,5*R*)-methyl 3-isobutyl-5-methoxyisoxazolidine-5-carboxylate (0.17 g, 0.78 mmol, 1.0 equiv). The product was isolated as a white solid (0.18 g, 0.74 mmol, 95%). [α]_{D}²⁵ (c = 2.0, CH₂Cl₂) = + 95.3; mp = 33-35 °C; ¹H NMR (300 MHz, CDCl₃) δ 6.75 (br t, 1H), 5.88-5.73 (m, 1H), 5.59 (br d, 1H), 5.21-5.09 (m, 2H), 4.01-3.79 (m, 2H), 3.50-3.32 (m, 1H), 3.25 (s, 3H), 2.60 (dd, *J* = 8.1, 13.6 Hz, 1H), 1.99 (dd, *J* = 8.4, 13.6 Hz, 1H), 1.72-1.53 (m, 1H), 1.54-1.27 (m, 2H), 0.93-0.85 (m, 6H); ¹³C NMR (100 MHz, CDCl₃) δ 167.4, 133.4, 116.7, 109.2, 59.3, 51.5, 48.2, 41.5, 40.5, 26.5, 22.7, 22.6; IR (thin film) v 2957, 2871, 2837, 1672, 1645, 1526, 1261, 1148, 991, 919 cm⁻¹; HRMS (ESI) calcd for C₁₂H₂₃N₂O₃ [M + H]⁺ 243.1703, found, 243.1697.

### D-Gulose-β³h-(4-methoxyphenyl)-isoxazolidine (14):

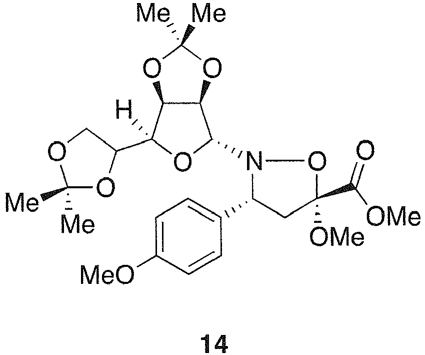

The cycloaddition was followed General Procedure (c) from methyl 2-methoxy acrylate **8** (1.7 g, 15 mmol, 2.1 equiv), D-gulose oxime **3** (2.0 g, 7.3 mmol, 1.0 equiv) and 4-methoxybenzaldehyde (1.0 g, 7.3 mmol, 1.0 equiv) in toluene (35 mL). The cycloadduct was purified by flash chromatography (3:1 hexanes/EtOAc) and recrystallized from hexanes to afford the product as a white solid (2.5 g, 4.9 mmol, 67%). [α]_{D}²⁵ (c = 0.3, CH₂Cl₂) = + 54.0; mp = 58-60 °C; ¹H NMR (300 MHz, CDCl₃) δ 7.36 (d, *J* = 8.7 Hz, 2H), 6.83 (d, *J* = 8.7 Hz, 2H), 5.08 (d, *J* = 6.0 Hz, 1H), 4.74 (s, 1H), 4.67 (dd, *J* = 4.2, 6.0 Hz, 1H), 4.32-4.18 (m, 2H), 4.18-4.06 (m, 1H), 3.92-3.81 (m, 4H), 3.77 (s, 3H), 3.60 (dd, *J* = 7.0, 8.3 Hz, 1H), 3.41 (s, 3H), 2.95 (dd, *J* = 8.4, 13.6 Hz, 1H), 2.56 (dd, *J* = 8.0, 13.6 Hz, 1H), 1.61 (s, 3H), 1.42 (s, 3H), 1.31 (s, 3H), 1.28 (s, 3H); ¹³C NMR (100 MHz, CDCl₃) δ 168.8, 159.2, 130.2, 128.8, 114.0, 112.6, 109.5, 104.4, 98.2, 84.4, 82.5, 80.7, 75.8, 66.2, 65.9, 55.2, 52.9, 51.8, 48.7, 26.6, 26.0, 25.3, 24.8; IR (thin film) v 2986, 2937, 1750, 1515, 1456, 1372, 1251, 1067 cm⁻¹; HRMS (ESI) calcd for C₂₅H₃₆NO₁₀ [M + H]⁺ 510.2334, found, 510.2332.

### β³h-(4-Methoxyphenyl)-isoxazolidine (15):

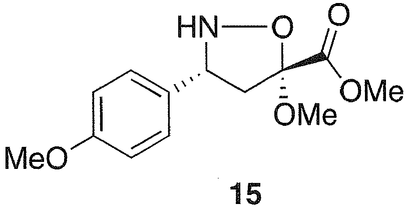

Auxiliary Cleavage was performed according to General Procedure (d) from D-gulose-*β*³h-(4-methoxyphenyl)-isoxazolidine **14** (2.6 g, 5.1 mmol, 1.0 equiv) and HClO₄ (1.3 mL, 15 mmol, 2.9 equiv) in CH₃CN (50 mL). The crude reaction mixture was purified by flash chromatography (1:1 hexanes/EtOAc) to afford the product as a white solid (0.96 g, 3.6 mmol, 71%).[α]_{D}²⁵ (c = 0.5, CH₂Cl₂) = + 42.2; mp = 92-93 °C; ¹H NMR (400 MHz, CDCl₃) δ 7.35 (d, *J* = 8.7 Hz, 2H), 6.89 (d, *J* = 8.7 Hz, 2H), 5.70 (br d, 1H), 4.48-5.57 (m, 1H), 3.87 (s, 3H), 3.80 (s, 3H), 3.42 (s, 3H), 2.91 (dd, *J* = 9.3, 13.5 Hz, 1H), 2.52 (dd, *J* = 7.4, 13.5 Hz, 1H); ¹³C NMR (100 MHz, CDCl₃) δ 168.0, 159.7, 129.0, 128.0, 114.2, 108.3, 63.7, 55.2, 52.8, 51.7, 48.4; IR (thin film) v 2951, 2838, 1749, 1516, 1437, 1059, 811 cm⁻¹; HRMS (ESI) calcd for C₁₃H₁₈NO₅ [M + H]⁺ 268.1179, found, 268.1183.

### (3R,5R)-N-(3-(Diethylamino)propyl)-5-methoxy-3-(4-methoxyphenyl)isoxazolidine-5-carboxamide (T⁶):

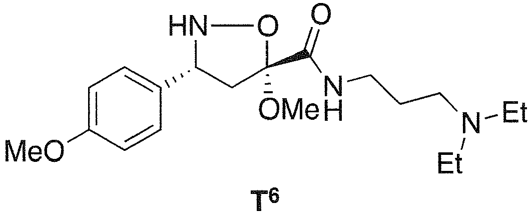

Terminator **T⁶** was prepared according to General Procedure (f) from *N,N*-diethyl-1,3-diaminopropane (0.60 mL, 3.7 mmol, 4.7 equiv) and *β*³h-(4-methoxyphenyl)-isoxazolidine **15** (0.17 g, 0.78 mmol, 1.0 equiv) in DMF (0.60 mL). The product was isolated as a colorless liquid (0.21 g, 0.58 mmol, 74%). [α]_{D}²⁵ (c = 0.5, CH₂Cl₂) = + 26.1; ¹H NMR (400 MHz, CDCl₃) δ 8.11 (br t, 1H), 7.32 (d, *J* = 8.5 Hz, 2H), 6.85 (d, *J* = 8.5 Hz, 2H), 5.71 (br d, 1H), 4.43-4.34 (m, 1H), 3.76 (s, 3H), 3.48-3.30 (m, 2H), 3.30 (s, 3H), 2.78 (dd, *J* = 8.4, 13.6 Hz, 1H), 2.60-2.43 (m, 7H), 1.70-1.62 (m, 2H), 1.03 (t, *J* = 7.1 Hz, 6H); ¹³C NMR (100 MHz, CDCl₃) δ 167.0, 159.6, 128.9, 128.0, 114.2, 109.3, 63.6, 55.2, 52.0, 51.2, 48.0, 46.7, 39.3, 25.6, 11.4; IR (thin film) v 2967, 2936, 2811, 1679, 1515, 1251, 1034, 830 cm⁻¹; HRMS (ESI) calcd for C₁₉H₃₂N₃O₄ [M + H]⁺ 366.2387, found, 366.2397.

### (3S,5R)-3-Benzyl-5-methoxy-N-(2-(pyrrolidin-1-yl)ethyl)isoxazolidine-5-carboxamide (T⁷):

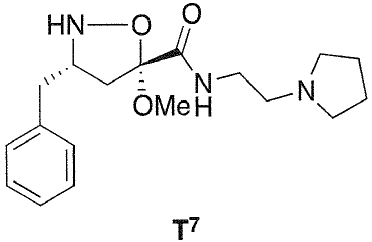

Terminator **T⁷** was prepared according to General Procedure (f) from 2-cyclopentylethanamine (0.47 mL, 3.7 mmol, 10 equiv) and (3*S*,5*R*)-methyl 3-benzyl-5-methoxyisoxazolidine-5-carboxylate (87 mg, 0.35 mmol, 1.0 equiv). The crude reaction mixture was purified by flash chromatography (9:1 CH₂Cl₂/CH₃OH) and the product was isolated as a clear liquid (87 mg, 0.26 mmol, 73%). [α]_{D}²⁵ (c = 1.0, CH₂Cl₂) = + 58.4; ¹H NMR (400 MHz, CDCl₃) δ 7.35-7.13 (m, 5H), 7.06 (br t, 1H), 5.75 (br d, 1H), 3.76-3.57 (m, 1H), 3.54-3.31 (m, 2H), 3.28 (s, 3H), 2.98 (dd, *J* = 6.0, 13.7 Hz, 1H), 2.76 (dd, *J* = 7.9, 13.6 Hz, 1H), 2.66-2.45 (m, 7H), 2.16 (dd, *J* = 7.9, 13.6 Hz, 1H), 1.87-1.61 (m, 4H); ¹³C NMR (100 MHz, CDCl₃) δ 167.5, 137.4, 128.7, 128.7, 126.7, 109.2, 61.8, 54.5, 53.9, 51.5, 47.2, 38.0, 37.4, 23.5; IR (thin film) v 3370, 2937, 2800, 1677, 1527, 1455, 1150, 1085, 701 cm⁻¹; HRMS (ESI) calcd for C₁₈H₂₈N₃O₃ [M + H]⁺ 334.2125, found, 334.2132.

### (3S,5R)-3-Isobutyl-5-methoxy-N-(pyridin-4-ylmethyl)isoxazolidine-5-carboxamide(T⁸):

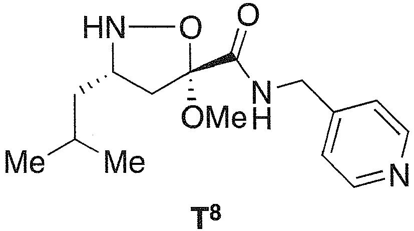

Terminator **T⁸** was prepared according to General Procedure (f) from 4-(aminomethyl)pyridine (0.40 mL, 4.0 mmol, 5.1 equiv) and (3*S*,5*R*)-methyl 3-isobutyl-5-methoxyisoxazolidine-5-carboxylate (0.17 g, 0.78 mmol, 1.0 equiv). The crude reaction mixture was purified by flash chromatography (9:1 CH₂Cl₂/CH₃OH) and the product was isolated as a white solid (0.18 g, 0.61 mmol, 78%). [α]_{D}²⁵ (c = 1.0, CH₂Cl₂) = + 68.6; mp = 82-84 °C; ¹H NMR (400 MHz, CDCl₃) δ 8.53 (d, *J* = 4.9 Hz, 2H), 7.13-7.20 (m, 3H), 5.59 (br d, 1H), 4.57-4.37 (m, 2H), 3.47-3.37 (m, 1H), 3.26 (s, 3H), 2.61 (dd, *J* = 8.2, 13.6 Hz, 1H), 2.02 (dd, *J* = 8.3, 13.6 Hz, 1H), 1.69-1.57 (m, 1H), 1.50-1.32 (m, 2H), 0.93-0.86 (m, 6H); ¹³C NMR (100 MHz, CDCl₃) δ 168.0, 150.1, 146.6, 122.1, 109.1, 59.4, 51.5, 48.0, 42.0, 40.5, 26.5, 22.6, 22.6; IR (thin film) v 3204, 2955, 1679, 1523, 1220, 1030 cm⁻¹; HRMS (ESI) calcd for C₁₅H₂₄N₃O₃ [M + H]⁺ 294.1812, found, 294.1812.

### (3S,5R)-3-Isobutyl-5-methoxy-N-(4-sulfamoylphenethyl)isoxazolidine-5-carboxamide (T⁹):

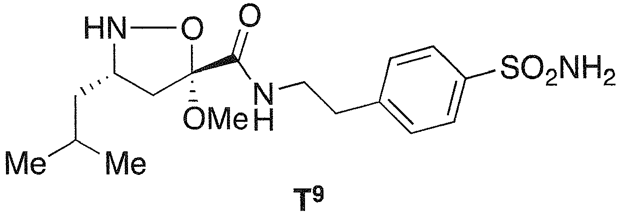

Terminator **T⁹** was prepared according to General Procedure (f) from 4-(2-aminoethyl)benzenesulfonamide (0.79 g, 4.0 mmol, 5.1 equiv) and (3*S*,5*R*)-methyl 3-isobutyl-5-methoxyisoxazolidine-5-carboxylate (0.17 g, 0.78 mmol, 1.0 equiv) and in DMF (2.0 mL). The crude reaction mixture was purified by flash chromatography (19:1 CH₂Cl₂/CH₃OH) and the product was isolated as a white solid (0.26 g, 0.68 mmol, 87%).[α]_{D}²⁵ (c = 0.4, CH₂Cl₂) = + 55.0; mp = 68-70 °C; ¹H NMR (400 MHz, CDCl₃) δ 7.85 (d, *J* = 8.4 Hz, 2H), 7.34 (d, *J* = 8.4 Hz, 2H), 6.78 (br t, 1H), 5.58 (br d, 1H), 4.98 (s, 2H), 3.76-3.61 (m, 1H), 3.61-3.50 (m, 1H), 3.41-3.28 (m, 1H), 3.17 (s, 3H), 2.93 (t, *J* = 7.0 Hz, 2H), 2.51 (dd, *J* = 8.1, 13.6 Hz, 1H), 1.97 (dd, *J* = 8.5, 13.6 Hz, 1H), 1.70-1.55 (m, 1H), 1.50-1.31 (m, 2H), 0.94-0.88 (m, 6H); ¹³C NMR (100 MHz, CDCl₃) δ 167.8, 143.8, 140.6, 129.4, 126.6, 109.1, 59.2, 51.5, 48.3, 40.4, 39.8, 35.3, 26.5, 22.7, 22.6; IR (thin film) v 2938, 2866, 1803, 1451, 1372, 1230, 1089, 849 cm⁻¹; HRMS (ESI) calcd for C₁₇H₂₇N₃NaO₅S [M + Na]⁺ 408.1564, found, 408.1550.

**LIST OF REFERENCE SIGNS**

| | | | |
|---|---|---|---|
| I | initiator | tBu | tert. Butyl |
| T | terminator | Boc | tert-Butyloxycarbonyl |
| M | monomer | Bz | benzyl |
| Me | methyl | Et | ethyl |
| Ph | phenyl | iPr | iso-propyl |

## Claims

1. Method for the generation of oligomers or a mixture of oligomers to form a chemical library by amide-forming oligomerization comprising the steps of
1) reacting a mixture of at least one initiator (I) with at least one monomer (M) to form a dimer (I-M) of the initiator (I) and the monomer (M) or
to form a pre-oligomer (I-M-M, I-M-M-M, ...) with an initiator (I) attached to a chain of more than one monomer (M), or a mixture thereof by amide-bond formation;
2) adding at least one terminator (T) for the formation of a linear oligomer (I-M-T, I-M-M-T, ...) or a mixture of linear oligomers by amide-bond formation;
or, for the formation of a cyclic oligomer or a mixture of cyclic oligomers by amide-bond formation, changing the reaction conditions relative to step 1) so as to form a linking covalent bond between the at least one initiator (I) and a monomer (M) of the dimer or pre-oligomer formed in step 1),
wherein the **initiator (I)** is selected from the group consisting of: 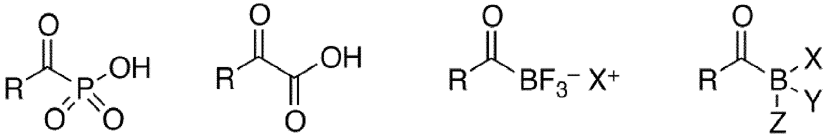 with, for the formation of a **linear** oligomer or a mixture of linear oligomers by amide-bond formation,
R being selected from the group consisting of: substituted or unsubstituted alkyl, heteroalkyl, cycloalkyl, heterocycloalkyl, aryl, heteroaryl, alkylaryl, heteroalkylaryl, alkenyl, heteroalkenyl, alkinyl, heteroalkinyl, -C(NH₂)R^{I},-C(NH₂){R^{I}-CO-C(NH₂)}ₙR^{I} with n=1, 2 and R^{I} being H or an amino-acid side chain, fluorescent dye, nucleic acid or derivative thereof, peptide nucleic acid, FLAG octapeptide (DYKDDDDK), biotin or affinity tag;
or with, for the formation of a **cyclic** oligomer or a mixture of cyclic oligomers by amide-bond formation,
R being selected from the group consisting of: -{(CH₂)}ₙR^{c}, -{(CHCH₃)}ₙR^{c},-{(CH(1,1-dimethylethyl))}ₙR^{c}, -{(CH(benzyl))}ₙR^{c}, in each case with n=1,2 and R^{c} being a linker structure allowing to form a linking covalent amide bond to the respective terminal monomer of the dimer or oligomer formed in step 1);
and in both cases with
X⁺ being a counterion, selected from the group consisting of: K⁺, Cs⁺, Li⁺, Na⁺, R₄N⁺, R₄P⁺ or R₃S⁺ with R being an organic substituent or H;
X, Y, Z, being, independently from each other, selected from the group consisting of: F, OR, N⁺R₃, N⁺R₂OR, N⁺R₂SR, and N⁺R₂NR₂, and are optionally forming a cyclic or a bicyclic structure; wherein R is an organic substituent or H;
or covalent dimers or trimers thereof with R in this case being a common linker element;
wherein the **monomer (M)** is selected from the group consisting of: 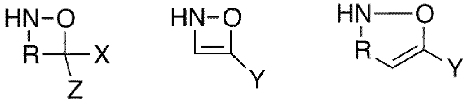 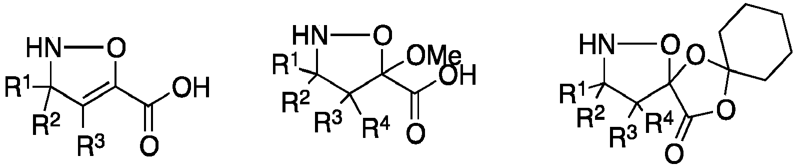 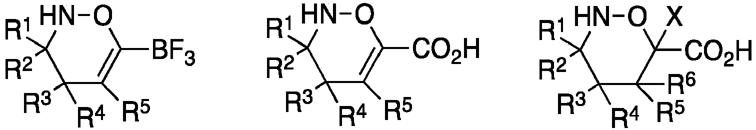 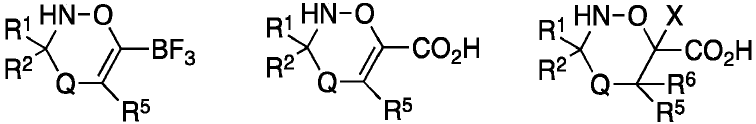 with
X being selected from the group consisting of: halogen, -OH, -COOH, -NH₂,-O-Alkyl, -O-Aryl, -O-CO-Alkyl, -O-CO-Aryl, -SH, S-Alkyl, -S-Aryl, N-Acyl, -NH-Alkyl, -NH-Aryl, -N(Alkyl)₂, -N(Aryl)₂, -N(Alkyl)(Aryl), -CO-NH-Alkyl, -CO-NH-Aryl, -CO-N(Alkyl)₂, -CO-N(Aryl)₂, -CO-N(Alkyl)(Aryl),, -CN, -NO₂, -N₃, -S(O)Aryl, -S(O)₂Aryl;
Y being selected from the group consisting of: -PO₃H, -COOH, -BF₃-X⁺,-BXYZ, wherein X⁺, X, Y, Z are defined as given above in the context of the initiator (I),
Z being selected from the group consisting of: -PO₃H, -COOH, -BF₃-X⁺,-BXYZ, wherein X⁺, X, Y, Z are defined as given above in the context of the initiator (I), as well as derivatives thereof which upon collapse of X and Z upon cleavage of the NO bond lead to Y;
R being selected from the group consisting of: O, S, NR¹, Si, CHR¹R²;
R¹-R⁶ being, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, as well as cyclic forms linking these among each other;
Q being selected from the group consisting of: O, S, Si, NR¹, where R¹ is an organic substituent or H;
and wherein the **terminator (T),** if used, is selected from the group consisting of: 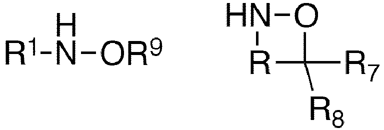 with
R being selected from the group consisting of: CH₂, (CH₂)₂, CHR^{T}, CH₂CHR^{T}, (CH₂)₃, (CH₂)₂CHR^{T}, CH₂CHR^{T}CH₂, (CH₂)₄, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
R¹ being selected from the group consisting of: substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, ester, carbamate, sulfonate, sulfinate, phosphate, silyl;
R⁷-R⁹ being, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, ester, carbamate, sulfonate, sulfinate, phosphate, silyl, as well as cyclic forms linking these among each other and carbonyl, imidate, thiomidate
or covalent dimers or trimers thereof with R¹ and/or at least one of R⁷ or R⁸ in this case being a common linker element.

2. Method according to claim 1, wherein the generation of oligomers or a mixture of oligomers is carried out using the steps of
1) reacting a mixture of at least one initiator (I) with at least one monomer (M) to form a dimer (I-M) of the initiator (I) and the monomer (M) or
to form a pre-oligomer (I-M-M, I-M-M-M, ...) with an initiator (I) attached to a chain of more than one monomer (M), or a mixture thereof by amide-bond formation;
2) adding at least one terminator (T) for the formation of a linear oligomer (I-M-T, I-M-M-T, ...) or a mixture of linear oligomers by amide-bond formation;
or, for the formation of a cyclic oligomer or a mixture of cyclic oligomers by amide-bond formation, changing the reaction conditions relative to step 1) so as to form a linking covalent bond between the at least one initiator (I) and preferably the respective terminal monomer (M) of the dimer or pre-oligomer formed in step 1).

3. Method according to claim 1 or 2, wherein one single initiator (I) is used in step 1) and, in case of the formation of a linear oligomer or a mixture of linear oligomers by amide-bond formation, one single terminator (T) is used in step 2).

4. Method according to any of the preceding claims, wherein the initiator (I) for the formation of a linear oligomer or a mixture of linear oligomers by amide-bond formation is selected from the group consisting of: 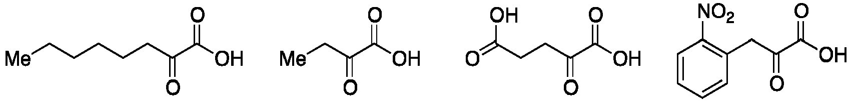 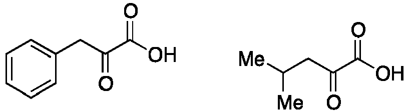 with Me= -CH₃.

5. Method according to any of the preceding claims, wherein the initiator (I) for the formation of a cyclic oligomer or a mixture of cyclic oligomers by amide-bond formation is selected in that the linker structure is selected from the group consisting of: a chain of one or two elements selected from the group of: amino acid, CO((CH₂)₂NH, and this chain terminated by a group selected from: 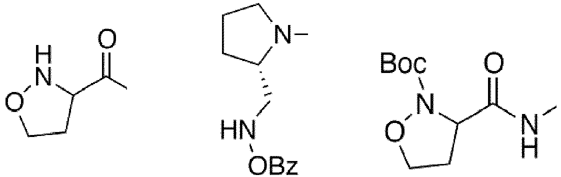 with Bz=benzyl; Boc= tert-butyloxycarbonyl.

6. Method according to any of the preceding claims, wherein the initiator (I) for the formation of a cyclic oligomer or a mixture of cyclic oligomers by amide-bond formation is given by a structure comprising at one initiator moiety selected from the group consisting of: 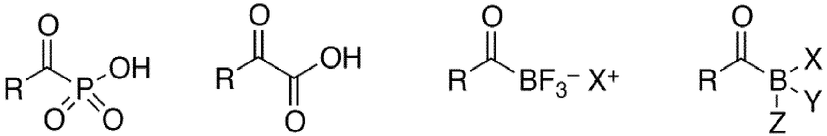 with
R being a linker element
X⁺ being a counterion, selected from the group consisting of: K⁺, Cs⁺, Li⁺, Na⁺, R₄N⁺, R₄P⁺ or R₃S⁺ with R being an organic substituent or H;
X, Y, Z, being, independently from each other, selected from the group consisting of: F, OR, N⁺R₃, N⁺R₂OR, N⁺R₂SR, and N⁺R₂NR₂, and are optionally forming a cyclic or a bicyclic structure; wherein R is an organic substituent or H;
and at least one terminator moiety selected from the group consisting of: 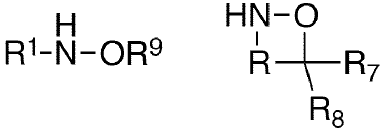 with
R being selected from the group consisting of: CH₂, (CH₂)₂, CHR^{T}, CH₂CHR^{T}, (CH₂)₃, (CH₂)₂CHR^{T}, CH₂CHR^{T}CH₂, (CH₂)₄, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
R¹ being a linker element;
R⁷-R⁹ being, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, ester, carbamate, sulfonate, sulfinate, phosphate, silyl, as well as cyclic forms linking these among each other and carbonyl, imidate, thiomidate, with the proviso that at least one of R⁷ or R⁸ is a linker element,
linked by a common linker element given by R in the initiator moiety and by R¹ or R⁷ or R⁸ in the terminator moiety;

7. Method according to claim 6, wherein the initiator (I) for the formation of a cyclic oligomer or a mixture of cyclic oligomers by amide-bond formation is selected from the group consisting of: 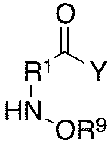 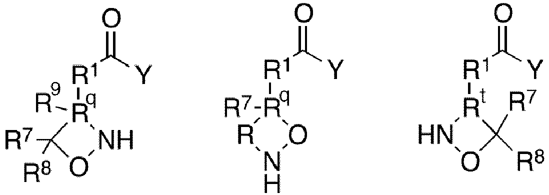 wherein
R¹ is a linker element;
R^{q} is a structural element complementing to a 4, 5, 6, or 7 membered ring, preferably selected from the group consisting of: -C-, -CH₂-C-, -CHR^{T}-C-,-(CH₂)₂-C-, --(CHR^{T})₂-C-, -(CH₂)-C-(CH₂)-, -(CHR^{T})-C-(CH₂)-, -(CHR^{T})-C-(CHR^{T})-, -(CH₂)₃-C, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
R^{t} is a structural element complementing to a 4, 5, 6, or 7 membered ring, preferably selected from the group consisting of: -CR^{U}-, -CH₂-CR^{U}-,-CHR^{T}-CR^{U}-, -(CH₂)₂-CR^{U}-, -(CHR^{T})₂-CR^{U}-, -(CH₂)-CR^{U}-(CH₂)-, -(CHR^{T})-CR^{U}-(CH₂)-, -(CHR^{T})-CR^{U}-(CHR^{T})-, -(CH₂)₃-CR^{U}, wherein R^{T} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, and wherein R^{U} is selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl;
R⁷-R⁹ being, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, as well as cyclic forms linking these among each other and carbonyl, imidate, thiomidate,
R being selected from the group consisting of: O, S, NR₁, SiR₁R₂, CR₁R₂; wherein R₁ and R₂ are, independently from each other, selected from the group consisting of: hydrogen, halogen, substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl,
Y being selected from the group consisting of: -PO₃H, -COOH, -BF₃-X⁺,-BXYZ, wherein X⁺, X, Y, Z are defined as given above in the context of the initiator (I);
wherein preferably this structure is selected from the group consisting of: 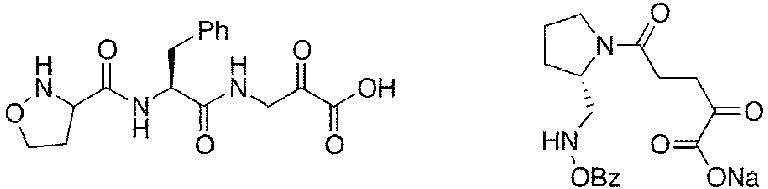 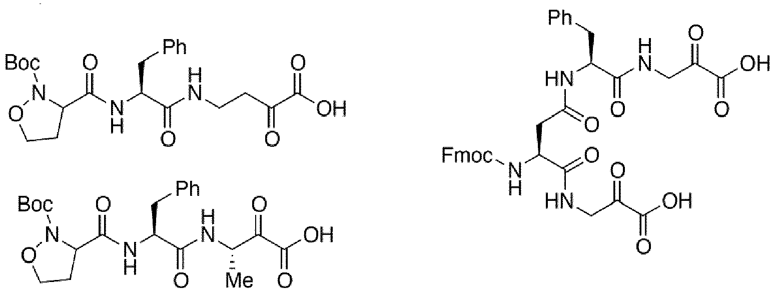 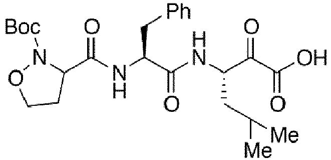 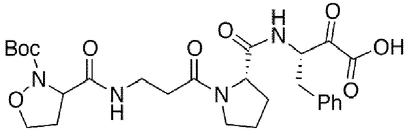 with boc=tert-butyloxycarbonyl, Ph=phenyl, Fmoc= fluorenylmethyleneoxycarbonyl, Me=-CH₃ Bz=benzyl.

8. Method according to one of the preceding claims, wherein the monomer (M) is selected from the group consisting of: 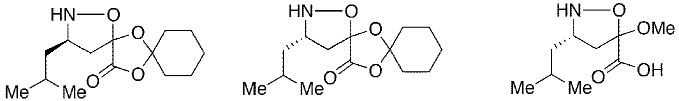 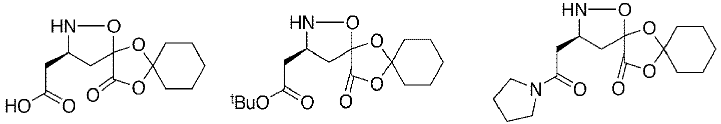 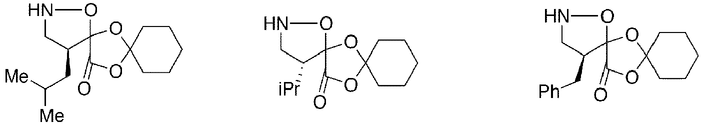 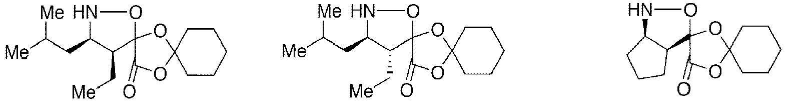 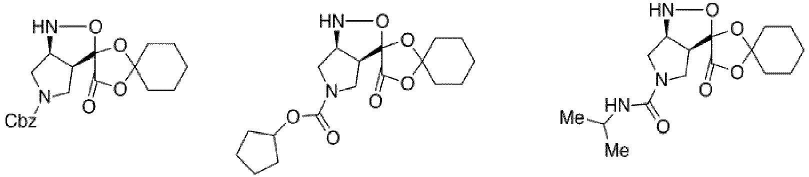 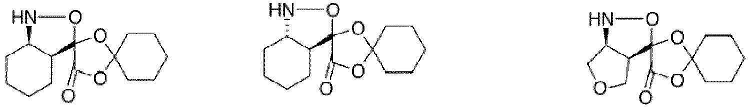 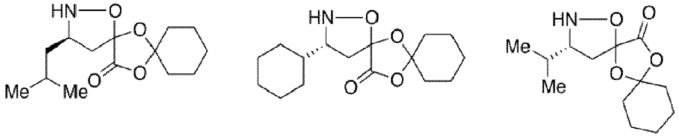 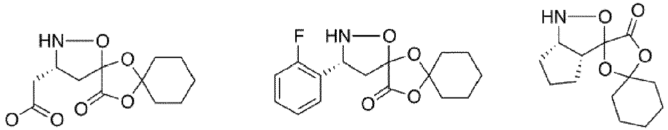 with Me== -CH₃, ^{t}Bu= 1,1-dimethylethyl, Cbz=benzyloxycarbonyl, iPr=isopropyl, Ph=phenyl.

9. Method according to any of the preceding claims, wherein the terminator (T) is selected from the group consisting of: 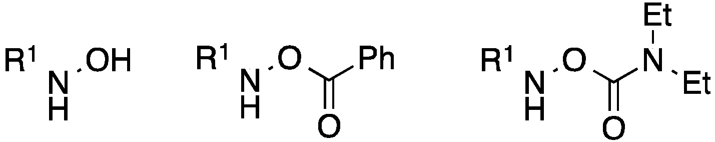 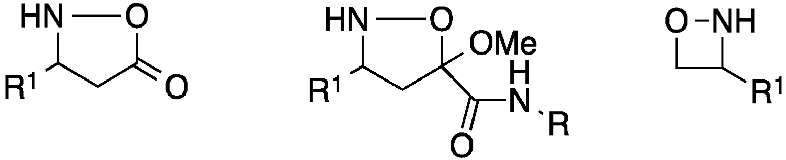 with
R¹ being selected from the group consisting of: substituted or unsubstituted alkyl, heteroalkyl, aryl, cycloalkyl, heterocycloalkyl, heteroaryl, alkylaryl, heteroalkylaryl, alkenyl, heteroalkenyl, alkinyl, heteroalkinyl, ester, carbamate, sulfonate, sulfinate, phosphate, silyl;
Me= -CH₃, Et= ethyl; Ph=phenyl.

10. Method according to any of the preceding claims, wherein step 1) and/or 2) are carried out, apart from solvent(s), without any added further chemical reagents or catalysts;

11. Method according to any of the preceding claims, wherein in step 1) and/or in step 2) organic solvents, water, aqueous buffer or combinations thereof are used.

12. Method according to any of the preceding claims, wherein in step 1) more than 1, preferably 2-6, more preferably 2-4 different monomers are used, and/or wherein in step 1) the reaction is carried over to lead to oligomers with at least 2 interlinked monomers, preferably in the range of 2-10, more preferably in the range of 2-6 interlinked monomers.

13. Method according to any of the preceding claims, wherein in step 1) the reaction conditions, preferably temperature and/or pressure, and/or reactant concentrations and/or reactant addition order and/or reactant addition time and/or reactant chirality are selected so as to lead, between different batches, to targeted different distributions of different oligomers in the mixture.

14. Method according to any of the preceding claims, wherein in step 1) one single initiator (I), one single monomer (M) and, in case of the generation of linear oligomers, in step 2) one single terminator (T) is used, and wherein the reaction conditions in step 1) and/or step 2) are adapted such as to form a specific trimer structure.

15. Method of identification of biologically and/or chemically active systems from a chemical library based on at least one mixture of oligomers made using a method according to any of the preceding claims, wherein the mixtures of oligomers are screened for activity prior to purification or separation of the compounds from the mixture.

16. Method according to claim 15, using a number of specifically differing mixtures made using a method according to claim 10 and/or 11, checking these mixtures for biological and/or chemical activity, inferring from activity patterns initiators and/or monomers and/or terminators inducing activity, preparing further mixtures using a method according to any of the preceding claims 1-12 based on the identified active initiators and/or monomers and/or terminators only, thereby successively reducing the number of possible active oligomers.

## Patentansprüche

1. Verfahren zum Erzeugen von Oligomeren oder einer Mischung von Oligomeren zwecks Bildung einer Chemikalienbibliothek durch amidbildende Oligomerisierung, umfassend die folgenden Schritte:
1) Umsetzen einer Mischung von mindestens einem Initiator (I) mit mindestens einem Monomer (M) unter Bildung eines Dimers (I-M) des Initiators (I) und des Monomers (M) oder unter Bildung eines Präoligomers (I-M-M, I-M-M-M, ...) mit einem Initiator (I), der an eine Kette von mehr als einem Monomer (M) gebunden ist, oder einer Mischung davon mittels Amidbindungsbildung;
2) Zusetzen von mindestens einem Terminator (T) für die Bildung eines linearen Oligomers (I-M-T, I-M-M-T, ...) oder einer Mischung von linearen Oligomeren mittels Amidbindungsbildung;
oder zwecks Bildung eines cyclischen Oligomers oder einer Mischung von cyclischen Oligomeren mittels Amidbindungsbildung Verändern der Reaktionsbedingungen im Vergleich zu Schritt 1) so, dass eine verknüpfende kovalente Bindung zwischen dem mindestens einen Initiator (I) und einem Monomer (M) des in Schritt 1) gebildeten Dimers oder Präoligomers gebildet wird,
wobei der **Initiator (I)** aus der Gruppe bestehend aus: 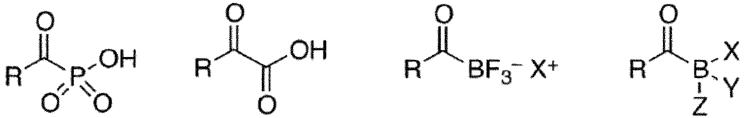 ausgewählt ist, wobei, für die Bildung eines **linearen** Oligomers oder einer Mischung von linearen Oligomeren mittels Amidbindungsbildung,
R aus der Gruppe bestehend aus den Folgenden ausgewählt ist: substituiertes oder unsubstituiertes Alkyl, Heteroalkyl, Cycloalkyl, Heterocycloalkyl, Aryl, Heteroaryl, Alkylaryl, Heteroalkylaryl, Alkenyl, Heteroalkenyl, Alkinyl, Heteroalkinyl,-C(NH₂)R^{I}, -C(NH₂){R^{I}-CO-C(NH₂)}ₙR^{I}, wobei n = 1, 2 und R¹ H oder eine Aminosäureseitenkette, ein Fluoreszenzfarbstoff, eine Nukleinsäure oder ein Derivat davon, eine Peptidnukleinsäure, ein FLAG-Octapeptid (DYKDDDDK), Biotin oder ein Affinitäts-Tag ist;
oder wobei für die Bildung eines **cyclischen** Oligomers oder einer Mischung von cyclischen Oligomeren mittels Amidbindungsbildung
R aus der Gruppe bestehend aus den Folgenden ausgewählt ist: -{(CH₂)}ₙR^{C}, -{(CHCH₃)}ₙR^{C}, -{(CH (1,1-Dimethylethyl))}ₙR^{C}, -{(CH(Benzyl))}ₙR^{C}, wobei jeweils n = 1, 2 und R^{C} eine Linkerstruktur ist, die die Bildung einer verknüpfenden kovalenten Amidbindung an das entsprechende terminale Monomer des in Schritt 1) gebildeten Dimers oder Oligomers gestattet;
und wobei in beiden Fällen
X⁺ ein Gegenion ausgewählt aus der Gruppe bestehend aus den Folgenden ist: K⁺, Cs⁺, Li⁺, Na⁺, R₄N⁺, R₄P⁺ oder R₃S⁺, wobei R ein organischer Substituent oder H ist;
X, Y, Z unabhängig voneinander aus der Gruppe bestehend aus den Folgenden ausgewählt sind: F, OR, NR₃, NR₂OR, NR₂SR und NR₂NR₂ und gegebenenfalls eine cyclische oder eine bicyclische Struktur bilden; wobei R ein organischer Substituent oder H ist;
oder kovalenten Dimeren oder Trimeren davon, wobei R in diesem Fall ein übliches Linkerelement ist;
wobei das **Monomer (M)** aus der Gruppe bestehend aus den Folgenden ausgewählt ist: 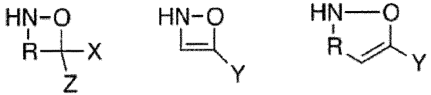 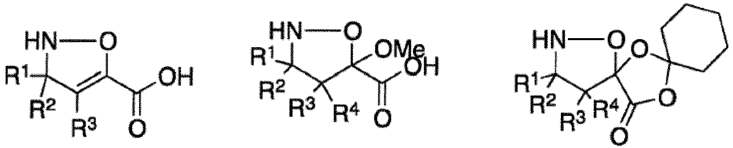 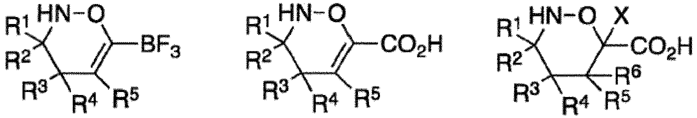 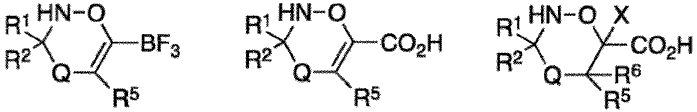 wobei
X aus der Gruppe bestehend aus den Folgenden ausgewählt ist: Halogen, -OH, -COOH, -NH₂, -O-Alkyl, -O-Aryl, -O-CO-Alkyl, -O-CO-Aryl, -SH, S-Alkyl, -S-Aryl, N-Acyl, -NH-Alkyl, -NH-Aryl, -N(Alkyl)₂, -N(Aryl)₂, -N(Alkyl) (Aryl), -C O-NH-Alkyl, -CO-NH-Aryl, -CO-N(Alkyl)₂, -CO-N(Aryl)₂, -CO-N(Alkyl)(Aryl), -CN, -NO₂, -N₃, -S (O)Aryl, -S(O)₂-Aryl;
Y aus der Gruppe bestehend aus den Folgenden ausgewählt ist: -PO₃H, -COOH, -BF₃⁻X⁺, -BXYZ, wobei X⁺, X, Y, Z wie oben im Zusammenhang mit dem Initiator (I) angeführt definiert sind,
Z aus der Gruppe bestehend aus den Folgenden ausgewählt ist: -PO₃H, -COOH, -BF₃⁻X⁺, -BXYZ, wobei X⁺, X, Y, Z wie oben in Zusammenhang mit dem Initiator (I) angeführt definiert sind, sowie Derivaten davon, die beim Zerfall von X und Z bei Spaltung der NO-Bindung zu Y führen;
R aus der Gruppe bestehend aus den Folgenden ausgewählt ist: 0, S, NR¹, Si, CHR¹R²;
R¹-R⁶ unabhängig voneinander aus der Gruppe bestehend aus den Folgenden ausgewählt sind: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl, sowie cyclischen Formen, die diese untereinander verbinden;
Q aus der Gruppe bestehend aus den Folgenden ausgewählt ist: 0, S, Si, NR¹, wobei R¹ ein organischer Substituent oder H ist;
und wobei der **Terminator (T),** falls er verwendet wird, aus der Gruppe bestehend aus den Folgenden ausgewählt ist: 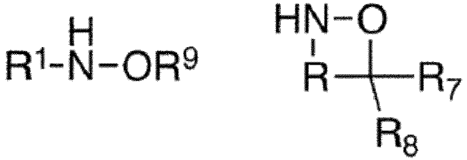 wobei
R aus der Gruppe bestehend aus den Folgenden ausgewählt ist: CH₂, (CH₂)₂, CHR^{T}, CH₂ CHR^{T}, (CH₂)₃, (CH₂)₂CHR^{T}, CH₂CHR^{T}CH₂, (CH₂)₄, wobei R^{T} aus der Gruppe bestehend aus den Folgenden ausgewählt ist: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl;
R¹ aus der Gruppe bestehend aus den Folgenden ausgewählt ist: substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl, Ester, Carbamat, Sulfonat, Sulfinat, Phosphat, Silyl;
R⁷-R⁹ unabhängig voneinander aus der Gruppe bestehend aus den Folgenden ausgewählt sind: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl, Ester, Carbamat, Sulfonat, Sulfinat, Phosphat, Silyl, sowie cyclischen Formen, die diese untereinander verknüpfen, und Carbonyl, Imidat, Thiomidat,
oder kovalenten Dimeren oder Trimeren davon, wobei R¹ und/oder mindestens eines von R⁷ oder R⁸ in diesem Fall ein gemeinsames Linkerelement ist.

2. Verfahren nach Anspruch 1, wobei die Erzeugung von Oligomeren oder einer Mischung von Oligomeren unter Einsatz der folgenden Schritte erfolgt:
1) Umsetzen einer Mischung von mindestens einem Initiator (I) mit mindestens einem Monomer (M) unter Bildung eines Dimers (I-M) des Initiators (I) und des Monomers (M) oder unter Bildung eines Präoligomers (I-M-M, I-M-M-M, ...) mit einem Initiator (I), der an eine Kette von mehr als einem Monomer (M) gebunden ist, oder einer Mischung davon mittels Amidbindungsbildung;
2) Zusetzen von mindestens einem Terminator (T) für die Bildung eines linearen Oligomers (I-M-T, I-M-M-T, ...) oder einer Mischung von linearen Oligomeren mittels Amidbindungsbildung;
oder zwecks Bildung eines cyclischen Oligomers oder einer Mischung von cyclischen Oligomeren mittels Amidbindungsbildung Verändern der Reaktionsbedingungen im Vergleich zu Schritt 1) so, dass eine verknüpfende kovalente Bindung zwischen dem mindestens einen Initiator (I) und vorzugsweise dem jeweiligen terminalen Monomer (M) des in Schritt 1) gebildeten Dimers oder Präoligomers gebildet wird,

3. Verfahren nach Anspruch 1 oder 2, wobei in Schritt 1) ein einziger Initiator (I) verwendet wird und bei der Bildung eines linearen Oligomers oder einer Mischung von linearen Oligomeren mittels Amidbindungsbildung in Schritt 2) ein einziger Terminator (T) verwendet wird.

4. Verfahren nach einem der vorhergehenden Ansprüche, wobei der Initiator (I) für die Bildung eines linearen Oligomers oder einer Mischung von linearen Oligomeren mittels Amidbindungsbildung aus der Gruppe bestehend aus den Folgenden ausgewählt ist: 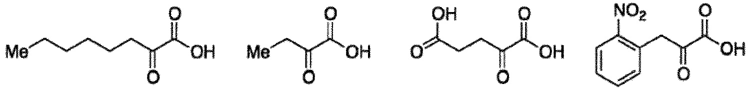 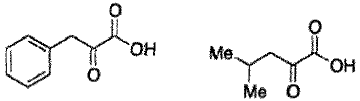 wobei Me = -CH₃.

5. Verfahren nach einem der vorhergehenden Ansprüche, wobei der Initiator (I) für die Bildung eines cyclischen Oligomers oder einer Mischung von cyclischen Oligomeren mittels Amidbindungsbildung dadurch ausgewählt wird, dass die Linkerstruktur aus der Gruppe bestehend aus den Folgenden ausgewählt wird: einer Kette von einem oder zwei Elementen, ausgewählt aus der folgenden Gruppe: Aminosäure, CO((CH₂)₂NH, und diese Kette wird durch eine Gruppe ausgewählt aus den Folgenden terminiert: 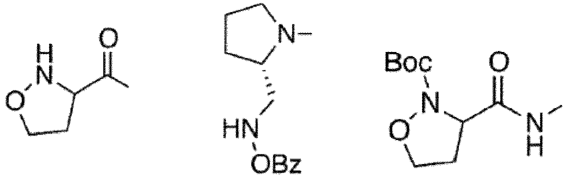 wobei Bz = Benzyl; Boc = tert.-Butyloxycarbonyl.

6. Verfahren nach einem der vorhergehenden Ansprüche, wobei der Initiator (I) für die Bildung eines cyclischen Oligomers oder einer Mischung von cyclischen Oligomeren mittels Amidbindungsbildung durch eine Struktur angegeben ist, die eine Initiatorgruppierung, ausgewählt aus der Gruppe bestehend aus den Folgenden, umfasst: 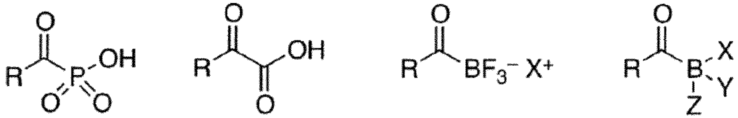 wobei
R ein Linkerelement ist
X⁺ ein Gegenion ausgewählt aus der Gruppe bestehend aus den Folgenden ist: K⁺, Cs⁺, Li⁺, Na⁺, R₄N⁺, R₄P⁺ oder R₃S⁺, wobei R ein organischer Substituent oder H ist;
X, Y, Z unabhängig voneinander aus der Gruppe bestehend aus den Folgenden ausgewählt sind: F, OR, N⁺R₃, N⁺R₂OR, N⁺R₂SR und N⁺R₂NR₂ und gegebenenfalls eine cyclische oder eine bicyclische Struktur bilden; wobei R ein organischer Substituent oder H ist;
und mindestens einer Terminatorgruppierung, ausgewählt aus der Gruppe bestehend aus den Folgenden: 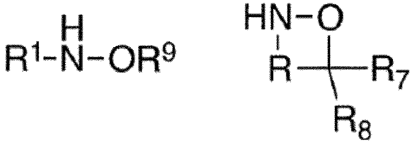 wobei
R aus der Gruppe bestehend aus den Folgenden ausgewählt ist: CH₂, (CH₂)₂, CHR^{T}, CH₂CHR^{T}, (CH₂)₃, (CH₂)₂CHR^{T}, CH₂CHR^{T}CH₂, (CH₂)₄, wobei R^{T} aus der Gruppe bestehend aus den Folgenden ausgewählt ist: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl;
wobei R¹ ein Linkerelement ist;
R⁷-R⁹ unabhängig voneinander aus der Gruppe bestehend aus den Folgenden ausgewählt sind: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl, Ester, Carbamat, Sulfonat, Sulfinat, Phosphat, Silyl, sowie cyclischen Formen, die diese untereinander verknüpfen, und Carbonyl, Imidat, Thiomidat, mit der Maßgabe, dass mindestens eines von R⁷ oder R⁸ ein Linkerelement ist,
die durch ein gemeinsames Linkerelement verknüpft sind, das in der Initiatorgruppierung durch R angegeben ist und in der Terminatorgruppierung durch R¹ oder R⁷ oder R⁸ angegeben ist.

7. Verfahren nach Anspruch 6, wobei der Initiator (I) für die Bildung eines cyclischen Oligomers oder einer Mischung von cyclischen Oligomeren mittels Amidbindungsbildung aus der Gruppe bestehend aus den Folgenden ausgewählt ist: 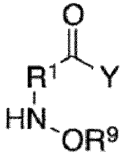 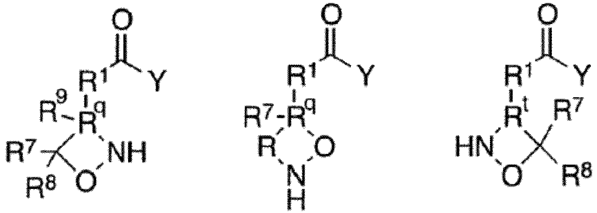 wobei
R¹ ein Linkerelement ist;
R^{q} ein Strukturelement, das auf einen 4-, 5-, 6- oder 7-gliedrigen Ring vervollständigt, ist, vorzugsweise ausgewählt aus der Gruppe bestehend aus den Folgenden: -C-, -CH₂-C-, -CHR^{T}-C-, -(CH₂ )₂-C-, --(CHR^{T})₂-C-, -(CH₂)-C-(CH₂)-, -(CHR^{T})-C-(CH₂)-, -(CHR^{T})-C-(CHR^{T})-, -(CH₂)₃-C, wobei R^{T} aus der Gruppe bestehend aus den Folgenden ausgewählt ist: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl;
R^{t} ein Strukturelement, das auf einen 4-, 5-, 6- oder 7-gliedrigen Ring vervollständigt, ist, vorzugsweise ausgewählt aus der Gruppe bestehend aus: -CR^{U}-, -CH₂CR^{U}-, -CHR^{T}-CR^{U}-,-(CH₂)₂-CR^{U}-, -(CHR^{T})₂-CR^{U}-, -(CH₂)-CR^{U}-(CH₂)-,-(CHR^{T})-CR^{U}-(CH₂)-, -(CHR^{T})-CR^{U}-(CHR^{T})-, -(CH₂)₃-CR^{U}, wobei R^{T} aus der Gruppe bestehend aus den Folgenden ausgewählt ist: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl, und wobei R^{U} aus der Gruppe bestehend aus den Folgenden ausgewählt ist: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl;
R⁷-R⁹ unabhängig voneinander aus der Gruppe bestehend aus den Folgenden ausgewählt sind: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl, sowie cyclischen Formen, die diese untereinander verknüpfen, und Carbonyl, Imidat, Thiomidat,
R aus der Gruppe bestehend aus den Folgenden ausgewählt ist: 0, S, NR₁, SiR₁R₂, CR₁R₂; wobei R₁ und R₂ unabhängig voneinander aus der Gruppe bestehend aus den Folgenden ausgewählt sind: Wasserstoff, Halogen, substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl,
Y aus der Gruppe bestehend aus den Folgenden ausgewählt ist: -PO₃H, -COOH, -BF₃⁻X⁺, -BXYZ, wobei X⁺, X, Y, Z wie oben im Zusammenhang mit dem Initiator (I) angegeben definiert sind;
wobei diese Struktur vorzugsweise aus der Gruppe bestehend aus den Folgenden ausgewählt ist: 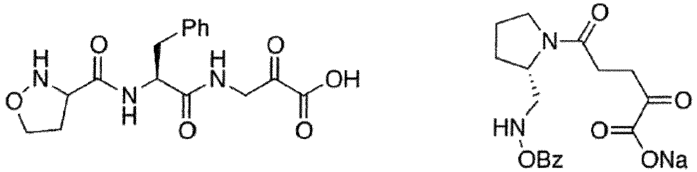 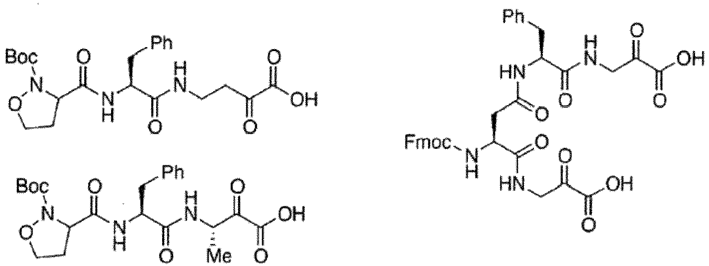 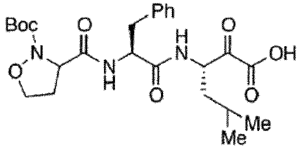 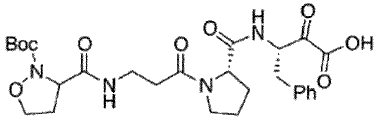 wobei boc = tert.-Butyloxycarbonyl, Ph = Phenyl, Fmoc = Fluorenylmethylenoxycarbonyl, Me = -CH₃, Bz = Benzyl.

8. Verfahren nach einem der vorhergehenden Ansprüche, wobei das Monomer (M) aus der Gruppe bestehend aus den Folgenden ausgewählt ist: 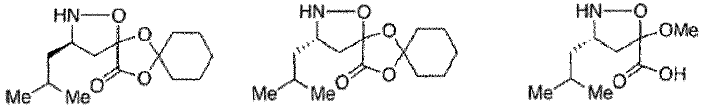 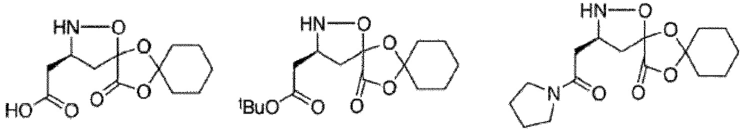 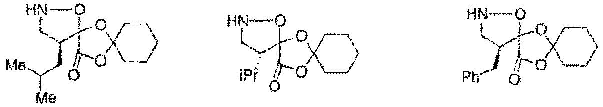 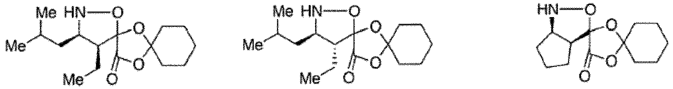 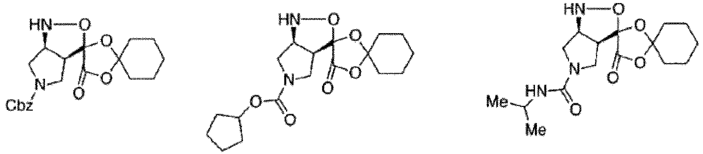 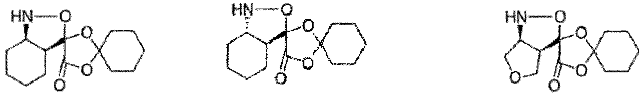 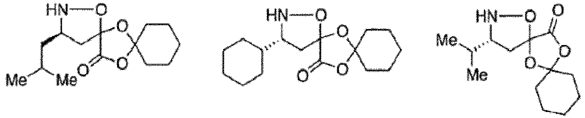 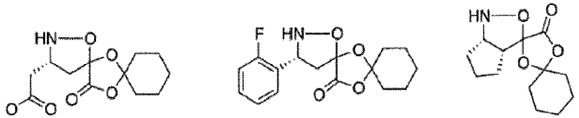 wobei Me = -CH₃, ^{t}Bu = 1, 1-Dimethylethyl, Cbz = Benzyloxycarbonyl, iPr = Isopropyl, Ph = Phenyl.

9. Verfahren nach einem der vorhergehenden Ansprüche, wobei der Terminator (T) aus der Gruppe bestehend aus den Folgenden ausgewählt ist: 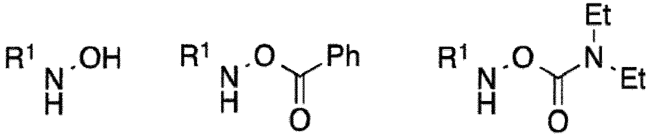 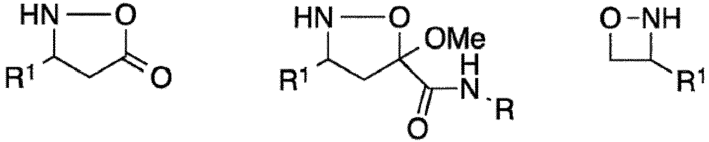 wobei
R¹ aus der Gruppe bestehend aus den Folgenden ausgewählt ist: substituiertem oder unsubstituiertem Alkyl, Heteroalkyl, Aryl, Cycloalkyl, Heterocycloalkyl, Heteroaryl, Alkylaryl, Heteroalkylaryl, Alkenyl, Heteroalkenyl, Alkinyl, Heteroalkinyl, Ester, Carbamat, Sulfonat, Sulfinat, Phosphat, Silyl;
Me = -CH₃, Et = Ethyl; Ph = Phenyl.

10. Verfahren nach einem der vorhergehenden Ansprüche, wobei Schritt 1) und/oder 2) abgesehen von Lösungsmittel(n) ohne irgendwelche weiteren zugesetzten chemischen Reagenzien oder Katalysatoren durchgeführt wird.

11. Verfahren nach einem der vorhergehenden Ansprüche, wobei organische Lösungsmittel, Wasser, wässrige Puffer oder Kombinationen davon in Schritt 1) und/oder in Schritt 2) verwendet werden.

12. Verfahren nach einem der vorhergehenden Ansprüche, wobei mehr als 1, vorzugsweise 2-6, stärker bevorzugt 2-4 unterschiedliche Monomere in Schritt 1) verwendet werden, und/oder wobei die Reaktion in Schritt 1) weitergeführt wird, um zu Oligomeren mit mindestens 2 miteinander verknüpften Monomeren, vorzugsweise im Bereich von 2-10, stärker bevorzugt im Bereich von 2-6 miteinander verknüpften Monomeren, zu führen.

13. Verfahren nach einem der vorhergehenden Ansprüche, wobei die Reaktionsbedingungen, vorzugsweise Temperatur und/oder Druck, und/oder Reaktantenkonzentrationen und/oder die Zugabereihenfolge der Reaktanten und/oder die Zugabezeit der Reaktanten und/oder die Chiralität der Reaktanten in Schritt 1) so ausgewählt werden, dass sie zu gezielten unterschiedlichen Verteilungen von unterschiedlichen Oligomeren in der Mischung zwischen den unterschiedlichen Chargen führen.

14. Verfahren nach einem der vorhergehenden Ansprüche, wobei in Schritt 1) ein einziger Initiator (I), ein einziges Monomer (M) und bei der Erzeugung von linearen Oligomeren in Schritt 2) ein einziger Terminator (T) verwendet wird, und wobei die Reaktionsbedingungen in Schritt 1) und/oder Schritt 2) so angepasst werden, dass eine spezifische Trimerstruktur gebildet wird.

15. Verfahren zum Identifizieren von biologisch und/oder chemisch aktiven Systemen in einer Chemikalienbibliothek, die auf mindestens einer Mischung von Oligomeren beruht, welche unter Verwendung eines Verfahrens nach einem der vorhergehenden Ansprüche erzeugt wurden, wobei die Mischungen von Oligomeren vor der Aufreinigung oder Trennung der Verbindungen aus der Mischung auf Aktivität durchmustert wurden.

16. Verfahren nach Anspruch 15, bei dem man eine Anzahl von spezifisch unterschiedlichen Mischungen, die unter Verwendung eines Verfahrens nach Anspruch 10 und/oder 11 hergestellt wurde, verwendet, diese Mischungen auf biologische und/oder chemische Aktivität überprüft, aufgrund von Aktivitätsmustern Schlüsse bezüglich Initiatoren und/oder Monomeren und/oder Terminatoren, die Aktivität induzieren, schließt, unter Verwendung eines Verfahrens nach einem der vorhergehenden Ansprüche 1-12 ausschließlich auf Basis der identifizierten aktiven Initiatoren und/oder Monomeren und/oder Terminatoren weitere Mischungen herstellt und dadurch nach und nach die Anzahl der möglichen aktiven Oligomere reduziert.

## Revendications

1. Procédé de génération d'oligomères ou d'un mélange d'oligomères pour former une banque chimique par oligomérisation par formation d'amide comprenant les étapes de
1) réaction d'un mélange d'au moins un initiateur (I) avec au moins un monomère (M)
pour former un dimère (I-M) de l'initiateur (I) et du monomère (M) ou
pour former un pré-oligomère (I-M-M, I-M-M-M, ...) avec un initiateur (I) lié à une chaîne de plus d'un monomère (M), ou un mélange de ceux-ci par formation de liaison amide ;
2) addition d'au moins un terminateur (T) pour la formation d'un oligomère linéaire (I-M-T, I-M-M-T, ...) ou d'un mélange d'oligomères linéaires par formation de liaison amide ;
ou, pour la formation d'un oligomère cyclique ou d'un mélange d'oligomères cycliques par formation de liaison amide, modification des conditions de réaction par rapport à l'étape 1) de façon à former une liaison covalente entre l'au moins un initiateur (I) et un monomère (M) du dimère ou pré-oligomère formé dans l'étape 1),
dans lequel l'initiateur (I) est choisi dans le groupe constitué de : 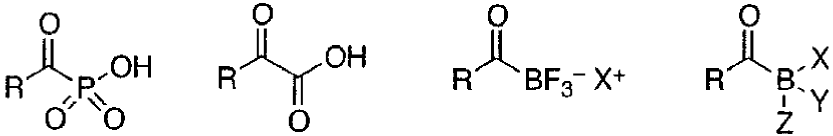
avec, pour la formation d'un oligomère linéaire ou un mélange d'oligomères linéaires par formation de liaison amide,
R étant choisi dans le groupe constitué de : alkyle, hétéroalkyle, cycloalkyle, hétérocycloalkyle, aryle, hétéroaryle, alkylaryle, hétéroalkylaryle, alcényle, hétéroalcényle, alcynyle, hétéroalcynyle substitué ou non substitué, -C(NH₂)R^{I}, -C(NH₂){R^{I}-CO-C(NH₂)}ₙR^{I} avec n = 1, 2 et R¹ étant H ou une chaîne latérale d'acide aminé, un colorant fluorescent, un acide nucléique ou un dérivé de ceux-ci, un acide nucléique peptidique, un octapeptide FLAG (DYKDDDDK), la biotine ou une étiquette d'affinité ;
ou avec, pour la formation d'un oligomère cyclique ou d'un mélange d'oligomères cycliques par formation de liaison amide,
R étant choisi dans le groupe constitué de : -{(CH₂)}ₙR^{C}, -{(CHCH₃)}ₙR^{C}, -{(CH(1,1-diméthyléthyl))}ₙR^{C}, -{(CH(benzyl))}ₙR^{C}, dans chaque cas avec n = 1,2 et R^{C} étant une structure de lieur permettant de former une liaison amide covalente avec le monomère terminal respectif du dimère ou oligomère formé dans l'étape 1) ;
et dans les deux cas avec
X⁺ étant un contre-ion, choisi dans le groupe constitué de : K⁺, Cs⁺, Li⁺, Na⁺, R₄N⁺, R₄P⁺ ou R₃S⁺ avec R étant un substituant organique ou H ;
X, Y, Z, étant, indépendamment les uns des autres, choisis dans le groupe constitué de : F, OR, N⁺R₃, N⁺R₂OR, N⁺R₂SR, et N⁺R₂NR₂, et forment facultativement une structure cyclique ou bicyclique ; où R est un substituant organique ou H ;
ou des dimères ou trimères covalents de ceux-ci avec R étant dans ce cas un élément de lieur commun ;
dans lequel le monomère (M) est choisi dans le groupe constitué de : 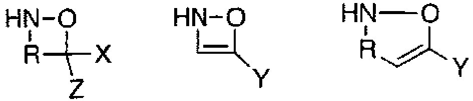 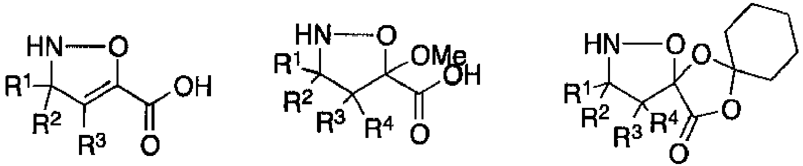 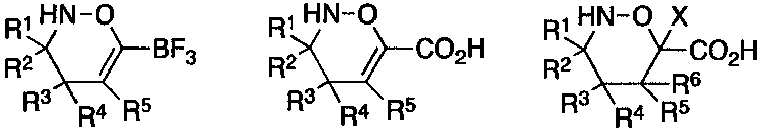 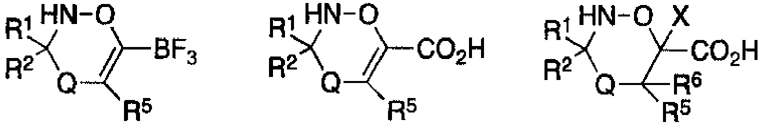 avec
X étant choisi dans le groupe constitué de : halogène, -OH, -COOH, -NH₂, -O-alkyle, -O-aryle, -O-CO-alkyle, -O-CO-aryle, -SH, S-alkyle, -S-aryle, N-acyle, -NH-alkyle, -NH-aryle, -N(alkyle)₂, -N(aryle)₂, -N(alkyl)(aryle), -CO-NH-alkyle, -CO-NH-aryle, -CO-N(alkyle)₂, -CO-N(aryle)₂, -CO-N(alkyl)(aryle), -CN, -NO₂, -N₃, -S(O) aryle, -S(O)₂aryle ;
Y étant choisi dans le groupe constitué de : -PO₃H, -COOH, -BF₃-X⁺, -BXYZ, où X⁺, X, Y, Z sont définis comme décrit ci-dessus dans le contexte de l'initiateur (I),
Z étant choisi dans le groupe constitué de : -PO₃H, -COOH, -BF₃-X⁺, -BXYZ, où X⁺, X, Y, Z sont définis comme décrit ci-dessus dans le contexte de l'initiateur (I), ainsi que des dérivés de ceux-ci qui, après séparation de X et Z après clivage de la liaison NO, conduisent à Y ;
R étant choisi dans le groupe constitué de : O, S, NR¹, Si, CHR¹R² ;
R¹-R⁶ étant, indépendamment les uns des autres, choisis dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué, ainsi que des formes cycliques reliant ceux-ci les uns aux autres ;
Q étant choisi dans le groupe constitué de : O, S, Si, NR¹, où R¹ est un substituant organique ou H ;
et dans lequel le terminateur (T), s'il est utilisé, est choisi dans le groupe constitué de : 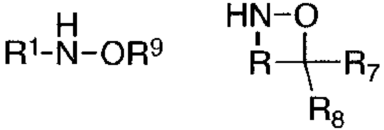
avec
R étant choisi dans le groupe constitué de : CH₂, (CH₂)₂, CHR^{T}, CH₂CHR^{T}, (CH₂)₃, (CH₂)₂CHR^{T}, CH₂CHR^{T}CH₂, (CH₂)₄, où R^{T} est choisi dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué ;
R¹ étant choisi dans le groupe constitué de : alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué, ester, carbamate, sulfonate, sulfinate, phosphate, silyle ;
R⁷-R⁹ étant, indépendamment les uns des autres, choisis dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué, ester, carbamate, sulfonate, sulfinate, phosphate, silyle, ainsi que des formes cycliques reliant ceux-ci les uns aux autres et carbonyle, imidate, thiomidate
ou des dimères ou trimères covalents de ceux-ci avec R¹ et/ou au moins l'un de R⁷ ou R⁸ étant dans ce cas un élément de lieur commun.

2. Procédé selon la revendication 1, dans lequel la génération d'oligomères ou d'un mélange d'oligomères est conduite en utilisant les étapes de
1) réaction d'un mélange d'au moins un initiateur (I) avec au moins un monomère (M)
pour former un dimère (I-M) de l'initiateur (I) et du monomère (M) ou
pour former un pré-oligomère (I-M-M, I-M-M-M, ...) avec un initiateur (I) lié à une chaîne de plus d'un monomère (M), ou un mélange de ceux-ci par formation de liaison amide ;
2) addition d'au moins un terminateur (T) pour la formation d'un oligomère linéaire (I-M-T, I-M-M-T, ...) ou d'un mélange d'oligomères linéaires par formation de liaison amide ;
ou, pour la formation d'un oligomère cyclique ou d'un mélange d'oligomères cycliques par formation de liaison amide, modification des conditions de réaction par rapport à l'étape 1) de façon à former une liaison covalente entre l'au moins un initiateur (I) et, de préférence, le monomère (M) terminal respectif du dimère ou pré-oligomère formé dans l'étape 1).

3. Procédé selon la revendication 1 ou 2, dans lequel un seul initiateur (I) est utilisé dans l'étape 1) et, dans le cas de la formation d'un oligomère linéaire ou d'un mélange d'oligomères linéaires par formation de liaison amide, un seul terminateur (T) est utilisé dans l'étape 2).

4. Procédé selon l'une quelconque des revendications précédentes, dans lequel l'initiateur (I) pour la formation d'un oligomère linéaire ou d'un mélange d'oligomères linéaires par formation de liaison amide est choisi dans le groupe constitué de : 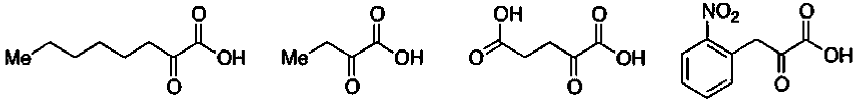 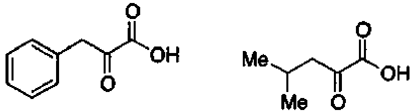 avec Me= -CH₃.

5. Procédé selon l'une quelconque des revendications précédentes, dans lequel l'initiateur (I) pour la formation d'un oligomère cyclique ou d'un mélange d'oligomères cycliques par formation de liaison amide est choisi de sorte que la structure de lieur soit choisie dans le groupe constitué de : une chaîne d'un ou deux éléments choisis dans le groupe de : un acide aminé, CO((CH₂)₂NH, et cette chaîne terminée par un groupe choisi parmi : 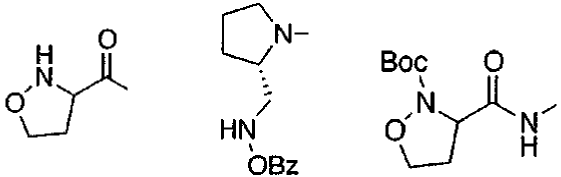 avec Bz=benzyle ; Boc= tert-butyloxycarbonyle.

6. Procédé selon l'une quelconque des revendications précédentes, dans lequel l'initiateur (I) pour la formation d'un oligomère cyclique ou d'un mélange d'oligomères cycliques par formation de liaison amide est défini par une structure comprenant un fragment d'initiateur choisi dans le groupe constitué de : 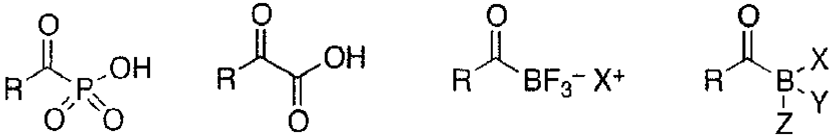 avec
R étant un élément de lieur
X⁺ étant un contre-ion, choisi dans le groupe constitué de : K⁺, Cs⁺, Li⁺, Na⁺, R₄N⁺, R₄P⁺ ou R₃S⁺ avec R étant un substituant organique ou H ;
X, Y, Z, étant, indépendamment les uns des autres, choisis dans le groupe constitué de : F, OR, N⁺R₃, N⁺R₂OR, N⁺R₂SR, et N⁺R₂NR₂, et forment facultativement une structure cyclique ou bicyclique ; où R est un substituant organique ou H ;
et au moins un fragment de terminateur choisi dans le groupe constitué de : 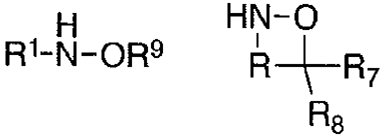 avec
R étant choisi dans le groupe constitué de : CH₂, (CH₂)₂, CHR^{T}, CH₂CHR^{T}, (CH₂)₃, (CH₂)₂CHR^{T}, CH₂CHR^{T}CH₂, (CH₂)₄, où R^{T} est choisi dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué ;
R¹ étant un élément de lieur ;
R⁷-R⁹ étant, indépendamment les uns des autres, choisis dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué, ester, carbamate, sulfonate, sulfinate, phosphate, silyle, ainsi que des formes cycliques reliant ceux-ci les uns aux autres et carbonyle, imidate, thiomidate, à condition qu'au moins l'un de R⁷ ou R⁸ soit un élément de lieur,
liés par un élément de lieur commun défini par R dans le fragment d'initiateur et par R¹ ou R⁷ ou R⁸ dans le fragment de terminateur.

7. Procédé selon la revendication 6, dans lequel l'initiateur (I) pour la formation d'un oligomère cyclique ou d'un mélange d'oligomères cycliques par formation de liaison amide est choisi dans le groupe constitué de : 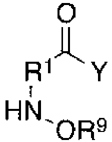 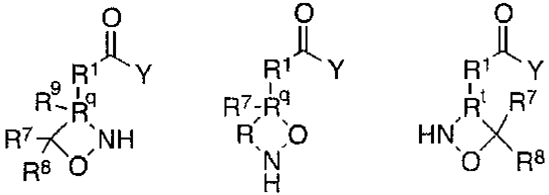 dans lequel
R¹ est un élément de lieur ;
R^{q} est un élément structural complémentant un cycle de 4, 5, 6 ou 7 chaînons, de préférence choisi dans le groupe constitué de : -C-, -CH₂-C-, -CHR^{T}-C-, -(CH₂)₂-C-, -(CHR^{T})₂-C-, -(CH₂)-C-(CH₂)-, -(CHR^{T})-C-(CH₂)-, -(CHR^{T})-C-(CHR^{T})-, -(CH₂)₃-C, où R^{T} est choisi dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué ;
R^{t} est un élément structural complémentant un cycle de 4, 5, 6 ou 7 chaînons, de préférence choisi dans le groupe constitué de : -CR^{U}-, -CH₂-CR^{U}-, - CHR^{T}-CR^{U}-, -(CH₂)₂-CR^{U}-, -(CHR^{T})₂-CR^{U}-, -(CH₂)-CR^{U}-(CH₂)-, -(CHR^{T})-CR^{U}-(CH₂)-, -(CHR^{T})-CR^{U}-(CHR^{T})-, -(CH₂)₃-CR^{U}, dans lequel R^{T} est choisi dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué, et où R^{U} est choisi dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué ;
R⁷-R⁹ étant, indépendamment les uns des autres, choisis dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué, ainsi que des formes cycliques reliant ceux-ci les uns aux autres et carbonyle, imidate, thiomidate,
R étant choisi dans le groupe constitué de : O, S, NR₁, SiR₁R₂, CR₁R₂ ; où R₁ et R₂ sont, indépendamment l'un de l'autre, choisis dans le groupe constitué de : hydrogène, halogène, alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué,
Y étant choisi dans le groupe constitué de : -PO₃H, -COOH, -BF₃X⁺, -BXYZ, où X⁺, X, Y, Z sont définis comme décrit ci-dessus dans le contexte de l'initiateur (I) ;
dans lequel, de préférence, cette structure est choisie dans le groupe constitué de : 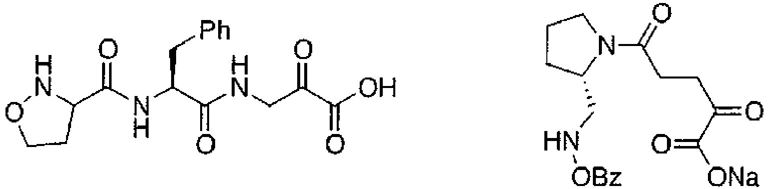 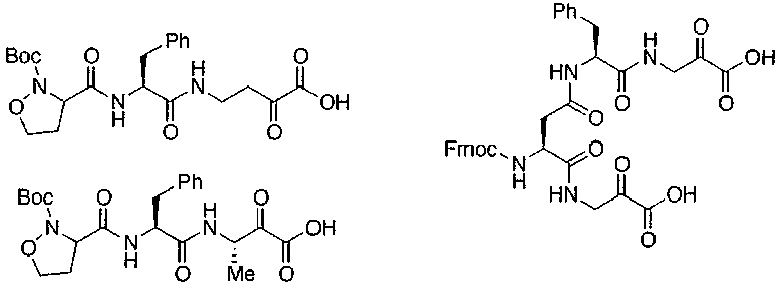 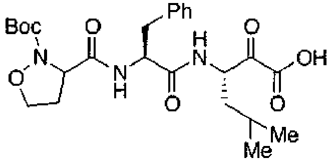 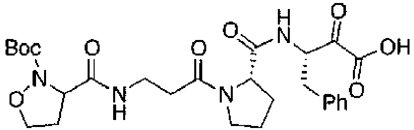 avec boc = tert-butyloxycarbonyle, Ph = phényle, Fmoc = fluorénylméthylèneoxycarbonyle, Me = -CH₃, Bz = benzyle.

8. Procédé selon l'une des revendications précédentes, dans lequel le monomère (M) est choisi dans le groupe constitué de : 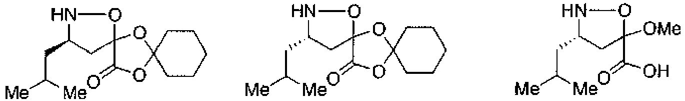 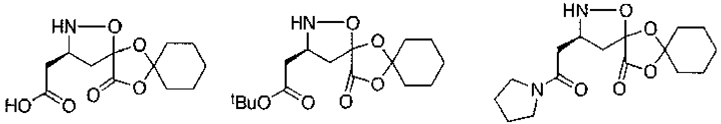 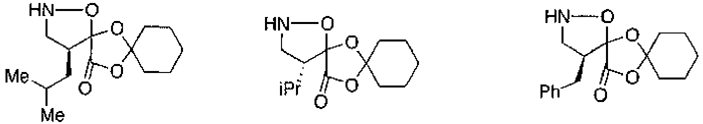 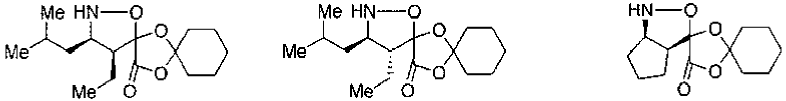 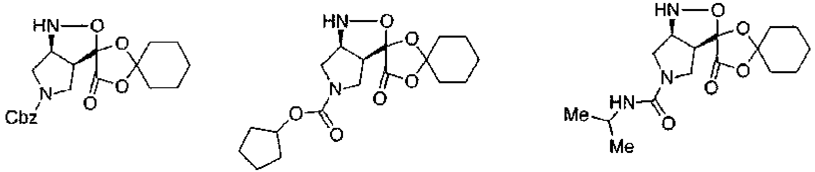 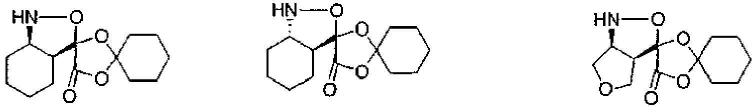 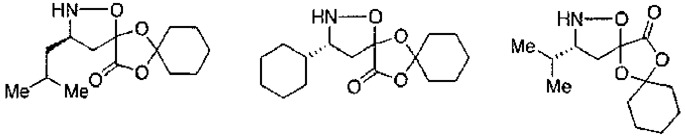 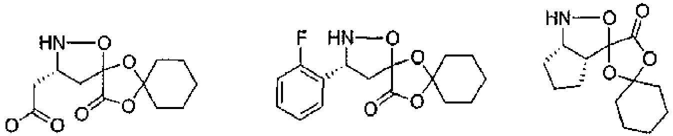 avec Me = -CH₃, ^{t}Bu = 1,1-diméthyléthyle, Cbz = benzyloxycarbonyle, iPr = isopropyle, Ph = phényle.

9. Procédé selon l'une quelconque des revendications précédentes, dans lequel le terminateur (T) est choisi dans le groupe constitué de : 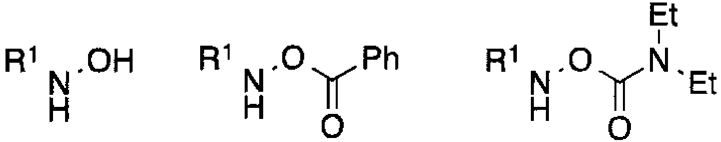 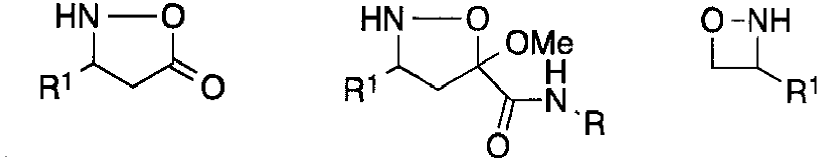 avec
R¹ étant choisi dans le groupe constitué de : alkyle, hétéroalkyle, aryle, cycloalkyle, hétérocycloalkyle, hétéroaryle, alkylaryle, hétéroalkylaryle, alcényle, hétéroalcényle, alcynyle, hétéroalcynyle substitué ou non substitué, ester, carbamate, sulfonate, sulfinate, phosphate, silyle ;
Me = -CH₃, Et = éthyle ; Ph = phényle.

10. Procédé selon l'une quelconque des revendications précédentes, dans lequel les étapes 1) et/ou 2) sont conduites, hormis le(s) solvant (s), sans aucun réactif ou catalyseur chimique supplémentaire ajouté.

11. Procédé selon l'une quelconque des revendications précédentes, dans lequel, dans l'étape 1) et/ou dans l'étape 2), des solvants organiques, de l'eau, un tampon aqueux ou des combinaisons de ceux-ci sont utilisés.

12. Procédé selon l'une quelconque des revendications précédentes, dans lequel, dans l'étape 1), plus de 1, de préférence 2 à 6, plus préférablement 2 à 4 monomères différents sont utilisés, et/ou dans lequel, dans l'étape 1), la réaction est conduite pour conduire à des oligomères avec au moins 2 monomères interconnectés, de préférence dans la plage de 2 à 10, plus préférablement dans la plage de 2 à 6 monomères interconnectés.

13. Procédé selon l'une quelconque des revendications précédentes, dans lequel, dans l'étape 1), les conditions de réaction, de préférence la température et/ou la pression, et/ou les concentrations des réactifs et/ou l'ordre d'ajout des réactifs et/ou le temps d'ajout des réactifs et/ou la chiralité des réactifs sont choisis de façon à conduire, entre différent lots, à différentes distributions ciblées de différents oligomères dans le mélange.

14. Procédé selon l'une quelconque des revendications précédentes, dans lequel, dans l'étape 1), un seul initiateur (I), un seul monomère (M) et, dans le cas de la génération d'oligomères linéaires, dans l'étape 2) un seul terminateur (T) est utilisé, et dans lequel les conditions de réaction dans l'étape 1) et/ou l'étape 2) sont adaptées de façon à former une structure de trimère spécifique.

15. Procédé d'identification de systèmes biologiquement et/ou chimiquement actifs depuis une banque chimique sur la base d'au moins un mélange d'oligomères préparés en utilisant un procédé selon l'une quelconque des revendications précédentes, dans lequel les mélanges d'oligomères sont criblés pour une activité avant la purification ou la séparation des composés à partir du mélange.

16. Procédé selon la revendication 15, utilisant une pluralité de mélanges différant spécifiquement préparés en utilisant un procédé selon la revendication 10 et/ou 11, le contrôle de ces mélanges pour une activité biologique et/ou chimique, la déduction à partir des profils d'activité d'initiateurs et/ou monomères et/ou terminateurs induisant une activité, la préparation de mélanges supplémentaires en utilisant un procédé selon l'une quelconque des revendications précédentes 1 à 12 sur la base des initiateurs et/ou monomères et/ou terminateurs actifs identifiés de façon unique, de façon à réduire successivement le nombre d'oligomères actifs possibles.
